# EUROPÄISCHE PATENTSCHRIFT

(11) **EP 1 254 238 B1**
(45) Veröffentlichungstag und Bekanntmachung des Hinweises auf die Patenterteilung: **16.09.2009**
(21) Anmeldenummer: 01913791.8
(22) Anmeldetag: 08.02.2001
(51) Int. Cl.: C12N 15/54, C12N 15/82, C12N 9/10, A01H 5/00, C07K 16/40, C12P 7/64, C11B 1/00, C11C 1/00, G01N 33/573, C12Q 1/48, A61K 38/45

(54) **NEUES ELONGASEGEN UND VERFAHREN ZUR HERSTELLUNG MEHRFACH UNGESÄTTIGTER FETTSÄUREN**
NOVEL ELONGASE GENE AND METHOD FOR PRODUCING MULTIPLE-UNSATURATED FATTY ACIDS
NOUVEAU GENE D'ELONGASE ET PROCEDE DE PREPARATION D'ACIDES GRAS A INSATURATION MULTIPLE

(30) Priorität: 09.02.2000 DE 10005973; 17.05.2000 DE 10023893; 19.12.2000 DE 10063387
(43) Veröffentlichungstag der Anmeldung: 06.11.2002
(73) Patentinhaber: BASF SE, 67056 Ludwigshafen (DE)
(72) Erfinder: HEINZ, Ernst, 22609 Hamburg (DE); ZANK, Thorsten, 22303 Hamburg (DE); ZÄHRINGER, Ulrich, 23845 Borstel (DE); LERCHL, Jens, 26831 Svalöv (SE); RENZ, Andreas, 67117 Limburgerhof (DE)
(74) Vertreter: Dick, Alexander
(86) Internationale Anmeldenummer: PCT/EP2001/001346
(87) Internationale Veröffentlichungsnummer: WO 2001/059128

(56) Entgegenhaltungen:
- WO-A-00/12720
- WO-A-98/46764
- MILLAR A AND KUNST L: "Very-long-chain fatty acid biosynthesis is controlled through the expression and specificity of the condensing enzyme" PLANT JOURNAL, BLACKWELL SCIENTIFIC PUBLICATIONS, OXFORD, GB, Bd. 12, Nr. 1, 1997, Seiten 121-131, XP002125076 ISSN: 0960-7412
- DATABASE EM_EST [Online] EMBL Heidelberg; AC/ID AW156099, 9. November 1999 (1999-11-09) QUATRANO R ET AL.: "ga22h08.y1 Moss EST library PPU Physcomitrella patens cDNA clone" XP002174837
- ZANK T K ET AL: "Cloning and functional expression of the first plant fatty acid elongase specific for DELTA6-polyunsaturated fatty acids." BIOCHEMICAL SOCIETY TRANSACTIONS, Bd. 28, Nr. 6, Dezember 2000 (2000-12), Seiten 654-658, XP002174836 ISSN: 0300-5127

## Beschreibung

### Gebiet der Erfindung

Die Erfindung betrifft neue Elongasegene mit den in Sequenz SEQ ID NO: 1, SEQ ID NO: 3, und SEQ ID NO: 9 genannten Sequenzen oder ihre Homologen, Derivate oder Analoga, ein Genkonstrukt, das diese Gene oder ihre Homologen, Derivate oder Analoga enthält, sowie seine Verwendung. Die Erfindung betrifft zu dem Vektoren oder transgene nicht-menschliche Organismen, die ein Elongasegen mit der Sequenz SEQ ID NO:1, SEQ ID NO:3, und SEQ ID NO:9 oder seine Homologen, Derivate oder Analoga enthalten. Weiterhin betrifft die Erfindung die Verwendung der Elongasegensequenzen allein oder in Kombination mit weiteren Elongasen und/oder weiteren Fettsäure-Biosynthesegenen. Diese Erfindung betrifft ein neues Elongasegen mit der Sequenz SEQ ID NR:1 oder seine Homologa, Derivate oder Analoga.

Ferner betrifft die Erfindung ein Verfahren zur Herstellung mehrfach ungesättigter Fettsäuren sowie ein Verfahren zum Einbringen von DNA in nicht-menschliche Organismen, die große Mengen an Ölen und insbesondere Ölen mit hohem Gehalt an ungesättigten Fettsäuren produzieren.

### Hintergrund der Erfindung

Bestimmte Produkte und Nebenprodukte natürlich vorkommender Stoffwechselprozesse in Zellen sind für ein breites Spektrum an Industrien, einschließlich der Futtermittel-, Nahrungsmittel-, Kosmetik- und pharmazeutischen Industrie, nützlich. Zu diesen gemeinsam als "Feinchemikalien" bezeichneten Molekülen gehören auch Lipide und Fettsäuren, unter denen eine beispielhafte Klasse die mehrfach ungesättigten Fettsäuren sind. Mehrfach ungesättigte Fettsäuren (polyunsaturated fatty acids, PUFAs) werden beispielsweise zu Nahrungsformulierungen für Kinder gegeben, um einen höheren Nährwert dieser Formulierungen zu erzeugen. PUFAs haben zum Beispiel einen positiven Einfluß auf den Cholesterinspiegel im Blut von Menschen und eignen sich daher zum Schutz gegen Herzkrankheiten. Feinchemikalien und mehrfach ungesättigte Fettsäuren (polyunsaturated fatty acids, PUFAs) lassen sich aus tierischen Quellen, wie beispielsweise Fisch oder Mikroorganismen, isolieren. Durch Anzucht dieser Mikroorganismen lassen sich große Mengen eines oder mehrerer gewünschter Moleküle produzieren und isolieren.

Besonders geeignete Mikroorganismen zur Herstellung von PUFAs sind Mikroorganismen wie Thraustochytrien oder Schizochytrien-Stämme, Algen wie Phaeodactylum tricornutum oder Crypthecodinium-Arten, Ciliaten, wie Stylonychia oder Colpidium, Pilze, wie Mortierella, Entomophthora oder Mucor. Durch Stammselektion ist eine Anzahl von Mutantenstämmen der entsprechenden Mikroorganismen entwickelt worden, die eine Reihe wünschenswerter Verbindungen, einschließlich PUFAs, produzieren. Die Selektion von Stämmen mit verbesserter Produktion eines bestimmten Moleküls ist jedoch ein zeitraubendes und schwieriges Verfahren. Von Nachteil ist außerdem, daß sich mit einem definierten Mikroorganismus nur bestimmte ungesättigte Fettsäuren bzw. nur ein bestimmtes Fettsäurespektrum herstellbar ist.

Alternativ kann die Produktion von Feinchemikalien geeigneterweise über die Produktion von Pflanzen, die so entwickelt sind, daß sie die vorstehend genannten PUFAs herstellen, im großen Maßstab durchgeführt werden. Besonders gut für diesen Zweck geeignete Pflanzen sind Ölfruchtpflanzen, die große Mengen an Lipidverbindungen enthalten wie Raps, Canola, Lein, Soja, Sonnenblumen, Borretsch und Nachtkerze. Aber auch andere Nutzpflanzen, die Öle oder Lipide und Fettsäuren enthalten, sind gut geeignet, wie in der eingehenden Beschreibung dieser Erfindung erwähnt. Mittels herkömmlicher Züchtung sind eine Reihe von Mutantenpflanzen entwickelt worden, die ein Spektrum an wünschenswerten Lipiden und Fettsäuren, Cofaktoren und Enzymen produzieren. Die Selektion neuer Pflanzensorten mit verbesserter Produktion eines bestimmten Moleküls ist jedoch ein zeitaufwendiges und schwieriges Verfahren oder sogar unmöglich, wenn die Verbindung in der entsprechenden Pflanze nicht natürlich vorkommen, wie im Fall von mehrfach ungesättigten C₂₀-Fettsäuren und C₂₂-Fettsäuren und solchen mit längeren Kohlenstoffketten.

### Zusammenfassung der Erfindung

Die Erfindung stellt neue Nukleinsäuremoleküle bereit, die zur Identifizierung und Isolierung von Elongasegenen der PUFA-Biosynthese geeignet sind und zur Modifikation von Ölen, Fettsäuren, Lipiden, von Lipiden stammenden Verbindungen und am stärksten bevorzugt zur Herstellungmehrfach ungesättigter Fettsäuren verwendet werden können, da nach wie vor ein großer Bedarf an neuen Genen, die für Enzyme codieren, die an der Biosynthese ungesättigter Fettsäuren beteiligt sind und es ermöglichen, diese in einem technischen Maßstab herzustellen, besteht. Insbesondere besteht ein Bedarf an Fettsäurebiosyntheseenzymen, die die Elongation von mehrfach ungesättigten Fettsäuren bevorzugt mit zwei oder mehr Doppelbindungen im Molekül ermöglichen. Die erfindungsgemäßen Nukleinsäure codieren für Enzyme, die diese Fähigkeit besitzen.

Mikroorganismen, wie Phaeodactylum, Colpidium, Mortierella, Entomophthora, Mucor, Crypthecodinium sowie andere Algen und Pilze und Pflanzen, insbesondere Ölfruchtpflanzen, werden gemeinhin in der Industrie zur Produktion einer Vielzahl von Feinchemikalien im großen Maßstab verwendet.

Unter der Voraussetzung der Verfügbarkeit von Klonierungsvektoren und Techniken zur genetischen Manipulation der obengenannten Mikroorganismen und Ciliaten, wie in WO 98/01572 und WO 00/23604 offenbart, oder Algen und verwandten Organismen, wie Phaeodactylum tricornutum, beschrieben in Falciatore et al. [1999, Marine Biotechnology 1(3):239-251]; sowie Dunahay et al. [1995, Genetic transformation of diatoms, J. Phycol. 31:10004-1012] und den Zitaten darin, können die erfindungsgemäßen Nukleinsäuremoleküle zur gentechnologischen Veränderung dieser Organismen verwendet werden, so daß sie bessere oder effizientere Produzenten einer oder mehrerer Feinchemikalien speziell ungesättigter Fettsäuren werden. Diese verbesserte Produktion oder Effizienz der Produktion einer Feinchemikalie kann durch eine direkte Wirkung der Manipulation eines erfindungsgemäßen Gens oder durch eine indirekte Wirkung dieser Manipulation hervorgerufen werden.

Moose und Algen sind die einzigen bekannten Pflanzensysteme, die erhebliche Mengen an mehrfach ungesättigten Fettsäuren, wie Arachidonsäure (= ARA) und/oder Eicosapentaensäure (= EPA) und/oder Docosahexaensäure (= DHA) herstellen. Moose enthalten PUFAs in Membranlipiden während Algen, algenverwandte Organismen und einige Pilze auch nennenswerte Mengen an PUFAs in der Triacylglycerolfraktion akkumulieren. Daher eignen sich Nukleinsäuremoleküle, die aus solchen Stämmen isoliert werden, die PUFAs auch in der Triacylglycerolfraktion akkumulieren, besonders zur Modifikation des Lipid- und PUFA-Produktionssystems in einem Wirt, insbesondere in Mikroorganismen, wie den vorstehend erwähnten Mikroorganismen, und Pflanzen, wie Ölfruchtpflanzen, beispielsweise Raps, Canola, Lein, Soja, Sonnenblume, Borretsch, Rizinus, Ölpalme, Färbersaflor (Carthamus tinctorius), Kokosnuß, Erdnuß oder Kakaobohne. Ferner können Nukleinsäuren aus Triacylglycerol-akkumulierenden Mikroorganismen zur Identifikation solcher DNA-Sequenzen und Enzyme in anderen Arten, die sich zur Modifikation der Biosynthese von Vorläufermolekülen von PUFAs in den entsprechenden Organismen eignen, verwendet werden.

Insbesondere Mikroorganismen wie Crypthecodinium cohnii und Thraustochytrium species sind Mikrooganismen, die PUFAs wie ARA, EPA oder DHA in Triacylglycerolen akkumulieren. Thraustochytrien sind phylogenetisch auch eng verwandt mit den Schizochytrien Stämmen. Obwohl diese Organismen nicht eng mit Moosen wie Physcomitrella verwandt sind, sind auf DNA-Sequenz- und insbesondere Polypeptid-Ebene Sequenzähnlichkeiten in einem solchen Maße feststellbar, daß DNA-Moleküle in heterologen Hybridisierungsexperimenten, Sequenzvergleichen und Experimenten unter Verwendung der Polymerasekettenreaktion auch aus evolutiv weit entfernten Organismen identifizierbar und isolierbar und funktionell charakterisierbar sind. Insbesondere lassen sich Konsensussequenzen ableiten, die sich zum heterologen Sichten oder zur funktionellen Komplementierung und Vorhersage von Genfunktionen in dritten Arten eignet. Die Fähigkeit, diese Funktionen zu identifizieren, z.B. die Vorhersage der Substratspezifität von Enzymen, kann daher von signifikanter Bedeutung sein. Ferner können diese Nukleinsäuremoleküle als Bezugssequenzen zur Kartierung verwandter Genome oder zur Ableitung von PCR-Primern dienen.

Die neuen Nukleinsäuremoleküle codieren für Proteine, die hier als PUFA-spezifische Elongasen (= PSEs oder im Singular PSE) bezeichnet werden. Diese PSEs können beispielsweise eine Funktion ausüben, die am Stoffwechsel (z.B. an der Biosynthese oder am Abbau) von Verbindungen, die zur Lipid- oder Fettsäuresynthese notwendig sind, wie PUFAs, beteiligt sind oder am Transmembrantransport einer oder mehrerer Lipid-/Fettsäureverbindungen entweder in die oder aus der Zelle teilnehmen.

In dieser neuenAnmeldung wird die Isolierung solcher neuen Elongasegene eingehender dargestellt. Wir haben zum ersten Mal Elongasegene isoliert, die zur Produktion langkettiger mehrfach ungesättigter Fettsäuren, vorzugsweise mit mehr als achtzehn oder zwanzig Kohlenstoffatomen im Kohlenstoffgrundgerüst der Fettsäure und/oder mindestens zwei Doppelbindungen in der Kohlenstoffkette, geeignet sind und dabei aus typischen Organismen stammen, die hohe Mengen an PUFAs in der Triacylgycerolfraktion enthalten. Daher ist hierin im Singular von einem PSE-Gen oder -Protein die Rede bzw im Plural von PSE-Genen oder -Proteinen. Andere bekannte Patentanmeldungen und Publikationen offenbaren oder zeigen kein funktionell aktives PSE-Gen, obgleich es verschiedene bekannte Patentanmeldungen gibt, welche die Verlängerung gesättigter Fettsäuren mit kurzer oder mittlerer Kette (WO 98/46776 und US 5,475,099) oder die Verlängerung oder Produktion langkettiger Fettsäuren, die dann aber nicht mehr als eine Doppelbindung haben oder zu langkettigen Fettsäure-Wachsestern führen (siehe: WO 98/54954, WO 96/13582, WO 95/15387) zeigen. Die hier vorliegende Erfindung beschreibt die Isolierung neuartiger Elongasen mit neuen Eigenschaften. Ausgehend von der in SEQ ID NO: 1 genannten Sequenz konnten, weitere Nukleinsäuren, die für Elongasen codieren, die ungesättigte Fettsäuren verlängern, gefunden werden.

WO 99/64616, WO 98/46763, WO 98/46764, WO 98/46765 beschreiben die Herstellung von PUFAs in transgenen Pflanzen und zeigen die Klonierung und funktionelle Expression entsprechender Desaturase-Aktivitäten, insbesondere aus Pilzen, zeigen aber kein PSE-codierendes Gen und keine funktionelle PSE-Aktivität. Die Expression der Desaturase-Aktivitäten führt zu einer Verschiebung der Fettsäurezusammensetzung in den transgenen Pflanzen eine Erhöhung des Gehalts an ungesättigten Fettsäuren wurde nicht beobachtet. Die Herstellung einer Triensäure mit C₁₈-Kohlenstoffkette ist gezeigt und anhand von gamma-Linolensäure beansprucht worden, es wurde jedoch bisher nicht die Herstellung sehr langkettiger mehrfach ungesättigter Fettsäuren (mit C₂₀- und längerer Kohlenstoffkette sowie von Triensäuren und höher ungesättigten Typen) gezeigt.

Zur Herstellung langkettiger PUFAs müssen die mehrfach ungesättigten C₁₆ oder C₁₈-Fettsäuren durch die enzymatische Aktivität einer Elongase um mindestens zwei Kohlenstoffatome verlängert werden. Die erfindungsgemäße Nukleinsäuresequenz SEQ ID NO:1 codiert die erste Pflanzenelongase, die C₁₆ oder C₁₈-Fettsäuren mit mindestens zwei Doppelbindungen in der Fettsäure um mindestens zwei Kohlenstoffatome verlängern kann. Nach einer Elongationsrunde führt diese Enzymaktivität zu C₂₀-Fettsäuren, und nach zwei, drei und vier Elongationsrunden zu C₂₂-, C₂₄- oder C₂₆-Fettsäuren. Mit Hilfe der weiteren offenbarten Elongasen (SEQ ID NO:3, SEQ ID NO:5, SEQ ID NO:7, SEQ ID NO:9 und SEQ ID NO:11) können ebenfalls längerkettige PUFAs synthetisiert werden. Diese können einzeln, zu mehreren oder z.B. additiv zur PUFA Elongase aus dem Moos Physcomitrella patens (SEQ ID NO:1) zur Erhöhung des PUFA Gehaltes in einem neuen Verfahren zur Produktion von PUFAs eingesetzt werden. Die Aktivität der erfindungsgemäßen Elongasen führt vorzugsweise zu C₂₀-Fettsäuren mit mindestens zwei Doppelbindungen im Fettsäuremolekül, vorzugsweise mit drei oder vier Doppelbindungen, besonders bevorzugt drei Doppelbindungen im Fettsäuremolekül und/oder C₂₂-Fettsäuren mit mindestens zwei Doppelbindungen im Fettsäuremolekül, vorzugsweise mit vier, fünf oder sechs Doppelbindungen, besonders bevorzugt mit fünf oder sechs Doppelbindungen im Molekül. Nachdem die Verlängerung mit dem erfindungsgemäßen Enzym stattgefunden hat, können weitere Desaturierungsschritte erfolgen, um die hoch desaturierten Fettsäuren zu erhalten. Daher führen die Produkte der Elongaseaktivitäten und der möglichen weiteren Desaturierung zu bevorzugten PUFAs mit einem höheren Desaturierungsgrad, wie Docosadiensäure, Arachidonsäure, ω6-Eicosatriendihomo-γ-linolensäure, Eicosapentaensäure, ω3-Eicosatriensäure, ω3-Eicosatetraensäure, Docosapentaensäure oder Docosahexaensäure. Substrate der erfindungsgemäßen Enzymaktivität sind zum Beispiel Taxolsäure; 7,10,13-Hexadecatriensäure, 6,9-Octadecadiensäure, Linolsäure, Pinolensäure, α-oder γ-Linolensäure oder Stearidonsäure sowie Arachidonsäure, Eicosatetraensäure, Docosapentaensäure, Eicosapentaensäure. Bevorzugte Substrate sind Linolsäure, γ-Linolensäure und/oder α-Linolensäure sowie Arachidonsäure, Eicosatetraensäure, Docosapentaensäure, Eicosapentaensäure. Besonders bevorzugt sind Arachidonsäure, Docosapentaensäure, Eicosapentaensäure. Die C₁₆ oder C₁₈-Fettsäuren mit mindestens zwei Doppelbindungen in der Fettsäure können durch die erfindungsgemäße enzymatische Aktivität in Form der freien Fettsäure oder in Form der Ester, wie Phospholipide, Glycolipide, Sphingolipide, Phosphoglyceride, Monoacylglycerin, Diacylglycerin oder Triacylglycerin, verlängert werden.
Für die menschliche Ernährung ist konjugierte Linolsäure "CLA" von besonderer Bedeutung. Unter CLA versteht man insbesondere Fettsäuren wie C18:2 ^{9 cis, 11trans} oder das Isomer C18:2 ^{10trans, 12 cis}, die aufgrund menschlicher Enzymsysteme nach Aufnahme im Körper desaturiert bzw. elongiert werden können und zu gesundheitsfördernden Effekten beitragen können. Mit erfindungsgemäßen Elongasen können auch solche konjugierten Fettsäuren mit wenigstens zwei Doppelbindungen im Molekül elongiert werden und damit solche gesundheitsfördernden Fettsäuren der menschlichen Ernährung zugeführt werden. Weitere Beispiel für konjugierte Fettsäuren sind alpha-Parinarsäure, Eleostearinsäure und Calendulasäure.

Unter der Voraussetzung von Klonierungsvektoren zur Verwendung in Pflanzen und bei der Pflanzentransformation, wie denjenigen, die veröffentlicht sind in und dort zitiert sind: Plant Molecular Biology and Biotechnology (CRC Press, Boca Raton, Florida), Kapitel 6/7, S. 71-119 (1993) ; F. F. White, Vectors for Gene Transfer in Higher Plants; in: Transgenic Plants, Bd. 1, Engineering and Utilization, Hrsgb.: Kung und R. Wu, Academic Press, 1993, 15-38; B. Jenes et al., Techniques for Gene Transfer, in: Transgenic Plants, Bd. 1, Engineering and Utilization, Hrsgb.: Kung und R. Wu, Academic Press (1993), 128-143; Potrykus, Annu. Rev. Plant Physiol. Plant Molec. Biol. 42 (1991), 205-225)), lassen sich die erfindungsgemäßen Nukleinsäuren zur gentechnologischen Veränderung eines breiten Spektrums an Pflanzen verwenden, so daß diese ein besserer, effizienterer oder veränderter Produzent eines oder mehrerer von Lipiden hergeleiteter Produkte, wie PUFAs, wird. Diese verbesserte Produktion oder Effizienz der Produktion eines von Lipiden hergeleiteten Produktes, wie PUFAs, kann durch direkte Wirkung der Manipulation oder eine indirekte Wirkung dieser Manipulation hervorgerufen werden.

Es gibt eine Reihe von Mechanismen, durch die die Veränderung eines erfindungsgemäßen PSE-Proteins die Ausbeute, Produktion und/oder Effizienz der Produktion einer Feinchemikalie aus einer Ölfruchtpflanze oder einem Mikroorganismus aufgrund eines veränderten Proteins direkt beeinflussen kann. Die Anzahl oder Aktivität des PSE-Proteins oder -Gens kann erhöht sein, so daß größere Mengen dieser Verbindungen de novo hergestellt werden, weil den Organismen diese Aktivität und Fähigkeit zur Biosynthese vor dem Einbringen des entsprechenden Gens fehlte. Auch die Verwendung verschiedener divergenter, d.h. auf DNA-Sequenzebene unterschiedlicher Sequenzen kann dabei vorteilhaft sein.

Das Einbringeneines PSE-Gens oder mehrerer PSE-Gene in einen Organismus oder eine Zelle kann nicht nur den Biosynthesefluß zum Endprodukt erhöhen, sondern auch die entsprechende Triacylglycerin-Zusammensetzung erhöhen oder de novo schaffen. Ebenso kann die Anzahl oder Aktivität anderer Gene, die am Import von Nährstoffen, die zur Biosynthese einer oder mehrerer Feinchemikalien (z.B. Fettsäuren, polaren und neutralen Lipiden) nötig sind, erhöht sein, so daß die Konzentration dieser Vorläufer, Cofaktoren oder Zwischenverbindungen innerhalb der Zellen oder innerhalb des Speicherkompartiments erhöht ist, wodurch die Fähigkeit der Zellen zur Produktion von PUFAs, wie im folgenden beschrieben, weiter gesteigert wird. Fettsäuren und Lipide sind selbst als Feinchemikalien wünschenswert; durch Optimierung der Aktivität oder Erhöhung der Anzahl einer oder mehrerer PSEs, die an der Biosynthese dieser Verbindungen beteiligt sind, oder durch Zerstören der Aktivität einer oder mehrerer PSEs, die am Abbau dieser Verbindungen beteiligt sind, kann es möglich sein, die Ausbeute, Produktion und/oder Effizienz der Produktion von Fettsäure- und Lipidmolekülen aus Pflanzen oder Mikroorganismen zu steigern.

Die Mutagenese des erfindungsgemäßen PSE-Gens kann auch zu einem PSE-Protein mit geänderten Aktivitäten führen, welche die Produktion einer oder mehrerer gewünschter Feinchemikalien direkt oder indirekt beeinflussen. Beispielsweise kann die Anzahl oder Aktivität des erfindungsgemäßen PSE-Gene gesteigert werden, so daß die normalen Stoffwechselabfälle oder -nebenprodukte der Zelle (deren Menge möglicherweise aufgrund der Überproduktion der gewünschten Feinchemikalie erhöht ist) effizient exportiert werden, bevor sie andere Moleküle oder Prozesse innerhalb der Zelle (welche die Lebensfähigkeit der Zelle senken würden) zerstören oder die Biosynthesewege der Feinchemikalie stören würden (wodurch die Ausbeute, Produktion oder Effizienz der Produktion der gewünschten Feinchemikalie verringert wird). Ferner können die relativ großen intrazellulären Mengen der gewünschten Feinchemikalie selbst toxisch für die Zelle sein oder Enzym-Rückkopplungsmechanismen, wie die allosterische Regulation, stören, beispielsweise könnte sie durch Steigerung der Aktivität oder Anzahl anderer stromabwärts folgender Enzyme oder Entgiftungsenzyme des PUFA-Wegs die Allokation der PUFA in die Triacylgylcerin-Fraktion steigern, man könnte die Lebensfähigkeit von Saatzellen erhöhen, was wiederum zu besserer Entwicklung von Zellen in Kultur oder zu Saaten führt, die die gewünschte Feinchemikalie produzieren. Das erfindungsgemäße PSE-Gen kann auch so manipuliert werden, daß die entsprechenden Mengen der verschiedenen Lipid- und Fettsäuremoleküle hergestellt werden. Dies kann eine einschneidende Wirkung auf die Lipidzusammensetzung der Membran der Zelle haben und erzeugt neue Öle zusätzlich zum Auftreten neusynthetisierter PUFAs. Da jeder Lipidtyp unterschiedliche physikalische Eigenschaften hat, kann eine Veränderung der Lipidzusammensetzung einer Membran die Membranfluidität erheblich verändern. Änderungen der Membranfluidität können sich auf den Transport von Molekülen über die Membran sowie auf die Unversehrtheit der Zelle auswirken, die beide eine entscheidende Wirkung auf die Produktion von Feinchemikalien besitzen. In Pflanzen können diese Änderungen überdies auch andere Merkmale, wie Toleranz gegenüber abiotischen und biotischen Streßsituationen, beeinflussen.

Biotische und abiotische Streßtoleranz ist ein allgemeines Merkmal, das man an ein breites Spektrum von Pflanzen, wie Mais, Weizen, Roggen, Hafer, Triticale, Reis, Gerste, Sojabohne, Erdnuß, Baumwolle, Raps und Canola, Maniok, Pfeffer, Sonnenblume und Tagetes, Solanaceen-Pflanzen, wie Kartoffel, Tabak, Aubergine und Tomate, Vicia-Arten, Erbse, Alfalfa, Buschpflanzen (Kaffee, Kakao, Tee), Salix-Arten, Bäume (Ölpalme, Kokosnuß) und ausdauernde Gräser und Futterfeldfrüchte,vererben möchte. Diese Feldfrüchte sind als weitere erfindungsgemäße Ausführungsform auch bevorzugte Zielpflanzen für die Gentechnologie. Besonders bevorzugte erfindungsgemäße Pflanzen sind Ölfruchtpflanzen, wie Sojabohne, Erdnuß, Raps, Canola, Sonnenblume, Safflor, Bäume (Ölpalme, Kokosnuß) oder Feldfrüchte, wie Mais, Weizen, Roggen, Hafer, Triticale, Reis, Gerste, Alfalfa, oder Buschpflanzen (Kaffee, Kakao, Tee).

Folglich betrifft ein Aspekt der Erfindung isolierte Nukleinsäuremoleküle (z.B. cDNAs), umfassend Nukleotidsequenzen, die eine PSE oder mehrere PSEs oder biologisch aktive Teile davon codieren, oder Nukleinsäurefragmente, die sich als Primer oder Hybridisierungssonden zum Nachweis oder zur Amplifikation PSE-codierender Nukleinsäuren (z.B. DNA oder mRNA) eignen. Bei besonders bevorzugten Ausführungsformen umfaßt das Nukleinsäuremolekül eine der in dargestellten Nukleotidsequenzenoder die codierende Region oder ein Komplement einer dieser Nukleotidsequenzen. Bei anderen besonders bevorzugten Ausführungsformen umfaßt das erfindungsgemäße isolierte Nukleinsäuremolekül eine Nukleotidsequenz, die an eine Nukleotidsequenz, wie in der Sequenz SEQ ID NO:1, SEQ ID NO:3, und SEQ ID NO:9 dargestellt, oder einen Teil davon hybridisiert oder zu mindestens etwa 50 %, vorzugsweise mindestens etwa 60 %, stärker bevorzugt mindestens etwa 70 %, 80 % oder 90 % und noch stärker bevorzugt mindestens etwa 95 %, 96 %, 97 %, 98 %, 99 % oder mehr dazu ist. Bei anderen bevorzugten Ausführungsformen codiert das isolierte Nukleinsäuremolekül eine der in der Sequenz dargestellten Aminosäuresequenzen. Das bevorzugte erfindungsgemäße PSE-Gen besitzt vorzugsweise auch mindestens eine der hier beschriebenen PSE-Aktivitäten.

Bei einer weiteren Ausführungsform codiert das isolierte Nukleinsäuremolekül ein Protein oder einen Teil davon, wobei das Protein oder der Teil davon eine Aminosäuresequenz enthält, die ausreichend homolog zu einer Aminosäuresequenz der Sequenz SEQ ID NO:2, SEQ ID NO:4, und SEQ ID NO:10 ist, daß das Protein oder der Teil davon eine PSE-Aktivität beibehält. Vorzugsweise behält das Protein oder der Teil davon, das/der von dem Nukleinsäuremolekül codiert wird, die Fähigkeit, am Stoffwechsel von zum Aufbau von Zellmembranen von Pflanzen notwendigen Verbindungen oder am Transport von Molekülen über diese Membranen teilzunehmen. Bei einer Ausführungsform ist das von dem Nukleinsäuremolekül codierte Protein zu mindestens etwa 50 %, vorzugsweise mindestens etwa 60 % und stärker bevorzugt mindestens etwa 70 %, 80 % oder 90 % und am stärksten bevorzugt mindestens etwa 95 %, 96 %, 97 %, 98 %, 99 % oder mehr homolog zu einer Aminosäuresequenz der Sequenz SEQ ID NO:2, SEQ ID NO:4, und SEQ ID NO:10. Bei einer weiteren bevorzugten Ausführungsform ist das Protein ein Vollängen-Protein, das im wesentlichen in Teilen homolog zu einer gesamten Aminosäuresequenz der SEQ ID NO:2, SEQ ID NO:4, und SEQ ID NO:10 (die von dem in SEQ ID NO:1, SEQ ID NO:3, und SEQ ID NO:9 gezeigten offenen Leserahmen herrührt) ist und in Vollänge mit dem Fachmann geläufigen Methoden und Experimenten isolierbar ist.

Bei einer anderen bevorzugten Ausführungsform rührt das isolierte Nukleinsäuremolekül von Phytophthora infestans, Physcomitrella patens, oder Thraustochytrium her und codiert ein Protein (z.B. ein PSE-Fusionsprotein), das eine biologisch aktive Domäne enthält, die zu mindestens etwa 50 % oder mehr homolog zu einer Aminosäuresequenz der sequenz SEQ ID NO:2, SEQ ID NO:4, und SEQ ID NO:10 ist und die Fähigkeit, am Stoffwechsel von zum Aufbau von Zellmembranen von Pflanzen notwendigen Verbindungen oder am Transport von Molekülen über diese Membranen teilzunehmen, beibehält oder zumindest eine der Elongationsaktivitäten resultierend in PUFAs wie ARA, EPA oder DHA oder deren Vorläufermoleküle oder eine der in der Tabelle 1 aufgeführten Aktivitäten besitzt, und umfaßt auch heterologe Nukleinsäuresequenzen, die ein heterologes Polypeptid oder regulatorische Proteine codieren.

**Tabelle 1: Fettsäuremuster von fünf transgenen Hefestämmen in Mol.-%. Die Anteile der zugeführten und aufgenommenen g-Linolensäure sind durch fettgedruckte Zahlen hervorgehoben, die der verlängerten Produkte sind unterstrichen und die der verlängerten γ-Linolensäure durch fettgedruckte Zahlen (letzte Zeile).**

| Fettsäuren [Mol.-%] | pYES2 | pY2PSE1a | pY2PSE1b | pY2PSE1c | pY2PSE1d |
|---|---|---|---|---|---|
| 16:0 | 17,0 | 17,6 | 16,4 | 16,3 | 17,6 |
| 16:1Δ⁹ | 28,0 | 26,8 | 28,0 | 27,9 | 25,1 |
| 18:0 | 6,5 | 6,0 | 6,4 | 5,6 | 6,1 |
| 18:1Δ⁹ | 25,9 | 23,5 | 27,0 | 25,2 | 21,4 |
| 18:3Δ^{6,9,12} | 22,6 | 15,7 | 13,2 | 16,4 | 22,8 |
| 20:3Δ^{8,11,14} | - | 10,3 | 9,0 | 8,6 | 7,1 |
| 18:3Δ^{6,9,12}- Elongation | - | 39,6 | 40,5 | 34,4 | 23,7 |

Bei einer anderen Ausführungsform ist das isolierte Nukleinsäuremolekül mindestens 15 Nukleotide lang und hybridisiert unter stringenten Bedingungen an ein Nukleinsäuremolekül, das eine Nukleotidsequenz der SEQ ID NO:1, SEQ ID NO:3, und SEQ ID NO:9 enthält. Vorzugsweise entspricht das isolierte Nukleinsäuremolekül einem natürlich vorkommenden Nukleinsäuremolekül. Stärker bevorzugt codiert das isolierte Nukleinsäuremolekül natürlich vorkommende Phytophthora- oder Thraustochytrium-PSE oder einen biologisch aktiven Teil davon.

Ein weiterer Aspekt der Erfindung betrifft Vektoren, z.B. rekombinante Expressionsvektoren, die mindestens ein erfindungsgemäßes Nukleinsäuremolekül enthalten, und Wirtszellen, in die diese Vektoren eingebracht worden sind, insbesondere Mikroorganismen, Pflanzenzellen, Pflanzengewebe, -organe oder ganze Pflanzen. Bei einer Ausführungsform kann eine solche Wirtszelle Feinchemikalien-Verbindungen, insbesondere PUFAs, speichern; zur Isolation der gewünschten Verbindung werden die Zellen geerntet. Die Verbindung (Öle, Lipide, Triacylglyceride, Fettsäuren) oder die PSE können dann aus dem Medium oder der Wirtszelle, welche bei Pflanzen Zellen sind, die Feinchemikalien enthalten oder speichern, am stärksten bevorzugt Zellen von Speichergeweben, wie Samenhüllen, Knollen, Epidermis- und Samenzellen, isoliert werden.

Noch ein weiterer Aspekt der Erfindung betrifft eine genetisch veränderte Pflanze, bevorzugt eine Ölfruchtpflanze, wie vorstehend erwähnt, besonders bevorzugt eine Raps-, Lein- oder Physcomitrella patens-Pflanze, in die ein PSE-Gen eingebracht worden ist. Bei einer Ausführungsform ist das Genom von Raps, Lein oder Physcomitrella patens durch Einbringen eines erfindungsgemäßen Nukleinsäuremoleküls, das eine Wildtyp- oder mutierte PSE-Sequenz codiert, als Transgen verändert worden. Bei einer anderen Ausführungsform ist ein endogenes PSE-Gen im Genom der Spenderorganismen Physcomitrella patens, Phytophthora infestans, oder Thraustochytrium beispielsweise durch homologe Rekombination mit einem veränderten PSE-Gen oder Mutagenese und Detektion mittels DNA-Sequenzen verändert, das heißt funktionell zerstört worden. Bei einer bevorzugten Ausführungsform gehört der Pflanzenorganismus zur Gattung Physcomitrella, Ceratodon, Funaria, Raps oder Lein, wobei Physcomitrella, Raps oder Lein bevorzugt ist. Bei einer bevorzugten Ausführungsform wird Physcomitrella, Raps oder Lein auch zur Produktion einer gewünschten Verbindung, wie Lipiden und Fettsäuren, wobei PUFAs besonders bevorzugt sind, verwendet.

Bei noch einer weiteren bevorzugten Ausführungsform kann das Moos Physcomitrella patens zur Demonstration der Funktion eines Elongasegens unter Verwendung homologer Rekombination auf der Basis der in dieser Erfindung beschriebenen Nukleinsäuren verwendet werden.

Noch ein weiterer Aspekt der Erfindung betrifft ein isoliertes PSE-Gen oder einen Teil, z.B. einen biologisch aktiven Teil, davon. Bei einer bevorzugten Ausführungsform kann die isolierte PSE oder ein Teil davon am Stoffwechsel von zum Aufbau von Zellmembranen in einem Mikroorganismusoder einer Pflanzenzelle not wendigen Verbindungen oder am Transport von Molekülen über dessen/deren Membranen teilnehmen. Bei einer weiteren bevorzugten Ausführungsform ist die isolierte PSE oder der Teil davon ausreichend homolog zu einer Aminosäuresequenz der SEQ ID NO:2, SEQ ID NO:4, und SEQ ID NO:10 das dieses Protein oder der Teil davon die Fähigkeit, am Stoffwechsel von zum Aufbau von Zellmembranen in Mikroorganismen oder Pflanzenzellen notwendigen Verbindungen oder am Transport von Molekülen über diese Membranen teilzunehmen, beibehält.

Die Erfindung stellt auch eine isolierte Präparation einer PSE bereit. Bei bevorzugten Ausführungsformen umfaßt das PSE-Gen eine Aminosäuresequenz der SEQ ID NO:2, SEQ ID NO:4, und SEQ ID NO:10. Bei einer weiteren bevorzugten Ausführungsform betrifft die Erfindung ein isoliertes Vollängenprotein, das im wesentlichen homolog zu einer gesamten Aminosäuresequenz der SEQ ID NO:2, SEQ ID NO:4, und SEQ ID NO:10 (die von den in SEQ ID NO:1, SEQ ID NO:3, und SEQ ID NO:9 gezeigten offenen Leserahmen codiert werden) ist. Bei einer weiteren Ausführungsform ist das Protein zu mindestens etwa 50 %, vorzugsweise mindestens etwa 60 % und stärker bevorzugt mindestens etwa 70 %, 80 % oder 90 % und am stärksten bevorzugt mindestens etwa 95 %, 96 %, 97 %, 98%, 99 % oder mehr homolog zu einer Aminosäuresequenz der Sequenz SEQ ID NO:2, SEQ ID NO:4, und SEQ ID NO:10. Bei anderen Ausführungsformen umfaßt die isolierte PSE eine Aminosäuresequenz, die zu mindestens etwa 50 % homolog zu einer der Aminosäuresequenzen SEQ ID NO:2, SEQ ID NO:4, und SEQ ID NO:10 ist und am Stoffwechsel von zum Aufbau von Fettsäuren in einem Mikroorganismus oder einer Pflanzenzelle notwendigen Verbindungen oder am Transport von Molekülen über diese Membranen teilnehmen kann oder eine oder mehrere der PUFA-verlängernden Aktivitäten hat, wobei die Verlängerung vorteilhaft von desaturierten C₁₆ oder C₁₈- oder C₂₀-Kohlenstoffketten mit Doppelbindungen an mindestens zwei Stellen erfolgt.

Alternativ kann die isolierte PSE eine Aminosäuresequenz umfassen, die von einer Nukleotidsequenz codiert wird, die an eine Nukleotidsequenz der SEQ ID NO:1, SEQ ID NO:3, und SEQ ID NO:9 hybridisiert, z.B. unter stringenten Bedingungen hybridisiert, oder zu mindestens etwa 50 %, vorzugsweise mindestens etwa 60 %, stärker bevorzugt mindestens etwa 70 %, 80 % oder 90 % und noch stärker bevorzugt mindestens etwa 95 %, 96 %, 97 %, 98 %, 99 % oder mehr homolog dazu ist. Es ist ebenfalls bevorzugt, daß die bevorzugten PSE-Formen ebenfalls eine der hier beschriebenen PSE-Aktivitäten besitzen.

Das PSE-Polypeptid oder ein biologisch aktiver Teil davon kann funktionsfähig mit einem nicht-PSE-Polypeptid verbunden werden, so daß ein Fusionsprotein gebildet wird. Bei bevorzugten Ausführungsformen hat dieses Fusionsprotein eine Aktivität, die sich von derjenigen der PSE allein unterscheidet. Bei anderen bevorzugten Ausführungsformen nimmt dieses Fusionsprotein am Stoffwechsel von Verbindungen, die zur Synthese von Lipiden und Fettsäuren, Cofaktoren und Enzymen in Mikroorganismen oder Pflanzen notwendig sind, oder am Transport von Molekülen über diese Membranen teil. Bei besonders bevorzugten Ausführungsformen moduliert das Einbringen dieses Fusionsproteins in eine Wirtszelle die Produktion einer gewünschten Verbindung durch die Zelle. Bei einer bevorzugten Ausführungsform enthalten diese Fusionsproteine auch Δ4-, Δ5- oder Δ6-, Δ8-, Δ15-, Δ17- oder Δ19-Desaturase-Aktivitäten allein oder in Kombination.

Ein weiterer Aspekt der Erfindung betrifft ein Verfahren zur Herstellung einer Feinchemikalie. Dieses Verfahren beinhaltet entweder die Züchtung eines geeigneten Mikroorganismus oder die Züchtung von Pflanzenzellen, -geweben, -organen oder ganzen pflanzen, umfassend die erfindungsgemäßen Nukleotidsequenzen der SEQ ID NO:1, SEQ ID NO:3, und SEQ ID NO:9 oder ihre Homologen, Derivate oder Analoga oder ein Genkonstrukt, das die SEQ ID NO:1, SEQ ID NO:3, und SEQ ID NO:9 oder ihre Homologen, Derivate oder Analoga umfaßt, oder einen Vektor, der diese Sequenzen oder das Genkonstrukt umfaßt, welches die Expression eines erfindungsgemäßen PSE-Nukleinsäuremoleküls herbeiführt, so daß eine Feinchemikalie produziert wird. Bei einer bevorzugten Ausführungsform umfaßt das Verfahren ferner den Schritt des Gewinnens einer Zelle, die eine solche erfindungsgemäße Elongase-Nukleinsäuresequenz enthält, wobei eine Zelle mit einer Elongase-Nukleinsäuresequenz, einem Genkonstrukt oder einem Vektor, welche die Expression einer erfindungsgemäßen PSE-Nukleinsäure herbeiführen, transformiert wird. Bei einer weiteren bevorzugten Ausführungsform umfaßt dieses Verfahren ferner den Schritt des Gewinnens der Feinchemikalie aus der Kultur. Bei einer besonders bevorzugten Ausführungsform gehört die Zelle zur Ordnung der Ciliaten, zu Mikroorganismen, wie Pilzen, oder zum Pflanzenreich, insbesondere zu Ölfruchtpflanzen, besonders bevorzugt sind Mikroorganismen oder Ölfruchtpflanzen.

Ein weiterer Aspekt der Erfindung betrifft Verfahren zur Modulation der Produktion eines Moleküls durch einen Mikroorganismus. Diese Verfahren umfassen das Zusammenbringen der Zelle mit einer Substanz, welche die PSE-Aktivität oder die PSE-Nukleinsäureexpression moduliert, so daß eine zellassoziierte Aktivität relativ zu der gleichen Aktivität in Abwesenheit der Substanz verändert wird. Bei einer bevorzugten Ausführungsform wird/werden ein oder zwei Stoffwechselweg(e) der Zelle für Lipide und Fettsäuren, Cofaktoren und Enzyme moduliert oder der Transport von Verbindungen über diese Membranen moduliert, so daß die Ausbeute oder die Rate der Produktion einer gewünschten Feinchemikalie durch diesen Mikroorganismus verbessert ist. Die Substanz, welche die PSE-Aktivität moduliert, kann eine Substanz sein, welche die PSE-Aktivität oder PSE-Nukleinsäureexpression stimuliert oder die als Zwischenprodukt bei der Fettsäurebiosynthese verwendet werden kann. Beispiele für Substanzen, welche die PSE-Aktivität oder PSE-Nukleinsäureexpression stimulieren, sind u.a. kleine Moleküle, aktive PSEs sowie PSEs-codierende Nukleinsäuren, die in die Zelle eingebracht worden sind. Beispiele für Substanzen, welche die PSE-Aktivität oder -Expression hemmen, sind u.a. kleine Moleküle und/oder Antisense-PSE-Nukleinsäuremoleküle.

Ein weiterer Aspekt der Erfindung betrifft Verfahren zur Modulation der Ausbeuten einer gewünschten Verbindung aus einer Zelle, umfassend das Einbringen eines Wildtyp- oder Mutanten-PSE-Gens, das entweder auf einem separaten Plasmid gehalten oder in das Genom der Wirtszelle integriert wird, in eine Zelle. Bei Integration in das Genom kann die Integration zufallsgemäß sein oder durch derartige Rekombination erfolgen, daß das native Gen durch die eingebrachte Kopie ersetzt wird, wodurch die Produktion der gewünschten Verbindung durch die Zelle moduliert wird, oder durch Verwendung eines Gens in trans, so daß das Gen mit einer funktionellen Expressionseinheit, welche mindestens eine die Expression eines Gens gewährleistende Sequenz und mindestens eine die Polyadenylierung eines funktionell transkribierten Gens gewährleistende Sequenz enthält, funktionell verbunden ist.

Bei einer bevorzugten Ausführungsform sind die Ausbeuten modifiziert. Bei einer weiteren bevorzugten Ausführungsform wird die gewünschte Chemikalie vermehrt, wobei unerwünschte störende Verbindungen vermindert werden können. Bei einer besonders bevorzugten Ausführungsform ist die gewünschte Feinchemikalie ein Lipid oder eine Fettsäure, ein Cofaktor oder ein Enzym. Bei besonders bevorzugten Ausführungsform ist diese Chemikalie eine mehrfach ungesättigte Fettsäure. Stärker bevorzugt ist sie ausgewählt aus Arachidonsäure (= ARA), Eicosapentaensäure (= EPA) oder Docosahexaensäure (= DHA).

### Eingehende Beschreibung der Erfindung

Die vorliegende Erfindung stellt PSE-Nukleinsäuren und -Proteinmoleküle bereit, die am Stoffwechsel von Lipiden und Fettsäuren, PUFA-Cofaktoren und Enzymen in dem Moos Physcomitrella patens, Phytophthora infestans, oder Traustochytrium oder am Transport lipophiler Verbindungen über Membranen beteiligt sind. Die erfindungsgemäßen Verbindungen lassen sich zur Modulation der Produktion von Feinchemikalien aus Organismen, beispielsweise Mikroorganismen, wie Ciliaten, Pilzen, Hefen, Bakterien, Algen, und/oder Pflanzen, wie Mais, Weizen, Roggen, Hafer, Triticale, Reis, Gerste, Sojabohne, Erdnuß, Baumwolle, Brassica-Arten, wie Raps, Canola und Rübsen, Pfeffer, Sonnenblume, Borretsch, Nachtkerze und Tagetes, Solanaceen-Pflanzen, wie Kartoffel, Tabak, Aubergine und Tomate, Vicia-Arten, Erbse, Maniok, Alfalfa, Buschpflanzen (Kaffee, Kakao, Tee), Salix-Arten, Bäume (Ölpalme, Kokosnuß) und ausdauernden Gräsern und Futterfeldfrüchten, entweder direkt (z.B. wenn die Überexpression oder Optimierung eines Fettsäurebiosynthese-Proteins einen direkten Einfluß auf die Ausbeute, Produktion und/oder Effizienz der Produktion der Fettsäure aus modifizierten Organismen hat) verwenden oder können eine indirekt Auswirkung haben, die dennoch zu einer Steigerung der Ausbeute, Produktion und/oder Effizienz der Produktion einer gewünschten Verbindung oder einer Abnahme unerwünschter Verbindungen führt (z.B. wenn die Modulation des Stoffwechsels von Lipiden und Fettsäuren, Cofaktoren und Enzymen zu Veränderungen der Ausbeute, Produktion und/oder Effizienz der Produktion oder der Zusammensetzung der gewünschten Verbindungen innerhalb der Zellen führt, was wiederum die Produktion einer oder mehrerer Feinchemikalien beeinflussen kann). Aspekte der Erfindung sind nachstehend weiter erläutert.

### I. Feinchemikalien und PUFAs

Der Begriff "Feinchemikalie" ist im Fachgebiet bekannt und umfaßt Moleküle, die durch einen Organismus produziert worden sind und Anwendungen in verschiedenen Industrien finden, wie, aber nicht beschränkt auf, die pharmazeutische, Landwirtschafts-, Nahrungsmittel- und Kosmetik-Industrie. Diese Verbindungen umfassen Lipide, Fettsäuren, Cofaktoren und Enzyme usw. (wie z.B. beschrieben in Kuninaka, A. (1996) Nucleotides and related compounds, S. 561-612, in Biotechnology Bd. 6, Rehm et al., Hrsgb., VCH: Weinheim und darin enthaltenen Literaturstellen), Lipide, gesättigte und ungesättigte Fettsäuren (z.B. Arachidonsäure), Vitamine und Cofaktoren (wie beschrieben in Ullmann's Encyclopedia of Industrial Chemistry, Bd. A27, Vitamins, S. 443-613 (1996) VCH: Weinheim und darin enthaltenen Literaturstellen; und Ong, A. S., Niki, E., & Packer, L. (1995) Nutrition, Lipids, HealthandDisease Proceedings of the UNESCO/Confederation of Scientific and Technological Associations in Malaysia and the Society for Free Radical Research - Asien, abgehalten am 1.-3. Sept. 1994 in Penang, Malaysia, AOCS Press (1995)), Enzyme und sämtliche anderen von Gutcho (1983) in Chemicals by Fermentation, Noyes Data Corporation, ISBN:0818805086, und darin angegebenen Literaturstellen beschriebenen Chemikalien. Der Stoffwechsel und die Verwendungen bestimmter Feinchemikalien sind nachstehend weiter erläutert.

Die Kombination verschiedener Vorläufermoleküle und Biosyntheseenzyme führt zur Herstellung verschiedener Fettsäuremoleküle, was eine entscheidende Auswirkung auf die Zusammensetzung der Membran hat. Es kann angenommen werden, daß PUFAs nicht nur einfach in Triacylglycerin, sondern auch in Membranlipide eingebaut werden.

Die Synthese von Membranen ist ein gut charakterisierter Prozeß, an dem eine Anzahl von Komponenten, einschließlich Lipiden als Teil der Bilayer-Membran, beteiligt sind. Die Produktion neuer Fettsäuren, wie PUFAs, kann daher neue Eigenschaften von Membranfunktionen innerhalb einer Zelle oder eines Organismus erzeugen.

Zellmembranen dienen einer Vielzahl von Funktionen in einer Zelle. Zuerst und in erster Linie grenzt eine Membran den Inhalt einer Zelle von der Umgebung ab, wodurch sie der Zelle Integrität verleiht. Membranen können auch als Schranken gegenüber dem Einstrom gefährlicher oder unerwünschter Verbindungen und auch gegenüber dem Ausstrom gewünschter Verbindungen dienen.

Detailliertere Beschreibungen und Beteiligungen von Membranen und die beteiligten Mechanismen siehe in: Bamberg, E., et al. (1993) Charge transport of ion pumps on lipid bilayer membranes, Q. Rev. Biophys. 26:1-25; Gennis, R.B. (1989) Pores, Channels and Transporters, in: Biomembranes, Molecular Structure and Function, Springer: Heidelberg, S. 270-322; und Nikaido, H., und Saier, H. (1992) Transport proteins in bacteria: common themes in their design, Science 258:936-942, und den in jeder dieser Literaturstellen enthaltenen Zitaten.

Die Lipidsynthese läßt sich in zwei Abschnitte unterteilen: die Synthese von Fettsäuren und ihre Bindung an sn-Glycerin-3-Phosphat sowie die Addition oder Modifikation einer polaren Kopfgruppe. Übliche Lipide, die in Membranen verwendet werden, umfassen Phospholipide, Glycolipide, Sphingolipide und Phosphoglyceride. Die Fettsäuresynthese beginnt mit der Umwandlung von Acetyl-CoA entweder in Malonyl-CoA durch die Acetyl-CoA-Carboxylase oder in Acetyl-ACP durch die Acetyltransacylase. Nach einer Kondensationsreaktion bilden diese beiden Produktmoleküle zusammen Acetoacetyl-ACP, das über eine Reihe von Kondensations-, Reduktions- und Dehydratisierungsreaktionen umgewandelt wird, so daß ein gesättigtes Fettsäuremolekül mit der gewünschten Kettenlänge erhalten wird. Die Produktion der ungesättigten Fettsäuren aus diesen Molekülen wird durch spezifische Desaturasen katalysiert, und zwar entweder aerob mittels molekularem Sauerstoff oder anaerob (bezüglich der Fettsäuresynthese in Mikroorganismen siehe F. C. Neidhardt et al. (1996) E. coli und Salmonella. ASM Press: Washington, D.C., S. 612-636 und darin enthaltene Literaturstellen; Lengeler et al. (Hrsgb.) (1999) Biology of Procaryotes. Thieme: Stuttgart, New York, und die enthaltene Literaturstellen, sowie Magnuson, K., et al. (1993) Microbiological Reviews 57:522-542 und die enthaltenen Literaturstellen).

Vorläufer für die PUFA-Biosynthese sind beispielsweise Linol- und Linolensäure. Diese C₁₈-Kohlenstoff-Fettsäuren müssen auf C₂₀ oder C₂₂ verlängert werden, damit Fettsäuren vom Eicosa- und Docosa-Kettentyp erhalten werden. Mit Hilfe verschiedener Desaturasen, wie Enzymen, welche Δ6-Desaturasen-, Δ5- und Δ4-Desaturaseaktivität aufweisen, können Arachidonsäure, Eicosapentaensäure und Docosahexaensäure sowie verschiedene andere langkettige PUFAs erhalten, extrahiert und für verschiedene Zwecke bei Nahrungsmittel-, Futter-, Kosmetik- oder pharmazeutischen Anwendungen verwendet werden.

Zur Herstellung langkettiger PUFAs müssen, wie oben erwähnt, die mehrfach ungesättigten C₁₆ oder C₁₈- bzw C₂₀-Fettsäuren um mindestens zwei Kohlenstoffatome durch die Enzymaktivität einer Elongase verlängert werden. Die erfindungsgemäßen Nukleinsäuresequenzen codieren erste mikrobielle Elongasen aus typischen PUFA in der Triacylglycerolfraktion enthaltenden Produzenten, die C₁₆ oder C₁₈- bzw C₂₀-Fettsäuren mit mindestens zwei Doppelbindungen in der Fettsäure um mindestens zwei Kohlenstoffatome verlängern können oder dies z.B. sequentiell nacheinander durch Überführen einer C₁₆ oder C₁₈-Fettsäure in eine C₂₀-Fettsäure und dann eine C₂₀- in eine C₂₂ oder höhere geradzahlinge 2C-Atomeinheiten enthaltende Fettsäure überführt. Nach einer Elongationsrunde führt diese Enzymaktivität zu C₂₀-Fettsäuren, und nach zwei, drei und vier Elongationsrunden zu C₂₂-, C₂₄- oder C₂₆-Fettsäuren. Mit der erfindungsgemäßen Elongase können auch längere PUFAs synthetisiert werden. Die Aktivität der erfindungsgemäßen Elongasen führt vorzugsweise zu C₂₀- und/oder C₂₂-Fettsäuren mit mindestens zwei Doppelbindungen im Fettsäuremolekül, C₂₀-Fettsäuren vorzugsweise mit drei, vier oder fünf Doppelbindungen, besonders bevorzugt drei Doppelbindungen im Fettsäuremolekül, C₂₂-Fettsäuren vorzugsweise mit drei, vier fünf oder sechs Doppelbindungen, besonders bevorzugt fünf oder sechs Doppelbindungen im Fettsäuremolekül. Nach der Elongation mit dem erfindungsgemäßen Enzym können weitere Desaturierungsschritte erfolgen. Daher führen die Produkte der Elongaseaktivitäten und der möglichen weiteren Desaturierung zu bevorzugten PUFAs mit höherem Desaturierungsgrad, wie Docosadiensäure, Arachidonsäure, ω6-Eicosatriendihomo-γ-linolensäure, Eicosapentaensäure, ω3-Eicosatriensäure, ω3-Eicosatetraensäure, Docosapentaensäure oder Docosahexaensäure. Substrate dieser erfindungsgemäßen Enzymaktivität sind zum Beispiel Taxolsäure, 7,10,13-Hexadecatriensäure, 6,9-Octadecadiensäure, Linolsäure, γ-Linolensäure, Pinolensäure, α-Linolensäure, Arachidonsäure, Eicosapentaensäure oder Stearidonsäure. Bevorzugte Substrate sind Linolsäure, γ-Linolensäure und/oder α-Linolensäure bzw Arachidonsäure, Eicosatetraensäure oder Eicosapentaensäure. Die C₁₆ oder C₁₈-oder C₂₀-Fettsäuren mit mindestens zwei Doppelbindungen in der Fettsäure können durch die erfindungsgemäße Enzymaktivität in Form der freien Fettsäure oder in Form der Ester, wie Phospholipide, Glykolipide, Sphingolipide, Phosphoglyceride, Monoacylglycerin, Diacylglycerin oder Triacylglycerin, verlängert werden.

Ferner müssen Fettsäuren anschließend an verschiedene Modifikationen transportiert und in das Triacylglycerin-Speicherlipid eingebaut werden. Ein weiterer wichtiger Schritt bei der Lipidsynthese ist der Transfer von Fettsäuren auf die polaren Kopfgruppen, beispielsweise durchGlycerin-Fettsäure-Acyltransferase (siehe Frentzen, 1998, Lipid, 100(4-5):161-166).

Veröffentlichungen über die Pflanzen-Fettsäurebiosynthese, Desaturierung, den Lipidstoffwechsel und Membrantransport von fetthaltigen Verbindungen, die Betaoxidation, Fettsäuremodifikation und Cofaktoren, Triacylglycerin-Speicherung und -Assemblierung einschließlich der Literaturstellen darin siehe in den folgenden Artikeln: Kinney, 1997, Genetic Engeneering, Hrsgb.: JK Setlow, 19:149-166; Ohlrogge und Browse, 1995, Plant Cell 7:957-970; Shanklin und Cahoon, 1998, Annu. Rev. Plant Physiol. Plant Mol. Biol. 49:611-641; Voelker, 1996, Genetic Engeneering, Hrsgb.: JK Setlow, 18:111-13; Gerhardt, 1992, Prog. Lipid R. 31:397-417; Gühnemann-Schäfer & Kindl, 1995, Biochim. Biophys Acta 1256:181-186: Kunau et al., 1995, Prog. Lipid Res. 34:267-342; Stymne et al., 1993, in: Biochemistry and Molecular Biology of Membrane and Storage Lipids of Plants, Hrsgb.: Murata und Somerville, Rockville, American Society of Plant Physiologists, 150-158, Murphy & Ross 1998, Plant Journal. 13(1):1-16.

Vitamine, Cofaktoren und "*Nutrazeuticals",* wie PUFAs, umfassen eine Gruppe von Molekülen, die höhere Tiere nicht mehr synthetisieren können und somit aufnehmen müssen oder die höhere Tiere nicht mehr ausreichend selbst herstellen können und somit zusätzlich aufnehmen müssen, obwohl sie leicht von anderen Organismen, wie Bakterien, synthetisiert werden. Die Biosynthese dieser Moleküle in Organismen, die sie produzieren können, wie in Bakterien, ist im großen und ganzen charakterisiert worden (Ullmann's Encyclopedia of Industrial Chemistry, "Vitamins", Bd. A27, S. 443-613, VCH: Weinheim, 1996; Michal, G. (1999) Biochemical Pathways: An Atlas of Biochemistry and Molecular Biology, John Wiley & Sons; Ong, A.S., Niki, E., & Packer, L. (1995) "Nutrition, Lipids, Health and Disease" Proceedings of the UNESCO/Confederation of Scientific and Technological Associations in Malaysia and the Society for Free Radical Research Asia, abgehalten am 1.-3. Sept. 1994 in Penang, Malaysia, AOCS Press, Champaign, IL X, 374 S).

Die oben erwähnten Moleküle sind entweder selbst biologisch aktive Moleküle oder Vorstufen biologisch aktiver Substanzen, die entweder als Elektronenüberträger oder Zwischenprodukte bei einer Vielzahl von Stoffwechselwegen dienen. Diese Verbindungen haben neben ihrem Nährwert auch einen signifikanten industriellen Wert als Farbstoffe, Antioxidantien und Katalysatoren oder andere Verarbeitungshilfsstoffe. (Einen Überblick über Struktur, Aktivität und industrielle Anwendungen dieser Verbindungen siehe z. B. in Ullmann' s Encyclopedia of Industrial Chemistry, "Vitamins", Bd. A27, S. 443-613, VCH: Weinheim, 1996). Mehrfach ungesättigte Fettsäuren haben verschiedene Funktionen und gesundheitsfördernde Wirkungen, beispielsweise bei koronarer Herzerkrankung, Entzündungsmechanismen, Kinderernährung usw. Veröffentlichungen und Literaturstellen, einschließlich darin zitierter Literaturstellen, siehe in: Simopoulos, 1999, Am. J. Clin. Nutr. 70 (3. Suppl.):560-569, Takahata et al., Biosc. Biotechnol. Biochem. 1998, 62(11):2079-2085, Willich und Winther, 1995, Deutsche Medizinische Wochenschrift 120(7):229ff.

### II. Elemente und Verfahren der Erfindung

Die vorliegende Erfindung beruht zumindest teilweise auf der Entdeckung neuer Moleküle, die hier als PSE-Nukleinsäure- und -Proteinmoleküle bezeichnet werden, welche eine Wirkung auf die Produktion von Zellmembranen in Physcomitrella patens, Crypthecodinium cohnii, Phytophthora infestans, Thraustochytrium und/oder Ceratodon purpureus ausüben und beispielsweise die Bewegung von Molekülen über diese Membranen beeinflussen. Bei einer Ausführungsform nehmen die PSE-Moleküle am Stoffwechsel von zum Aufbau von Zellmembranen in Organismen, wie Mikroorganismen und Pflanzen, notwendigen Verbindungen teil oder beeinflussen indirekt den Transport von Molekülen über diese Membranen. Bei einer bevorzugten Ausführungsform hat die Aktivität der erfindungsgemäßen PSE-Moleküle zur Regulation der Produktion von Membrankomponenten und des Membrantransports eine Auswirkung auf die Produktion der gewünschten Feinchemikalie durch diesen Organismus. Bei einer besonders bevorzugten Ausführungsform ist die Aktivität der erfindungsgemäßen PSE-Moleküle moduliert, so daß die Ausbeute, Produktion und/oder Effizienz der Produktion der Stoffwechselwege von Mikroorganismen oder Pflanzen, welche die erfindungsgemäßen PSEs regulieren, moduliert sind und die Effizienz des Transport von Verbindungen durch die Membranen verändert ist, was entweder direkt oder indirekt die Ausbeute, Produktion und/oder Effizienz der Produktion einer gewünschten Feinchemikalie durch Mikroorganismen und Pflanzen moduliert.

Der Begriff PSE oder PSE-Polypeptidumfaßt Proteine, die amStoffwechsel von zum Aufbau von Zellmembranen in Organismen, wie Mikroorganismen und Pflanzen, notwendigen Verbindungen oder am Transport von Molekülen über diese Membranen teilnehmen. Beispiele für PSEs sind in der SEQ ID NO:1, SEQ ID NO:3, SEQ ID NO: 5, SEQ ID NO:7, SEQ ID NO: 9 und SEQ ID NO: 11 oder ihren Homologen, Derivaten oder Analoga offenbart. Die Begriffe PSE oder PSE-Nukleinsäuresequenz (en) umfassen Nukleinsäuresequenzen, die eine PSE codieren und bei denen ein Teil eine codierende Region und ebenfalls entsprechende 5'- und 3' -untranslatierte Sequenzbereiche sein können. Beispiele für PSE-Gene sind die in SEQ ID NO:1, SEQ ID NO:3, SEQ ID NO:5, SEQ ID NO:7, SEQ ID NO:9 und SEQ ID NO:11 dargestellten Sequenzen. Die Begriffe Produktion oder Produktivität sind im Fachgebiet bekannt und beinhalten die Konzentration des Fermentationsproduktes (zum Beispiel der gewünschten Feinchemikalie), das in einer bestimmten Zeitspanne und einem bestimmten Fermentationsvolumen gebildet wird (z.B. kg Produkt pro Stunde pro Liter). Der Begriff Effizienz der Produktion umfaßt die Zeit, die zur Erzielung einer bestimmten Produktionsmenge nötig ist (z.B. wie lange die Zelle zur Aufrichtung einer bestimmten Durchsatzrate einer Feinchemikalie benötigt). Der Begriff Ausbeute oder Produkt/Kohlenstoff-Ausbeute ist im Fachgebiet bekannt und umfaßt die Effizienz der Umwandlung der Kohlenstoffquelle in das Produkt (d.h. die Feinchemikalie). Dies wird gewöhnlich beispielsweise ausgedrückt als kg Produkt pro kg Kohlenstoffquelle. Durch Erhöhen der Ausbeute oder Produktion der Verbindung wird die Menge der gewonnenen Moleküle oder der geeigneten gewonnenen Moleküle dieser Verbindung in einer bestimmten Kulturmenge über einen festgelegten Zeitraum erhöht. Die Begriffe Biosynthese oder Biosyntheseweg sind im Fachgebiet bekannt und umfassen die Synthese einer Verbindung, vorzugsweise einer organischen Verbindung, durch eine Zelle aus Zwischenverbindungen, beispielsweise in einem Mehrschritt- und stark regulierten Prozeß. Die Begriffe Abbau oder Abbauweg sind im Fachgebiet bekannt und umfassen die Spaltung einer Verbindung, vorzugsweise einer organischen Verbindung, durch eine Zelle in Abbauprodukte (allgemeiner gesagt, kleinere oder weniger komplexe Moleküle) beispielsweise in einem Mehrschritt- und stark regulierten Prozeß. Der Begriff Stoffwechsel ist im Fachgebiet bekannt und umfaßt die Gesamtheit der biochemischen Reaktionen, diein einem Organismus stattfinden. Der Stoffwechsel einer bestimmten Verbindung (z.B. der Stoffwechsel einer Fettsäure) umfaßt dann die Gesamtheit der Biosynthese-, Modifikations- und Abbauwege dieser Verbindung in der Zelle, die diese Verbindung betreffen.

Bei einer anderen Ausführungsform können die erfindungsgemäßen PSE-Moleküle die Produktion eines gewünschten Moleküls, wie einer Feinchemikalie, in einem Mikroorganismus oder in Pflanzen modulieren. Es gibt eine Reihe von Mechanismen, durch die die Veränderung einer erfindungsgemäßen PSR die Ausbeute, Produktion und/oder Effizienz der Produktion einer Feinchemikalie aus einem Mikroorganismus- oder Pflanzenstamm, die dieses veränderte Protein enthalten, direkt beeinflussen kann. Die Anzahl oder Aktivität von PSEs, die am Transport von Feinchemikalienmolekülen innerhalb oder aus der Zelle beteiligt sind, kann erhöht werden, so daß größere Mengen dieser Verbindungen über Membranen transportiert werden, aus denen sie leichter gewonnen und ineinander umgewandelt werden. Ferner sind Fettsäuren, Triacylglycerine und/oder Lipide selbst wünschenswerte Feinchemikalien; durch Optimierung der Aktivität oder Steigern der Anzahl einer oder mehrerer erfindungsgemäßer PSEs, die an der Biosynthese dieser Verbindungen beteiligt sind, oder durch Stören der Aktivität einer oder mehrerer PSEs, die am Abbau dieser Verbindungen beteiligt sind, kann es möglich sein, die Ausbeute, Produktion und/oder Effizienz der Produktion von Fettsäure- und Lipidmolekülen aus Organismen, wie Mikroorganismen oder Pflanzen, zu erhöhen.

Die Mutagenese des erfindungsgemäßen PSE-Gens kann auch PSEs mit verändertenAktivitätenhervorbringen, welche die Produktion einer oder mehrerer gewünschter Feinchemikalien aus Mikroorganismen oder Pflanzen indirekt beeinflussen. Beispielsweise können erfindungsgemäße PSEs, die am Export von Abfallprodukten beteiligt sind, eine größere Anzahl oder höhere Aktivität aufweisen, so daß die normalen Stoffwechselabfälle der Zelle (deren Menge möglicherweise aufgrund der überproduktion der gewünschten Feinchemikalie erhöht ist) effizient exportiert werden, bevor sie die Moleküle in der Zelle schädigen können (was die Lebensfähigkeit der Zelle herabsetzen würde) oder die Feinchemikalien-Biosynthesewege stören können (was die Ausbeute, Produktion oder Effizienz der Produktion einer gewünschten Feinchemikalie senken würde). Die relativ großen intrazellulären Mengen der gewünschten Feinchemikalie selbst können ferner für die Zelle toxisch sein, so daß man durch Steigern der Aktivität oder Anzahl von Transportern, die diese Verbindungen aus der Zelle exportieren können, die Lebensfähigkeit der Zelle in Kultur steigern kann, was wiederum zu einer größeren Anzahl an Zellen in der Kultur führt, welche die gewünschte Feinchemikalie produzieren. Die erfindungsgemäßen PSEs können auch so manipuliert werden, daß die entsprechenden Mengen unterschiedlicher Lipid- und Fettsäuremoleküle produziert werden. Dies kann eine erhebliche Auswirkung auf die Lipidzusammensetzung der Zellmembran haben. Da jeder Lipidtyp unterschiedliche physikalische Eigenschaften aufweist, kann eine Veränderung der Lipidzusammensetzung einer Membran die Membranfluidität signifikant verändern. Änderungen der Membranfluidität können den Transport vonMolekülen über die Membran sowie die Integrität der Zelle beeinflussen, was jeweils eine erhebliche Auswirkung auf die Produktion von Feinchemikalien aus Mikroorganismen und Pflanzen in Fermentationskultur im großen Maßstab hat. Pflanzenmembranen verleihen spezifische Eigenschaften, wie Toleranz gegenüber Wärme, Kälte, Salz, Trockenheit sowie Toleranz gegen Pathogene, wie Bakterien und Pilze. Daher kann die Modulation der Membrankomponenten eine grundlegende Wirkung auf die Überlebensfähgikeit der Pflanzen unter den oben genannten Streßparametern haben. Dies kann über Änderungen in Signalkaskaden oder direkt über die veränderte Membranzusammensetzung erfolgen (siehe zum Beispiel: Chapman, 1998, Trends in Plant Science, 3 (11) :419-426) und Signalkaskaden (siehe Wang 1999, Plant Physiology, 120:645-651) oder die Kältetoleranz, wie offenbart in WO 95/18222, beeinflussen.

Die erfindungsgemäßen isolierten Nukleinsäuresequenzen sind beispielsweise im Genom eines Thraustochytrium-Stamms enthalten, der über die American Type Culture Collection (ATCC-Sammlung) mit der Stammnummer ATCC26185 (Thraustochytrium) verfügbar ist. Im Fall von Crypthecodinium sind die isolierten Nukleinsäuresequenzen beispielsweise vom Provasoli-Guillard National Center for Culture of Marine Phytoplancton ((CCMP) West Boothbay Harbour, ME, USA)mit der Stammkultur-Nr. CCMP316 zugänglich. Im Fall der Phytophthora infestans sind die genannten Nukleinsäuremoleküle aus dem Stamm ATCC 48886 isoliert.

Die Nukleotidsequenz der isolierten Physcomitrella-, Crypthecodinium-, Phytophthora infestans- oder Thraustochytrium-cDNA und die abgeleiteten Aminosäuresequenzen der Physcomitrella patens-PSEs sind in den SEQ ID NO: 1 bis SEQ ID NO:12 gezeigt. Es wurden Computeranalysen durchgeführt, die diese Nukleotidsequenzen als Sequenzen klassifizieren und/oder identifizieren, die am Stoffwechsel von Zellmembrankomponenten beteiligte Proteine oder am Transport von Verbindungen über Zellmembranen beteiligte Proteine bzw. der PUFA Biosynthese codieren. EST's mit der Datenbankeingabe-Nr. PP001019019F, CC001042041R, PI001002014R sowie TC002034029R, TC002034029R-11 und TC002014093R in der Datenbank des Erfinders stellen die erfindungsgemäßen Sequenzen in SEQ ID NO:1, SEQ ID NO:3, SEQ ID NO:5, SEQ ID NO:7, SEQ ID NO:9 und SEQ ID NO:11 dar. Auf analoge Weise wurden die partiellen Polypeptide als PP001019019F, CC001042041R, PI001002014R sowie TC002034029R, TC002034029R-11 und TC002014093R bezeichnet und stellen die erfindungsgemäßen Sequenzen in SEQ ID NO:2, SEQ ID NO:4, SEQ ID NO:6, SEQ ID NO:8, SEQ ID NO:10 und SEQ ID NO:12 gemäß Tabelle 2 dar. Das vollständige Fragment-Insert des ESTs TC002034029R wurde sequenziert und ergab die SEQ ID NO:3, welche die vollständige Sequenz von TC002034029R ist. TC002034029R-11 beschreibt eine Volllängensequenz einer Elongase aus Thraustochytrium. Die Namensgebung der übrigen Klone ist analog. Zudem wurde den verschiedenen Klonen Gennamen entsprechend zugewiesen. Abkürzungen: Tc = Thraustochytrium, Cc = Crypthecodinium, Pp = Physcomitrella patens, Pi = Phytophthora infestans.

**Tabelle 2**

| Bezeichnung est-Name | Genname | Polypeptid SEQ ID NO | Nukleinsäure SEQ ID NO |
|---|---|---|---|
| PP001019019F | Pp_PSE1 | 2 | 1 |
| TC002034029R | Tc_PSE1 | 4 | 3 |
| TC002014093R | Tc_PSE2 | 6 | 5 |
| CC001042041R | Cc_PSE1 | 8 | 7 |
| TC002034029R-11 | Tc_PSE1_1 | 10 | 9 |
| PI001002014R | Pi_PSE1 | 12 | 11 |

Die vorliegende Erfindung betrifft auch Proteine mit einer Aminosäuresequenz, die im wesentlichen homolog zu einer Aminosäuresequenz der SEQ ID NO:2, SEQ ID NO:4, und SEQ ID NO:10 ist. Wie hier verwendet, ist ein Protein mit einer Aminosäuresequenz, die im wesentlichen homolog zu einer ausgewählten Aminosäuresequenz ist, zu mindestens etwa 50 % homolog zu der ausgewählten Aminosäuresequenz, z.B. der gesamten ausgewählten Aminosäuresequenz. Ein Protein mit einer Aminosäuresequenz, die im wesentlichen homolog zu einer ausgewählten Aminosäuresequenz ist, kann auch zu mindestens etwa 50 bis 60 %, vorzugsweise mindestens etwa 60 bis 70 % und stärker bevorzugt mindestens etwa 70 bis 80 %, 80 bis 90 % oder 90 bis 95 % und am stärksten bevorzugt mindestens etwa 96 %, 97 %, 98 %, 99 % oder mehr homolog zu einer ausgewählten Aminosäuresequenz sein. Die erfindungsgemäße PSE oder der biologisch aktive Teil oder das Fragment davon kann am Stoffwechsel von zumAufbau von Zellmembranen in Mikroorganismen oder Pflanzen notwendigen Verbindungen oder am Transport von Molekülen über diese Membranen teilnehmen oder eine oder mehrere der zur Elongation von C₁₆ oder C₁₈-bzw C₂₀-PUFAs benötigten Aktivitäten besitzen, so daß C₂₀-, C₂₂- oder C₂₄-PUFAs sowie verwandte PUFAs erhalten werden.

Verschiedene Aspekte der Erfindung sind eingehender in den folgenden Unterabschnitten beschrieben.

### A. Isolierte Nukleinsäuremoleküle

Eine Ausführungsform der Erfindung sind isolierte Nukleinsäuren, die von PUFA produzierenden Mikroorganismen stammen und für Polypeptide codieren, die C₁₆ oder C₁₈-Fettsäuren mit mindestens zwei Doppelbindungen in der Fettsäure um mindestens zwei Kohlenstoffatome verlängern oder C₂₀-Fettsäuren mit mindestens zwei Doppelbindungen in der Fettsäure um mindestens zwei Kohlenstoffatome verlängern.

Eine weitere erfindungsgemäße Ausführungsform sind isolierte Nukleinsäuren, umfassend Nukleotidsequenzen, die für Polypeptide codieren, die C₁₆, C₁₈- oder C₂₀-Fettsäuren mit mindestens zwei Doppelbindungen in der Fettsäure verlängern und ausgewählt ist aus der Gruppe, bestehend aus
a) den in SEQ ID NO: 1, SEQ ID NO: 3, oder SEQ ID NO:9 dargestellten Nukleinsäuresequenzen,
b) einer Nukleinsäuresequenz, die gemäß dem degenerierten genetischen Code von einer der in SEQ ID NO:1, SEQ ID NO:3, oder SEQ ID NO:9 dargestellten Sequenzen stammt, oder
c) Derivate der in SEQ ID NO:1, SEQ ID NO:3, oder SEQ ID NO:9 dargestellten Sequenz, die für Polypeptide der in SEQ ID NO:2, SEQ ID NO:4, oder SEQ ID NO:10 dargestellten Aminosäuresequenz codieren und mindestens 50 % Homologie auf Aminosäureebene aufweisen, ohne daß die enzymatische Wirkung der Polypeptide wesentlich reduziert ist.

Die oben genannten erfindungsgemäßen Nukleinsäuren, die als C₁₆-, C₁₈- oder C₂₀-Elongase wirken, stammen von Organismen, wie Ciliaten, Pilzen, Algen,Pflanzen oder Dinoflagellaten,die PUFAs synthetisieren können, vorzugsweise von Pflanzen oder Algen, besonders bevorzugt aus der Gattung Phytophthora, Physcomitrella, Thraustochytrium oder Schizochytrium und am stärksten bevorzugt von Phytophthora infestans, Physcomitrellapatens, oder Thraustochytrium sp., Schizochytrium sp. oder nah verwandten Organismen.

Ein Aspekt der Erfindung betrifft isolierte Nukleinsäuremoleküle, die PSE-Polypeptide oder biologisch aktive Teile davon codieren, sowie Nukleinsäurefragmente, die zur Verwendung als Hybridisierungssonden oder Primer zur Identifizierung oder Amplifizierung einer PSE-codierenden Nukleinsäure (z.B. PSE-DNA) ausreichen. Der Begriff "Nukleinsäuremolekül", wie hier verwendet, soll DNA-Moleküle (z.B. cDNA oder genomische DNA) und RNA-Moleküle (z.B. mRNA) sowie DNA- oder RNA-Analoga, die mittels Nukleotidanaloga erzeugt werden, umfassen. Dieser Begriff umfaßt zudem die am 3'- und am 5'-Ende des codierenden Genbereichs gelegene untranslatierte Sequenz: mindestens etwa 100 Nukleotide der Sequenz stromaufwärts des 5'-Endes des codierenden Bereichs und mindestens etwa 20 Nukleotide der Sequenz stromabwärts des 3'-Endes des codierenden Genbereichs. Das Nukleinsäuremolekül kann einzelsträngig oder doppelsträngig sein, ist aber vorzugsweise doppelsträngige DNA. Ein "isoliertes" Nukleinsäuremolekül wird von anderen Nukleinsäuremolekülen abgetrennt, die in der natürlichen Quelle der Nukleinsäure vorliegen. Eine "isolierte" Nukleinsäure hat vorzugsweise keine Sequenzen, welche die Nukleinsäure in der genomischen DNA des Organismus, aus dem die Nukleinsäure stammt, natürlicherweise flankieren (z. B. Sequenzen, die sich an den 5'- und 3'-Enden der Nukleinsäure befinden). Bei verschiedenen Ausführungsformen kann das isolierte PSE-Nukleinsäuremolekül zum Beispiel weniger als etwa 5 kb, 4 kb, 3 kb, 2 kb, 1 kb, 0,5 kb oder 0,1 kb an Nukleotidsequenzen enthalten, die natürlicherweise das Nukleinsäuremolekül in der genomischen DNA der Zelle, aus der die Nukleinsäure stammt (z.B. eine Physcomitrella patens-Zelle) flankieren. Ein "isoliertes" Nukleinsäuremolekül, wie ein cDNA-Molekül, kann überdies im wesentlichen frei von anderem zellulären Material oder Kulturmedium sein, wenn es durch rekombinante Techniken hergestellt wird, oder frei von chemischen Vorstufen oder anderen Chemikalien sein, wenn es chemisch synthetisiert wird.

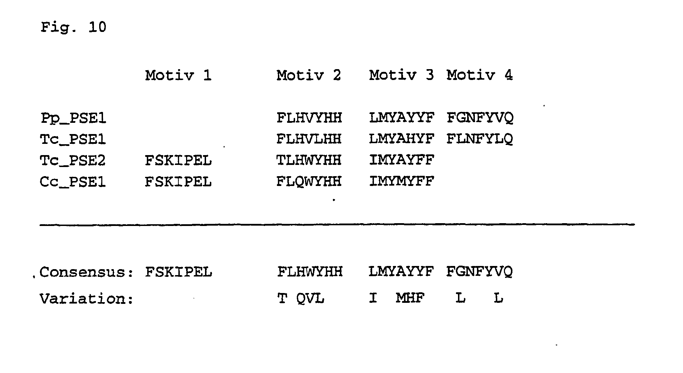

Ein erfindungsgemäßes Nukleinsäuremolekül, z.B. ein Nukleinsäuremolekül mit einer Nukleotidsequenz der SEQ ID NO:1, SEQ ID NO:3, und SEQ ID NO:9 oder eines Teils davon, kann unter Verwendung molekularbiologischer Standardtechniken und der hier bereitgestellten Sequenzinformation isoliert werden. Auch kann mit Hilfe von Vergleichsalgorithmen beispielsweise eine homologe Sequenz oder homologe, konservierte Sequenzbereiche auf DNA oder Aminosäureebene identifiziert werden. Beispielsweise kann eine Phytophthora-, Physcomitrella-, oder Thraustochytrium-cDNA aus einer Phythophthora, Physcomitrella-, oder Thraustochtrium-Bank isoliert werden, indem die vollständige SEQ ID NO:1, SEQ ID NO:3, und SEQ ID NO:9 oder ein Teil davon als Hybridisierungssonde sowie Standard-Hybridisierungstechniken (wie z.B. beschrieben in Sambrook et al., Molecular Cloning: A Laboratory Manual. 2. Aufl., Cold Spring Harbor Laboratory, Cold Spring HarborLaboratoryPress, Cold Spring Harbor, NY, 1989) verwendetwerden. Überdies läßt sich ein Nukleinsäuremolekül, umfassend eine vollständige Sequenz der SEQ ID NO:1, SEQ ID NO:3, und SEQ ID NO:9 oder einen Teil davon, durch Polymerasekettenreaktion isolieren, wobei Oligonukleotidprimer, die auf der Basis dieser Sequenz oder von Teilen davon, insbesondere Regionen um His-Box-Motive der SEQ ID NO:2, SEQ ID NO:4, und SEQ ID NO:10 erstellt werden oder Modifikationen ebensolcher in einzelnen definierten Aminosäuren, verwendet werden (z.B. kann ein Nukleinsäuremolekül, umfassend die vollständigen Sequenz der SEQ ID NO:1, SEQ ID NO:3, und SEQ ID NO:9 oder einen Teil davon, durch Polymerasekettenreaktion unter Verwendung von Oligonukleotidprimern isoliert werden, die auf der Basis dieser gleichen Sequenz der SEQ ID NO:1, SEQ ID NO:3, und SEQ ID NO:9 erstellt worden sind) . Weiterhin sind hierfür besonders geeignet solche Teilsequenzen, wie sie in Figur 10 dargestellt sind. Zum Beispiel läßt sich mRNA aus Zellen isolieren (z.B. durch das Guanidiniumthiocyanat-Extraktionsverfahren von Chirgwin et al. (1979) Biochemistry 18:5294-5299) und cDNA mittels Reverser Transkriptase (z.B. Moloney-MLV-Reverse-Transkriptase, erhältlich von Gibco/BRL, Bethesda, MD, oder AMV-Reverse-Transkriptase, erhältlich von Seikagaku America, Inc., St. Petersburg, FL) herstellen. Synthetische Oligonukleotidprimer zur Amplifizierung mittels Polymerasekettenreaktion lassen sich auf der Basis einer der in SEQ ID NO:1, SEQ ID NO:3, und SEQ ID NO:9 gezeigten Nukleotidsequenzen oder mit Hilfe der in Figur 10 dargestellten Aminosäuresequenzen erstellen. Eine erfindungsgemäße Nukleinsäure kann unter Verwendung von cDNA oder alternativ von genomischer DNA als Matrize und geeigneten Oligonukleotidprimern gemäß Standard-PCR-Amplifikationstechniken amplifiziert werden. Die so amplifizierte Nukleinsäure kann in einen geeigneten Vektor kloniert werden und mittels DNA-Sequenzanalyse charakterisiert werden. Oligonukleotide, die einer PSE-Nukleotidsequenz entsprechen, können durch Standard-Syntheseverfahren, beispielsweise mit einem automatischen DNA-Synthesegerät, hergestellt werden.

Die in SEQ ID NO:1, SEQ ID NO:3, und SEQ ID NO:9 gezeigte cDNA umfaßt Sequenzen, die PSEs codieren, (d.h. den "codierenden Bereich") sowie 5'-untranslatierte Sequenzen und 3'-untranslatierte Sequenzen. Alternativ kann das Nukleinsäuremolekül nur den codierenden Bereich einer der Sequenzen SEQ ID NO:1, SEQ ID NO:3, und SEQ ID NO:9 umfassen oder kann ganze genomische Fragmente, die aus genomischer DNA isoliert sind, enthalten.

Die SEQ ID NO:2, SEQ ID NO:4, und SEQ ID NO:10 wird durch die gleiche EST-Eingabenummer-Bezeichnung identifiziert, wie SEQ ID NO:1, SEQ ID NO:3, und SEQ ID NO:9, so daß sie leicht korreliert werden können.

Bei einer weiteren bevorzugten Ausführungsform umfaßt ein erfindungsgemäßes isoliertes Nukleinsäuremolekül ein Nukleinsäuremolekül, das ein Komplement einer der in SEQ ID NO:1, SEQ ID NO:3, und SEQ ID NO:9 gezeigten Nukleotidsequenzen oder eines Teils davon ist. Ein Nukleinsäuremolekül, das zu einer der in SEQ ID NO:1, SEQ ID NO:3, und SEQ ID NO:9 gezeigten Nukleotidsequenzen komplementärist, ist dann aus reichend komplementär, wenn es mit einer der in SEQ ID NO:1, SEQ ID NO:3, und SEQ ID NO:9 angegebenen Sequenzen hybridisieren kann, wodurch ein stabiler Duplex entsteht.

Homologe der neuen Elongase-Nukleinsäuresequenzen mit der Sequenz SEQ ID NO:1, SEQ ID NO:3, und SEQ ID NO:9 bedeutet beispielsweise allelische Varianten mit mindestens etwa 50 bis 60 %, vorzugsweise mindestens etwa 60 bis 70 %, stärker bevorzugt mindestens etwa 70 bis 80 %, 80 bis 90 % oder 90 bis 95 % und noch stärker bevorzugt mindestens etwa 95 %, 96 %, 97 %, 98 %, 99 % oder mehr Homologie zu einer in SEQ ID NO:1, SEQ ID NO:3, und SEQ ID NO:9 gezeigten Nukleotidsequenzen oder ihren Homologen, Derivaten oder Analoga oder Teilen davon. Bei einer weiteren bevorzugten Ausführungsform umfaßt ein isoliertes erfindungsgemäßes Nukleinsäuremolekül eine Nukleotidsequenz, die an eine der in SEQ ID NO:1, SEQ ID NO:3, und SEQ ID NO:9 gezeigten Nukleotidsequenzen oder einen Teil davon hybridisiert, z.B. unter stringenten Bedingungen hybridisiert. Allelische Varianten umfassen insbesondere funktionelle Varianten, die sich durch Deletion, Insertion oder Substitution von Nukleotiden aus/in der in SEQ ID NO:1, SEQ ID NO:3, und SEQ ID NO:9 dargestellten Sequenz erhalten lassen, wobei aber die Absicht ist, daß die Enzymaktivität der davon herrührenden synthetisierten Proteine für die Insertion eines oder mehrerer Gene vorteilhafterweise beibehalten wird. Proteine, die noch die enzymatische Aktivität der Elongase besitzen, bedeutet Proteine mit mindestens 10 %, vorzugsweise 20 %, besonders bevorzugt 30 %, ganz besonders bevorzugt 40 % der ursprünglichen Enzymaktivität, verglichen mit dem durch SEQ ID NO:2, SEQ ID NO:4, und SEQ ID NO:10 codierten Protein. Elongasen, noch die vorgenannten Aktivitäten aufweisen, sind Elongasen deren enzymatische Aktivität nicht wesentlich reduziert sind.

Homologen der SEQ ID NO:1, SEQ ID NO:3, und SEQ ID NO:9 bedeutet beispielsweise auch bakterielle, Pilz- und Pflanzenhomologen, verkürzte Sequenzen, einzelsträngige DNA oder RNA der codierenden und nicht-codierenden DNA-Sequenz.

Homologen der SEQ ID NO:1, SEQ ID NO:3, und SEQ ID NO:9 bedeutet auch Derivate, wie beispielsweise Promotorvarianten. Die Promotoren stromaufwärts der angegebenen Nukleotidsequenzen können durch einen oder mehrere Nukleotidaustausche, durch Insertion(en) und/oder Deletion(en) modifiziert werden, ohne daß jedoch die Funktionalität oder Aktivität der Promotoren gestört wird. Es ist weiterhin möglich, daßdie Aktivität der Promotoren durch Modifikation ihrer Sequenz erhöht ist oder daß sie vollständig durch aktivere Promotoren, sogar aus heterologen Organismen, ersetzt werden.

Überdies kann das erfindungsgemäße Nukleinsäuremolekül nur einen Teil des codierenden Bereichs einer der Sequenzen in SEQ ID NO:1, SEQ ID NO:3, und SEQ ID NO:9 umfassen, zum Beispiel ein Fragment, das als Sonde oder Primer verwendet werden kann, oder ein Fragment, welches einen biologisch aktiven Abschnitt einer PSE codiert. Die aus der Klonierung des PSE-Gens von Physcomitrella patens, Phytophthora infestans, und Thraustochytrium ermittelten Nukleotidsequenzen ermöglichen die Erzeugung von Sonden und Primern, die zur Identifizierung und/oder Klonierung von PSE-Homologen in anderen Zelltypen und Organismen sowie PSE-Homologen aus anderen Moosen oder verwandten Arten gestaltet sind. Die Sonde/der Primer umfaßt gewöhnlich im wesentlichen gereinigtes Oligonukleotid. Das Oligonukleotid umfaßt gewöhnlich einen Nukleotidsequenzbereich, der unter stringenten Bedingungen an mindestens etwa 12, vorzugsweise etwa 16, stärker bevorzugt etwa 25, 40, 50 oder 75 aufeinanderfolgende Nukleotide eines Sense-Stranges einer der in SEQ ID NO:1, SEQ ID NO:3, und SEQ ID NO:9 angegebenen Sequenzen, eines Antisense-Stranges einer der in SEQ ID NO:1, SEQ ID NO:3, und SEQ ID NO:9 angegebenen Sequenzen oder seiner Homologen, Derivate oder Analoga oder natürlich vorkommender Mutanten davon hybridisiert. Primer auf der Basis einer Nukleotidsequenz der SEQ ID NO:1, SEQ ID NO:3, und SEQ ID NO:9 können in PCR-Reaktionen zur Klonierung von PSE-Homologen verwendet werden. Sonden auf der Basis der PSE-Nukleotidsequenzen können zum Nachweis von Transkripten oder genomischen Sequenzen, die das gleiche oder homologe Proteine codieren, verwendet werden. Bei bevorzugten Ausführungsformen umfaßt die Sonde zudem eine daran gebundene Markierungsgruppe, z.B. ein Radioisotop, eine fluoreszierende Verbindung, ein Enzym oder einen Enzym-Cofaktor. Diese Sonden können als Teil eines Test-Kits für genomische Marker zur Identifizierung von Zellen, die eine PSE misexprimieren, beispielsweise durch Messen einer Menge einer PSE-codierenden Nukleinsäure in einer Zellenprobe, z.B. Messen der PSE-mRNA-Spiegel, oder zur Bestimmung, ob ein genomisches PSE-Gen mutiert oder deletiert ist, verwendet werden.

Bei einer Ausführungsform codiert das erfindungsgemäße Nukleinsäuremolekül ein Protein oder einen Teil davon, das/der eine Aminosäuresequenz umfaßt, die ausreichend homolog zu einer Aminosäuresequenz der SEQ ID NO:2, SEQ ID NO:4, und SEQ ID NO:10 ist, daß das Protein oder der Teil davon die Fähigkeit, am Stoffwechsel von zum Aufbau von Zellmembranen in Mikroorganismen oder Pflanzen notwendigen Verbindungen oder am Transport von Molekülen über diese Membranen teilzunehmen, beibehält. Wie hier verwendet, betrifft der Begriff "ausreichend homolog" Proteine oder Teile davon, deren Aminosäuresequenzen eine minimale Anzahl identischer oder äquivalenter Aminosäurereste (z.B. einen Aminosäurerest mit einer ähnlichen Seitenkette, wie ein Aminosäurerest in einer der Sequenzen der SEQ IDNO:2 bis 12) zu einer Aminosäuresequenz der SEQ ID NO:2, SEQ ID NO:4, und SEQ ID NO:10 aufweisen, so daß das Protein oder der Teil davon am Stoffwechsel von zum Aufbau von Zellmembranen in Mikroorganismen oder Pflanzen notwendigen Verbindungen oder am Transport von Molekülen über diese Membranen teilnehmen kann. Proteinbestandteile dieser Stoffwechselwege für Membrankomponenten oder Membrantransportsysteme können, wie hier beschrieben, eine Rolle bei der Produktion und Sekretion einer oder mehrerer Feinchemikalien spielen. Beispiele für diese Aktivitäten sind hier ebenfalls beschrieben. Somit trägt die "Funktion einer PSE" entweder direkt oder indirekt zur Ausbeute, Produktion und/oder Effizienz der Produktion einer oder mehrerer Feinchemikalien bei. Beispiele für PSE-Substratspezifitäten der katalytischen Aktivität sind in Tabelle 1 angegeben.

Bei einer weiteren Ausführungsform codieren Derivate des erfindungsgemäßen Nukleinsäuremoleküls Proteine mit mindestens etwa 50 bis 60 %, vorzugsweise mindestens etwa 60 bis 70 % und stärker bevorzugt mindestens etwa 70 bis 80 %, 80 bis 90 %, 90 bis 95 % und am stärksten bevorzugt mindestens etwa 96 %, 97 %, 98 %, 99 % oder mehr Homologie zu einer vollständigen Aminosäuresequenz der SEQ ID NO:2, SEQ ID NO:4, und SEQ ID NO:10. Die Homologie der Aminosäuresequenz wurde über den gesamten Sequenzbereich mit dem Programm PileUp (J. Mol. Evolution., 25, 351-360, 1987, Higgins et al., CABIOS, 5, 1989:151-153) oder BESTFIT oder GAP bestimmt (Henikoff, S. and Henikoff, J. G. (1992). Amino acid substitution matrices from protein blocks. Proc. Natl. Acad. Sci. USA 89: 10915-10919.)

Teile von Proteinen, die von den erfindungsgemäßen PSE-Nukleinsäuremolekülen codiert werden, sind vorzugsweise biologisch aktive Teile einer der PSEs. Wie hier verwendet, soll der Begriff "biologisch aktiver Teil einer PSE", einen Abschnitt, z.B. eine Domäne/ein Motiv, einer PSE umfassen, der am Stoffwechsel von zum Aufbau von Zellmembranen in Mikroorganismen oder Pflanzen notwendigen Verbindungen oder am Transport von Molekülen über diese Membranen teilnehmen kann oder eine in Tabelle 1 angegebene Aktivität aufweist. Zur Bestimmung, ob eine PSE oder ein biologisch aktiver Teil davon am Stoffwechsel von zum Aufbau von Zellmembranen in Mikroorganismen oder Pflanzen notwendigen Verbindungen oder am Transport von Molekülen über diese Membranen teilnehmen kann, kann ein Test der enzymatischen Aktivität durchgeführt werden. Diese Testverfahren, wie eingehend in Beispiel 8 des Beispielteils beschrieben, sind dem Fachmann geläufig.

Zusätzliche Nukleinsäurefragmente, die biologisch aktive Abschnitte einer PSE codieren, lassen sich durch Isolierung eines Teils einer der Sequenzen in SEQ ID NO:2, SEQ ID NO:4, und SEQ ID NO:10, Exprimieren des codierten Abschnitt der PSE oder des Peptids (z.B. durch rekombinante Expression in vitro) undBestimmender Aktivität des codierten Teils der PSE oder des Peptids herstellen.

Die Erfindung umfaßt zudem Nukleinsäuremoleküle, die sich von einer der in SEQ ID NO:1, SEQ ID NO:3, und SEQ ID NO:9 gezeigten Nukleotidsequenzen (und Teilen davon) aufgrund des degenerierten genetischen Codes unterscheiden und somit die gleiche PSE codieren wie diejenige, die von den in SEQ ID NO:1, SEQ ID NO:3, und SEQ ID NO:9 gezeigten Nukleotidsequenzen codiert wird. Bei einer anderen Ausführungsform hat ein erfindungsgemäßes isoliertes Nukleinsäuremolekül eine Nukleotidsequenz, die ein Protein mit einer in SEQ ID NO:2, SEQ ID NO:4, und SEQ ID NO:10 gezeigten Aminosäuresequenz codiert. Bei einer weiteren Ausführungsform codiert das erfindungsgemäße Nukleinsäuremolekül ein Vollängen-PSE-Protein, das zu einer Aminosäuresequenz der SEQ ID NO:2, SEQ ID NO:4, und SEQ ID NO:10 (die von einem in SEQ ID NO:1, SEQ ID NO:3, und SEQ ID NO:9 gezeigten offenen Leseraster codiert wird) im wesentlichen homolog ist und durch gängige Methoden identifizierbar und isolierbar ist.

Zusätzlich zu den in SEQ ID NO:1, SEQ ID NO:3, und SEQ ID NO:9 gezeigten PSE-Nukleotidsequenzen erkennt der Fachmann, daßDNA-Sequenzpolymorphismen, die zu Änderungen in den Aminosäuresequenzen der PSEs führen, innerhalb einer Population (z.B. der Physcomitrella- Phytophthora-, oder Thraustochytrium-Population) existieren können. Diese genetischen Polymorphismen im PSE-Gen können zwischen Individuen innerhalb einer Population aufgrund von natürlicher Variation existieren. Wie hier verwendet, bedeuten die Begriffe "Gen" und "rekombinantes Gen" Nukleinsäuremoleküle mit einem offenen Leserahmen, der eine PSE, vorzugsweise eine Phytophthora, Physcomitrella, oder Thraustochytrium -PSE, codiert. Diese natürlichen Varianten bewirken üblicherweise eine Varianz von 1 bis 5 % in der Nukleotidsequenz des PSE-Gens. Sämtliche und alle dieser Nukleotidvariationen und daraus resultierende Aminosäurepolymorphismen in PSE, die das Ergebnis natürlicher Variation sind und die funktionelle Aktivität von PSEs nicht verändern, sollen im Umfang der Erfindung enthalten sein.

Nukleinsäuremoleküle, die den natürlichen Varianten entsprechen, und nicht Physcomitrella-, Phytophthora-, oder Thraustochytrium -Homologen, -Derivate und -Analoga der Phytophthora-, Physcomitrella-, oder Thraustochytrium -cDNA können auf der Grundlage ihrer Homologie zu der hier offenbarten Phytophthora-, Physcomitrella-, oder Thraustochytrium -PSE-Nukleinsäure unter Verwendung der Physcomitrella-, Phytophthora-, oder Thraustochytrium-cDNA oder eines Teils davon als Hybridisierungssonde gemäß Standard-Hybridisierungstechniken unter stringenten Hybridisierungsbedingungen isoliert werden. Bei einer anderen Ausführungsform ist ein erfindungsgemäßes isoliertes Nukleinsäuremolekül mindestens 15 Nukleotide lang und hybridisiert unter stringenten Bedingungen mit dem Nukleinsäuremolekül, das eine Nukleotidsequenz der SEQ ID NO:1, SEQ ID NO:3, und SEQ ID NO:9 umfaßt. Bei anderen Ausführungsformen ist die Nukleinsäure mindestens 25, 50, 100, 250 oder mehr Nukleotide lang. Der Begriff "hybridisiert unter stringenten Bedingungen", wie hier verwendet, soll Hybridisierungs- und Waschbedingungen beschreiben, unter denen Nukleotidsequenzen, die mindestens 60 % homolog zueinander sind, gewöhnlich aneinander hybridisiert bleiben. Die Bedingungen sind vorzugsweise derart, daß Sequenzen, die mindestens etwa 65 %, stärker bevorzugt mindestens etwa 70 % und noch stärker bevorzugt mindestens etwa 75 % oder stärker zueinander homolog sind, gewöhnlich aneinander hybridisiert bleiben. Diese stringenten Bedingungen sind dem Fachmann bekannt und lassen sich in Current Protocols in Molecular Biology, John Wiley & Sons, N. Y. (1989), 6.3.1-6.3.6., finden. Ein bevorzugtes, nicht einschränkendes Beispiel für stringente Hybridisierungsbedingungen sind Hybridisierungen in 6 x Natriumchlorid/Natriumcitrat (sodium chloride/sodiumcitrate = SSC) bei etwa 45°C, gefolgt von einem oder mehreren Waschschritten in 0,2 x SSC, 0,1 % SDS bei 50 bis 65°C. Dem Fachmann ist bekannt, daß diese Hybridisierungsbedingungen sich je nach dem Typ der Nukleinsäure und, wenn beispielsweise organische Lösungsmittel vorliegen, hinsichtlich der Temperatur und der Konzentration des Puffers unterscheiden. Die Temperatur unterscheidet sich beispielsweise unter "Standard-Hybridisierungsbedingungen" je nach dem Typ der Nukleinsäure zwischen 42°C und 58°C in wäßrigem Puffer mit einer Konzentration von 0,1 bis 5 x SSC (pH 7,2) . Falls organisches Lösungsmittel im obengenannten Puffer vorliegt, zum Beispiel 50 % Formamid, ist die Temperatur unter Standardbedingungen etwa 42°C. Vorzugsweise sind die Hybridisierungsbedingungen für DNA:DNA-Hybride zum Beispiel 0,1 x SSC und 20°C bis 45°C, vorzugsweise zwischen 30°C und 45°C. Vorzugsweise sind die Hybridisierungsbedingungen für DNA:RNA-Hybride zum Beispiel 0,1 x SSC und 30°C bis 55°C, vorzugsweise zwischen 45°C und 55°C. Die vorstehend genannten Hybridisierungstemperaturen sind beispielsweise für eine Nukleinsäure mit etwa 100 bp (= Basenpaare) Länge und einem G + C-Gehalt von 50 % in Abwesenheit von Formamid bestimmt. Der Fachmann weiß, wie die erforderlichen Hybridisierungsbedingungen anhand von Lehrbüchern, wie dem vorstehend erwähnten oder aus den folgenden Lehrbüchern Sambrook et al. , "Molecular Cloning", Cold Spring Harbor Laboratory, 1989; Hames und Higgins (Hrsgb.) 1985, "Nucleic Acids Hybridization: A Practical Approach", IRL Press at Oxford University Press, Oxford; Brown (Hrsgb.) 1991, "Essential Molecular Biology: A Practical Approach", IRL Press at Oxford University Press, Oxford, bestimmt werden können.

Vorzugsweise entspricht ein erfindungsgemäßes isoliertes Nukleinsäuremolekül, das unter stringenten Bedingungen an eine Sequenz der SEQ ID NO:1, SEQ ID NO:3, und SEQ ID NO:9 hybridisiert, einem natürlich vorkommenden Nukleinsäuremolekül. Wie hier verwendet, betrifft ein "natürlich vorkommendes" Nukleinsäuremolekül ein RNA- oder DNA-Molekül mit einer Nukleotidsequenz, die in der Natur vorkommt (z.B. ein natürliches Protein codiert). Bei einer Ausführungsform codiert die Nukleinsäure eine natürliche vorkommende Physcomitrella patens-PSE, Phytophthora infestans-PSE, oder Thraustochytrium-PSE.

Zusätzlich zu natürlich vorkommenden Varianten der PSE-Sequenz, die in der Population existieren können, erkennt der Fachmann ferner daß auch Änderungen durch Mutation in eine Nukleotidsequenz der SEQ ID NO:1, SEQ ID NO:3, und SEQ ID NO:9 eingebracht werden können, was zu Änderungen der Aminosäuresequenz der codierten PSE führt, ohne daß die Funktionsfähigkeit des PSE-Proteins beeinträchtigt wird. Beispielsweise lassen sich Nukleotidsusbtitutionen, die an "nicht-essentiellen" Aminosäureresten zu Aminosäuresubstitutionen führen, in einer Sequenz der SEQ ID NO:1, SEQ ID NO:3, und SEQ ID NO:9 herstellen. Ein "nicht-essentieller" Aminosäurerest ist ein Rest, der sich in einer Wildtypsequenz einer der PSEs (SEQ ID NO:2, SEQ ID NO:4, und SEQ ID NO:10) verändern läßt, ohne daß die Aktivität der PSE verändert wird, wohingegen ein "essentieller" Aminosäurerest für die PSE-Aktivität erforderlich ist. Andere Aminosäurereste (z.B. diejenigen, die in der Domäne mit PSE-Aktivität nicht konserviert oder lediglich semikonserviert sind) können jedoch für die Aktivität nicht essentiell sein und lassen sich somit wahrscheinlich verändern, ohne daß die PSE-Aktivität verändert wird.

Folglich betrifft ein weiterer Aspekt der Erfindung Nukleinsäuremoleküle, die PSEs codieren, die veränderte Aminosäurereste enthalten, die für die PSE-Aktivität nicht essentiell sind. Diese PSEs unterscheiden sich in der Aminosäuresequenz von einer Sequenz in SEQ ID NO:2, SEQ ID NO:4, und SEQ ID NO:10 und behalten dennoch zumindest eine der hier beschriebenen PSE-Aktivitäten. Das isolierte Nukleinsäuremolekül umfaßt bei einer Ausführungsform eine Nukleotidsequenz, die ein Protein codiert, wobei das Protein eine Aminosäuresequenz mit mindestens etwa 50 % Homologie zu einer Aminosäuresequenz der SEQ ID NO:2, SEQ ID NO:4, und SEQ ID NO:10 umfaßt und am Stoffwechsel von zum Aufbau von Zellmembranen in Phytophthora, Physcomitrella, oder Thraustochytrium notwendigen Verbindungen oder am Transport von Molekülen über diese Membranen teilnehmen kann. Das von dem Nukleinsäuremolekül codierte Protein ist vorzugsweise mindestens etwa 50 bis 60 % homolog zu einer der Sequenzen in SEQ ID NO:2, SEQ ID NO:4, und SEQ ID NO:10 stärker bevorzugt mindestens etwa 60 bis 70 % homolog zu einer der Sequenzen in SEQ ID NO:2, SEQ ID NO:4, und SEQ ID NO:10 noch stärker bevorzugt mindestens etwa 70 bis 80 %, 80 bis 90 %, 90 bis 95 % homolog zu einer der Sequenzen in SEQ ID NO:2, SEQ ID NO:4, und SEQ ID NO:10 und am stärksten bevorzugt mindestens etwa 96 %, 97 %, 98 % oder 99 % homolog zu einer der Sequenzen in SEQ ID NO:2, SEQ ID NO:4, und SEQ ID NO:10.

Zur Bestimmung der prozentualen Homologie von zwei Aminosäuresequenzen (z.B. einer der Sequenzen der SEQ ID NO:2, SEQ ID NO:4, und SEQ ID NO:10 und einer mutierten Form davon) oder von zwei Nukleinsäuren werden die Sequenzen zum Zweck des optimalen Vergleichs untereinander geschrieben (z.B. können Lücken in die Sequenz eines Proteins oder einer Nukleinsäure eingefügt werden, um ein optimales Alignment mit dem anderen Protein oder der anderen Nukleinsäure zu erzeugen). Die Aminosäurereste oder Nukleotide an den entsprechenden Aminosäurepositionen oder Nukleotidpositionen werden dann verglichen. Wenn eine Position in einer Sequenz (z.B. einer der Sequenzen der SEQ ID NO:2, SEQ ID NO:4, und SEQ ID NO:10 ) durch den gleichen Aminosäurerest oder das gleiche Nukleotid wie die entsprechende Stelle in der anderen Sequenz (z.B. einer mutierten Form der aus SEQ ID NO:2, SEQ ID NO:4, und SEQ ID NO:10 ausgewählten Sequenz) belegt wird, dann sind die Moleküle an dieser Position homolog (d.h. Aminosäure- oder Nukleinsäure-"Homologie", wie hier verwendet, entspricht Aminosäure- oder Nukleinsäure-"Identität"). Die prozentuale Homologie zwischen den beiden Sequenzen ist eine Funktion der Anzahl an identischen Positionen, die den Sequenzen gemeinsam sind (d.h. % Homologie = Anzahl der identischen Positionen/Gesamtanzahl der Positionen x 100).

Ein isoliertes Nukleinsäuremolekül, das eine PSE codiert, die zu einer Proteinsequenz der SEQ ID NO:2, SEQ ID NO:4, und SEQ ID NO:10 homolog ist, kann durch Einbringen einer oder mehrerer Nukleotidsubstitutionen, -additionen oder -deletionen in eine Nukleotidsequenz der SEQ ID NO:1, SEQ ID NO:3, und SEQ ID NO:9 erzeugt werden, so daß eine oder mehrere Aminosäuresubstitutionen, -additionen oder -deletionen in das codierte Protein eingebracht werden. Mutationen können in eine der Sequenzen der SEQ ID NO:1, SEQ ID NO:3, und SEQ ID NO:9 durch Standardtechniken, wie stellenspezifische Mutagenese und PCR-vermittelte Mutagenese, eingebracht werden. Vorzugsweise werden konservative Aminosäuresubstitutionen an einer oder mehreren der vorhergesagten nicht-essentiellen Aminosäureresten hergestellt. Bei einer "konservativen Aminosäuresubstitution" wird der Aminosäurerest gegen einen Aminosäurerest mit einer ähnlichen Seitenkette ausgetauscht. Im Fachgebiet sind Familien von Aminosäureresten mit ähnlichen Seitenketten definiert worden. Diese Familien umfassen Aminosäurenmit basischen Seitenketten (z.B. Lysin, Arginin, Histidin), sauren Seitenketten (z.B.Asparaginsäure,Glutaminsäure), ungeladenen polaren Seitenketten (z.B. Glycin, Asparagin, Glutamin, Serin, Threonin, Tyrosin, Cystein), unpolaren Seitenketten, (z.B. Alanin, Valin, Leucin, Isoleucin, Prolin, Phenylalanin,Methionin,Tryptophan), beta-verzweigten Seitenketten (z.B. Threonin, Valin, Isoleucin) und aromatischen Seitenketten (z.B. Tyrosin, Phenylalanin, Tryptophan, Histidin). Ein vorhergesagter nicht-essentieller Aminosäurerest in einer PSE wird somit vorzugsweise durch einen anderen Aminosäurerest aus der gleichen Seitenkettenfamilie ausgetauscht. Alternativ können bei einer anderen Ausführungsform die Mutationen zufallsgemäß über die gesamte oder einen Teil der PSE-codierenden Sequenz eingebracht werden, z.B. durch Sättigungsmutagenese, und die resultierenden Mutanten können nach der hier beschriebenen PSE-Aktivität durchmustert werden, um Mutanten zu identifizieren, die PSE-Aktivität beibehalten. Nach der Mutagenese einer der Sequenzen der SEQ ID NO:1, SEQ ID NO:3, und SEQ ID NO:9 kann das codierte Protein rekombinant exprimiert werden, und die Aktivität des Proteins kann z.B. unter Verwendung der hier beschriebenen Tests (siehe Beispielteil) bestimmt werden.

Zusätzlich zu den Nukleinsäuremolekülen, welche die vorstehend beschriebenen PSEs codieren, betrifft ein weiterer Aspekt der Erfindung isolierte Nukleinsäuremoleküle, die "Antisense" dazu sind. Eine "Antisense"-Nukleinsäure umfaßt eine Nukleotidsequenz, die zu einer "Sense"-Nukleinsäure, welche ein Protein codiert, komplementär ist, z.B. komplementär zum codierenden Strang eines doppelsträngigen cDNA-Moleküls oder komplementär zu einer mRNA-Sequenz. Eine Antisense-Nukleinsäure kann folglich über Wasserstoffbrückenbindungen an eine Sense-Nukleinsäure binden. Die Antisense-Nukleinsäure kann zu einem gesamten PSE-codierenden Strang oder nur zu einem Teil davon komplementär sein. Bei einer Ausführungsform ist ein Antisense-Nukleinsäuremolekül "Antisense" zu einem "codierenden Bereich" des codierenden Strangs einer Nukleotidsequenz, die eine PSE codiert. Der Begriff "codierender Bereich" betrifft den Bereich der Nukleotidsequenz, der Codons umfaßt, die in Aminosäurereste translatiert werden (z.B. den gesamten codierenden Bereich, der mit dem Stopcodon beginnt und endet, d.h. dem letzten Codon vor dem Stopcodon). Bei einer weiteren Ausführungsform ist das Antisense-Nukleinsäuremolekül "Antisense" zu einem "nicht-codierenden Bereich" des codierenden Strangs einer Nukleotidsequenz, die PSE codiert. Der Begriff "nicht-codierender Bereich" betrifft 5'- und 3'-Sequenzen, die den codierenden Bereich flankieren und nicht in Aminosäuren translatiert werden (d.h. die man auch als 5'- und 3'-untranslatierte Bereiche bezeichnet).

Unter Voraussetzung der hier offenbarten PSE-codierenden Sequenzen des codierenden Stranges (z.B. die in SEQ ID NO:1, SEQ ID NO:3, und SEQ ID NO:9 dargestellten Sequenzen) können erfindungsgemäße Antisense-Nukleinsäuren gemäß den Regeln der Watson-Crick-Basenpaarung gestaltet werden. Das Antisense-Nukleinsäuremolekül kann komplementär zum gesamten codierenden Bereich von PSE-mRNA sein, ist aber stärker bevorzugt ein Oligonukleotid, das nur zu einem Teil des codierenden oder nicht-codierenden Bereichs von PSE-mRNA "Antisense" ist. Das Antisense-Oligonukleotid kann z.B. zu dem Bereich, der die Translationsstartstelle von PSE-mRNA umgibt, komplementär sein. Ein Antisense-Oligonukleotid kann z.B. etwa 5, 10, 15, 20, 25, 30, 35, 40, 45 oder 50 und mehr Nukleotide lang sein. Vorteilhaft ist ein Antisense-Oligonukleotis 15 bis 25 Nukleotide lang. Eine erfindungsgemäße Antisense-Nukleinsäure kann unter Verwendung chemischer Synthese und enzymatischer Ligationsreaktionen mittels im Fachgebiet bekannter Verfahren konstruiert werden. Eine Antisense-Nukleinsäure (z.B. ein Antisense-Oligonukleotid) kann z.B. chemisch synthetisiert werden, wobei natürlich vorkommende Nukleotide oder verschiedentlich modifizierte Nukleotide verwendet werden, die so gestaltet sind, daß sie die biologische Stabilität der Moleküle erhöhen oder die physikalische Stabilität des zwischen der Antisense- und der Sense-Nukleinsäure gebildeten Duplexes erhöhen, beispielsweise können Phosphorthioat-Derivate und acridinsubstituierte Nukleotide verwendet werden. Beispiele für modifizierte Nukleotide, die zur Erzeugung der Antisense-Nukleinsäure verwendet werden können, sind u.a. 5-Fluoruracil, 5-Bromuracil, 5-Chloruracil, 5-Ioduracil, Hypoxanthin, Xanthin, 4-Acetylcytosin, 5-(Carboxyhydroxylmethyl)uracil, 5-Carboxymethylaminomethyl-2-thiouridin, 5-Carboxymethylaminomethyluracil, Dihydrouracil, Beta-D-Galactosylqueosin, Inosin, N6-Isopentenyladenin, 1-Methylguanin, 1-Methylinosin, 2,2-Dimethylguanin, 2-Methyladenin, 2-Methylguanin, 3-Methylcytosin, 5-Methylcytosin, N6-Adenin, 7-Methylguanin, 5-Methylaminomethyluracil, 5-Methoxyaminomethyl-2-thiouracil, Beta-D-Mannosylqueosin,5'-Methoxycarboxymethyluracil, 5-Methoxyuracil, 2-Methylthio-N6-isopentyladenin, Uracil-5-oxyessigsäure (v), Wybutoxosin, Pseudouracil, Queosin, 2-Thiocytosin, 5-Methyl-2-thiouracil, 2-Thiouracil, 4-Thiouracil, 5-Methyluracil, Uracil-5-oxyessigsäuremethylester, Uracil-5-oxyessigsäure (v), 5-Methyl-2-thiouracil, 3-(3-Amino-3-N-2-carboxypropyl)uracil, (acp3)w und 2,6-Diaminopurin. Die Antisense-Nukleinsäure kann alternativ biologisch unter Verwendung eines Expressionsvektors hergestellt werden, in den eine Nukleinsäure in Antisense-Richtung subkloniert worden ist (d.h. RNA, die von der eingebrachten Nukleinsäure transkribiert wird, ist zu einer Zielnukleinsäure von Interesse in Antisense-Richtung orientiert, was im nachstehenden Unterabschnitt weiter beschrieben ist).

Die erfindungsgemäßen Antisense-Nukleinsäuremoleküle werden üblicherweise an eine Zelle verabreicht oder in situ erzeugt, so daß sie mit der zellulären mRNA und/oder der genomischen DNA, die eine PSE codiert, hybridisieren oder daran binden, um dadurch die Expression des Proteins, z. B. durch Hemmung der Transkription und/oder Translation, zu hemmen. Die Hybridisierung kann durch herkömmliche Nukleotidkomplementarität unter Bildung eines stabilen Duplexes oder z.B. im Fall eines Antisense-Nukleinsäuremoleküls, das DNA-Duplices bindet, durch spezifische Wechselwirkungen in der großen Furche der Doppelhelix erfolgen. Das Antisense-Molekül kann so modifiziert sein, daß es spezifisch an einen Rezeptor oder an ein auf einer ausgewählten Zelloberfläche exprimiertes Antigen bindet, z.B. durch Binden des Antisense-Nukleinsäuremoleküls an ein Peptid oder einen Antikörper, das/der an einen Zelloberflächenrezeptor oder ein Antigen bindet. Das Antisense-Nukleinsäuremolekül kann auch unter Verwendung der hier beschriebenen Vektoren den Zellen zugeführt werden. Zur Erzielung ausreichender intrazellulärer Konzentrationen der Antisense-Moleküle sind Vektorkonstrukte, in denen sich das Antisense-Nukleinsäuremolekül unter der Kontrolle eines starken prokaryotischen, viralen oder eukaryotischen, einschließlich pflanzlichen, Promotors befindet, bevorzugt.

Bei einer weiteren Ausführungsform ist das erfindungsgemäße Antisense-Nukleinsäuremolekül ein α-anomeres Nukleinsäuremolekül. Ein a-anomeres Nukleinsäuremolekül bildet spezifische doppelsträngige Hybride mit komplementärer RNA, wobei die Stränge im Gegensatz zu gewöhnlichen β-einheiten parallel zueinander verlaufen. [Gaultier et al. (1987) Nucleic Acids Res. 15:6625-6641]. Das Antisense-Nukleinsäuremolekül kann zudem ein 2'-o-Methylribonukleotid [Inoue et al. (1987) Nucleic Acids Res. 15:6131-6148] oder ein chimäres RNA-DNA-Analogon [Inoue et al. (1987) FEBS Lett. 215:327-330] umfassen.

Bei einer weiteren Ausführungsform ist eine erfindungsgemäße Antisense-Nukleinsäure ein Ribozym. Ribozyme sind katalytische RNA-Moleküle mit Ribonukleaseaktivität, die eine einzelsträngige Nukleinsäure, wie eine mRNA, spalten können, zu der sie einen komplementären Bereich haben. Somit können Ribozyme z.B. Hammerhead-Ribozyme [beschrieben in Haselhoff und Gerlach (1988) Nature 334:585-591] zur katalytischen Spaltung von PSE-mRNA-Transkripten verwendet werden, um dadurch die Translation von PSE-mRNA zu hemmen. Ein Ribozym mit Spezifität für eine PSE-codierende Nukleinsäure kann auf der Basis der Nukleotidsequenz einer hier offenbarten PSE-cDNA (d.h. 38_ck21_g07fwd in (SEQ ID NO:1, SEQ ID NO:3, und SEQ ID NO:9) oder auf der Basis einer gemäß den in dieser Erfindung gelehrten Verfahren zu isolierenden heterologen Sequenz gestaltet werden. Beispielsweise kann ein Derivat einer Tetrahymena-L-19-IVS-RNA konstruiert werden, wobei die Nukleotidsequenz der aktiven Stelle komplementär zu der Nukleotidsequenz ist, die in einer PSE-codierenden mRNA gespalten werden soll. Siehe z.B. Cech et al., US 4,987,071 und Cech et al., US 5, 116, 742. Alternativ kann PSE-mRNA zur Selektion einer katalytischen RNA mit einer spezifischen Ribonukleaseaktivität aus einem Pool von RNA-Molekülen verwendet werden [siehe z.B. Bartel, D., und Szostak, J.W. (1993) Science 261:1411-1418].

Alternativ läßt sich die PSE-Gen-Expression hemmen, indem Nukleotidsequenzen, die komplementär zum regulatorischen Bereich einer PSE-Nukleotidsequenz (z.B. einem PSE-Promotor und/oder -Enhancer) sind, so dirigiert werden, daß Dreifachhelix-Strukturen gebildet werden, welche die Transkription eines PSE-Gens in Zielzellen hemmen [siehe allgemein Helene, C. (1991) Anticancer Drug Res. 6(6) 569-84; Helene, C., et al. (1992) Ann. N.Y. Acad. Sci. 660:27-36; und Maher. L.J. (1992) Bioassays 14(12):807-815].

### B. Genkonstrukt

Eine weitere Ausführungsform der Erfindung ist ein neues Genkonstrukt, was eine isolierte Nukleinsäure enthält, die von Physcomitrella, Phytophthora, oder Thraustochytrium stammt und ein Polypeptid codiert, das C₁₆, C₁₈- oder C₂₀-Fettsäuren mit mindestens zwei Doppelbindungen in der Fettsäure um mindestens zwei Kohlenstoffatome verlängert, oder die Gensequenz der SEQ ID NO:1, SEQ ID NO:3, und SEQ ID NO:9, ihre Homologen, Derivate oder Analoga, die funktionsfähig mit einem oder mehreren Regulationssignalen, vorteilhafterweise zur Steigerung der Genexpression, verbunden sind, enthält. Beispiele für diese Regulationssequenzen sind Sequenzen, an die Induktoren oder Repressoren binden und so die Expression der Nukleinsäure regulieren. Zusätzlich zu diesen neuen Regulationssequenzen kann die natürliche Regulation dieser Sequenzen vor den eigentlichen Strukturgenen noch vorhanden sein und, wenn geeignet, genetisch modifiziert worden sein, so daß die natürliche Regulation ausgeschaltet worden ist und die Expression der Gene gesteigert worden ist. Das Genkonstrukt kann jedoch auch eine einfachere Struktur haben, d.h. daß keine zusätzlichen Regulationssignale vor der sequenz SEQ ID NO:1, SEQ ID NO:3, und SEQ ID NO:9 oder ihren Homologen inseriert worden sind und der natürliche Promotor mit seiner Regulation nicht deletiert worden ist. Statt dessen ist die natürliche Regulationssequenz so mutiert worden, daß keine Regulation mehr stattfindet und die Genexpression verstärkt ist. Das Genkonstrukt kann außerdem vorteilhafterweise eine oder mehrere sogenannte Enhancer-Sequenzen, die funktionsfähig mit dem Promotor verbunden sind und die gesteigerte Expression der Nukleinsäuresequenz ermöglichen, umfassen. Es ist auch möglich, am 3'-Ende der DNA-Sequenzen zusätzlich vorteilhafte Sequenzen zu inserieren, beispielsweise weitere Regulationselemente oder Terminatoren. Die Elongasegene können im Genkonstrukt in einer oder mehreren Kopien vorliegen. Es ist vorteilhaft für die Insertion weiterer Gene in Organismen, wenn weitere Gene im Genkonstrukt vorliegen.

Vorteilhafte Regulationssequenzen für das neue Verfahren liegen beispielsweise in Promotoren vor, wie dem cos-, tac-, trp-, tet-, trp-tet-, lpp-, lac-, lpp-lac-, lacI^{q-}, T7-, T5-, T3-, gal-, trc-, ara-, SP6-, λ-P_{R}- oder λ-P_{L}-Promotor und werden vorteilhafterweise in Gram-negativen Bakterien angewendet. Weitere vorteilhafte Regulationssequenzen liegen beispielsweise in den Gram-positiven Promotoren amy und SPO2, in den Hefe- oder Pilzpromotoren ADC1, MFα, AC, P-60, CYC1, GAPDH, TEF, rp28, ADH oder in den Pflanzenpromotoren CaMV/35S [Franck et al., Cell 21 (1980) 285-294], PRP1 [Ward et al., Plant. Mol. Biol. 22 (1993)], SSU, OCS, lib4, usp, STLS1, B33, nos oder im Ubiquitin- oder Phaseolin-Promotor vor. In diesem Zusammenhang vorteilhaft sind ebenfalls induzierbare Promotoren, wie die in EP-A-0 388 186 (Benzylsulfonamid-induzierbar), Plant J. 2, 1992:397-404 (Gatz et al., Tetracyclin-induzierbar), EP-A-0 335 528 (Abzisinsäure-induzierbar) oder WO 93/21334 (Ethanol- oder Cyclohexenol-induzierbar) beschriebenen Promotoren. Weitere geeignete Pflanzenpromotoren sind der Promotor von cytosolischer FBPase oder der ST-LSI-Promotor der Kartoffel (Stockhaus et al., EMBO J. 8, 1989, 2445), der Phosphoribosylpyrophosphatamidotransferase-Promotor aus Glycine max (Genbank-Zugangsnr. U87999) oder der in EP-A-0 249 676 beschriebene nodienspezifische Promotor. Besonders vorteilhafte Promotoren sind Promotoren, welche die Expression in Geweben ermöglichen, die an der Fettsäurebiosynthese beteiligt sind. Ganz besonders vorteilhaft sind samenspezifische Promotoren, wie der usp-, LEB4-, Phaseolin- oder Napin-Promotor. Weitere besonders vorteilhafte Promotoren sind samenspezifische Promotoren, die für monokotyle oder dikotyle Pflanzen verwendet werden können und in US 5, 608, 152 (Napin-Promotor aus Raps), WO 98/45461 (Phaseolin-Promotor aus Arobidopsis), US 5,504,200 (Phaseolin-Promotor aus Phaseolus vulgaris), WO 91/13980 (Bce4-Promotor aus Brassica), von Baeumlein et al., Plant J., 2, 2, 1992:233-239 (LEB4-Promotor aus einer Leguminose) beschrieben sind, wobei sich diese Promotoren für Dikotyledonen eignen. Die folgenden Promotoren eignen sich beispielsweise für Monokotyledonen lpt-2- oder lpt-1-Promotor aus Gerste (WO 95/15389 und WO 95/23230), Hordein-Promotor aus Gerste und andere, in WO 99/16890 beschriebene geeignete Promotoren.

Es ist im Prinzip möglich, alle natürlichen Promotoren mit ihren Regulationssequenzen, wie die oben genannten, für das neue Verfahren zu verwenden. Es ist ebenfalls möglich und vorteilhaft, zusätzlich synthetische Promotoren zu verwenden.

Das Genkonstrukt kann, wie oben beschrieben, auch weitere Gene umfassen, die in die Organismen eingebracht werden sollen. Es ist möglich und vorteilhaft, in die Wirtsorganismen Regulationsgene, wie Gene für Induktoren, Repressoren oder Enzyme, welche durch ihre Enzymaktivität in die Regulation eines oder mehrerer Gene eines Biosynthesewegs eingreifen, einzubringen und darin zu exprimieren. Diese Gene können heterologen oder homologen Ursprungs sein. Die inserierten Gene können ihren eigenen Promotor haben oder auch unter der Kontrolle des Promotors der Sequenz SEQ ID NO:1, SEQ ID NO:3, SEQ ID NO:5, SEQ ID NO:7, SEQ ID NO:9 und SEQ ID NO:11 oder seiner Homologen, Derivate oder Analoga stehen.

Das Genkonstrukt umfaßt vorteilhafterweise zur Expression der anderen vorliegenden Gene weitere 3'- und/oder 5'-terminale Regulationssequenzen zur Steigerung der Expression, die in Abhängigkeit vom gewählten Wirtsorganismus und dem Gen oder den Genen für die optimale Expression ausgewählt werden.

Diese Regulationssequenzen sollen, wie oben erwähnt, die spezifische Expression der Gene und die Proteinexpression ermöglichen. Dies kann je nach dem Wirtsorganismus beispielsweise bedeuten, daß das Gen nur nach Induktion exprimiert oder überexprimiert wird oder daß es sofort exprimiert und/oder überexprimiert wird.

Die Regulationssequenzen oder -faktoren können außerdem vorzugsweise eine vorteilhafte Wirkung auf die Expression der eingebrachten Gene haben und diese somit steigern. Auf diese Weise ist es möglich, daß die Regulationselemente unter Verwendung starker Transkriptionssignale, wie Promotoren und/oder Enhancer, vorteilhafterweise auf Transkriptionsebene verstärkt werden. Es ist jedoch weiterhin auch möglich, die Translation zum Beispiel durch Verbesserung der mRNA-Stabilität zu verstärken. Vorteilhafterweise werden die erfindungsgemäßen Nukleinsäuresequenzen zusammen mit mindestens einem Reportergen in ein Genkonstrukt (= Expressionskassette, Nukleinsäurekonstrukt) kloniert, die in den Organismus über einen Vektor oder direkt in das Genom eingebracht wird. Dieses Reportergen sollte eine leichte Detektierbarkeit über einen Wachstums-, Fluoreszenz-, Chemo-, Biolumineszenz- oder Resistenzassay oder über eine photometrische Messung ermöglichen. Beispielhaft seien als Reportergene Antibiotika-oder Herbizidresistenzgene, Hydrolasegene, Fluoreszenzproteingene, Biolumineszenzgene, Zucker- oder Nukleotidstoffwechselgene oder Biosynthesegene wie das Ura3-Gen, das Ilv2-Gen, das Luciferasegen, das b-Galactosidasegen, das gfp-Gen, das 2-Desoxyglucose-6-phosphat-Phosphatasegen, das b-Glucuronidase-Gen, b-Lactamasegen, das Neomycinphosphotransferasegen, das Hygromycinphosphotransferasegen oder das BASTA (= Gluphosinatresistenz)-Gen genannt. Diese Gene ermöglichen eine leichte Messbarkeit und Quantifizierbarkeit der Transcriptionsaktivität und damit der Expression der Gene. Damit lassen sich Genomstellen identifizieren, die eine unterschiedliche Produktivität zeigen.

Die erfindungsgemäßen Nukleinsäuresequenzen mit SEQ ID NO:1, SEQ ID NO:3, und SEQ ID NO:9, die für Elongasen codieren können in einer oder mehreren Kopien in der Expressionskassette (= Genkonstrukt) enthalten sein.

In der Expressionskassette (= Genkonstrukt, Nukleinsäurekonstrukt) kann noch mindestens eine weitere Nukleinsäure, die für einGenbevorzugt aus der Fettsäurebiosynthese codiert, enthalten sein, die in die Wirtsorganismen eingebracht werden sollen. Diese Gene können unter getrennter Regulation oder unter der gleichen Regulationsregion wie die Gene erfindungsgemäßen Elongase liegen. Bei diesen Genen handelt es sich beispielsweise um weitere Biosynthesegene vorteilhaft der Fettsäurebiosynthese, die eine gesteigerte Synthese ermöglichen. Beispielsweise seien die Gene für die Δ19-, Δ17-, Δ15-, Δ12-, Δ9-, Δ8-, Δ6-, Δ5-, Δ4-Desaturase, die verschiedenen Hydroxylasen, die Δ12-Acetylenase, die Acyl-ACP-Thioesterasen, β-Ketoacyl-ACP-Synthasen oder β-Ketoacyl-ACP-Reductasen genannt. Vorteilhaft werden die Desaturasegene im Nukleinsäurekonstrukt verwendet. Auch diese Gene können in einer oder mehreren Kopien im Genkonstrukt enthalten sein.

### C. Rekombinante Expressionsvektoren und Wirtszellen

Ein weiterer Aspekt der Erfindung betrifft Vektoren, vorzugsweise Expressionsvektoren, die eine erfindungsgemäße Nukleinsäure oder ein erfindungsgemäßes Genkonstrukt enthalten, die eine PSE (oder einen Teil davon) codieren. Wie hier verwendet, betrifft der Begriff "Vektor" ein Nukleinsäuremolekül, das eine andere Nukleinsäure transportieren kann, an welche es gebunden ist. Ein Vektortyp ist ein"Plasmid" , was für eine zirkuläre doppelsträngige DNA-Schleife steht, in die zusätzlichen DNA-Segmente ligiert werden können. Ein weiterer Vektortyp ist ein viraler Vektor, wobei zusätzliche DNA-Segmente in das virale Genom ligiert werden können. Bestimmte Vektoren können in einer Wirtszelle, in die sie eingebracht worden sind, autonom replizieren (z.B. Bakterienvektoren mit bakteriellem Replikationsursprung und episomale Säugervektoren). Andere Vektoren (z.B. nicht-episomale Säugervektoren) werden beim Einbringen in die Wirtszelle in das Genom einer Wirtszelle integriert und dadurch zusammen mit dem Wirtsgenom repliziert. Zudem können bestimmte Vektoren die Expression von Genen, mit denen sie funktionsfähig verbunden sind, steuern. Diese Vektoren werden hier als "Expressionsvektoren" bezeichnet. Gewöhnlich haben Expressionsvektoren, die für DNA-Rekombinationstechniken geeignet sind, die Form von Plasmiden. In der vorliegenden Beschreibung können "Plasmid" und "Vektor" austauschbar verwendet werden, da das Plasmid die am häufigsten verwendete Vektorform ist. Die Erfindung soll jedoch diese anderen Expressionsvektorformen, wie virale Vektoren (z.B. replikationsdefiziente Retroviren, Adenoviren und adenoverwandte Viren), die ähnliche Funktionen ausüben, umfassen. Ferner soll der Begriff Vektor auch andere Vektoren, die dem Fachmann bekannt sind, wie Phagen, Viren, wie SV40, CMV, Baculovirus, Adenovirus, Transposons, IS-Elemente, Phasmide, Phagemide, Cosmide, lineare oder zirkuläre DNA sowie RNA umfassen.

Die erfindungsgemäßen rekombinanten Expressionsvektoren umfassen eine erfindungsgemäße Nukleinsäure oder ein erfindungsgemäßes Genkonstrukt in einer Form, die sich zur Expression der Nukleinsäure in einer Wirtszelle eignet, was bedeutet, daß die rekombinanten Expressionsvektoren eine oder mehrere Regulationssequenzen, ausgewählt auf der Basis der zur Expression zu verwendenden Wirtszellen, die mit der zu exprimierenden Nukleinsäuresequenz funktionsfähig verbunden ist, umfaßt. In einem rekombinanten Expressionsvektor bedeutet "funktionsfähig verbunden", daß die Nukleotidsequenz von Interesse derart an die Regulationssequenz(en) gebunden ist, daß die Expression der Nukleotidsequenz möglich ist und sie aneinander gebunden sind, so daß beide Sequenzen die vorhergesagte, der Sequenz zugeschriebene Funktion erfüllen (z.B. in einem In-vitro-Transkriptions-/Translationssystem oder in einer Wirtszelle, wenn der Vektor in die Wirtszelle eingebracht wird). Der Begriff "Regulationssequenz" soll Promotoren, Enhancer und andere Expressionskontrollelemente (z.B. Polyadenylierungssignale) umfassen. Diese Regulationssequenzen sind z.B. beschrieben in Goeddel: Gene Expression Technology: Methods in Enzymology 185, Academic Press, San Diego, CA (1990), oder siehe: Gruber und Crosby, in: Methods in Plant Molecular Biology and Biotechnolgy, CRC Press, Boca Raton, Florida, Hrsgb.: Glick und Thompson, Kapitel 7, 89-108, einschließlich der Literaturstellen darin. Regulationssequenzen umfassen solche, welche die konstitutive Expression einer Nukleotidsequenz in vielen Wirtszelltypen steuern, und solche, welche die direkte Expression der Nukleotidsequenz nur in bestimmten Wirtszellen unter bestimmten Bedingungen steuern. Der Fachmann weiß, daß die Gestaltung des Expressionsvektors von Faktoren, wie der Auswahl der zu transformierenden Wirtszelle, dem Ausmaß der Expression des gewünschten Proteins usw., abhängen kann. Die erfindungsgemäßen Expressionsvektoren können in Wirtszellen eingebracht werden, um dadurch Proteine oder Peptide, einschließlich Fusionsproteinen oder -peptiden, herzustellen, die von den Nukleinsäuren, wie hier beschrieben, codiert werden (z.B. PSEs, mutante Formen von PSEs, Fusionsproteine usw.).

Die erfindungsgemäßen rekombinanten Expressionsvektoren können zur Expression von PSEs in prokaryotischen oder eukaryotischen Zellen gestaltet sein. Beispielsweise können PSE-Gene in bakteriellen Zellen, wie C. glutamicum, Insektenzellen (unter Verwendung von Baculovirus-Expressionsvektoren),Hefe- und anderen Pilzzellen[siehe Romanos, M.A., et al. (1992) "Foreign gene expression in yeast : a review", Yeast8:423-488; vandenHondel, C.A.M.J.J., etal. (1991) "Heterologous gene expression in filamentous fungi", in: More Gene Manipulations in Fungi, J.W. Bennet & L.L. Lasure, Hrsgb. , S. 396-428: Academic Press: San Diego; und van den Hondel, C.A.M.J.J., & Punt, P.J. (1991) "Gene transfer systems and vector development for filamentous fungi, in: Applied Molecular Genetics of Fungi, Peberdy, J.F., et al., Hrsgb., S. 1-28, Cambridge University Press: Cambridge], Algen [Falciatore et al., 1999, Marine Biotechnology. 1, 3:239-251)], Ciliaten der Typen: wie Holotrichia, Peritrichia, Spirotrichia, Suctoria, Tetrahymena, Paramecium, Colpidium, Glaucoma, Platyophrya, Potomacus, Pseudocohnilembus, Euplotes, Engelmaniella und Stylonychia, insbesondere der Gattung Stylonychia lemnae, mit Vektoren nach einem Transformationsverfahren, wie beschrieben in WO 98/01572, sowie Zellen vielzelliger Pflanzen [siehe Schmidt, R. undWillmitzer, L. (1988) "High efficiency Agrobacterium tumefaciens-mediated transformation of Arabidopsis thaliana leaf and cotyledon explants" Plant Cell Rep.:583-586; Plant Molecular Biology and Biotechnology, C Press, Boca Raton, Florida, Kapitel 6/7, S.71-119 (1993); F.F. White, B. Jenes et al., Techniques for Gene Transfer, in: Transgenic Plants, Bd. 1, Engineering and Utilization, Hrsgb.: Kung und R. Wu, Academic Press (1993), 128-43; Potrykus, Annu. Rev. Plant Physiol. Plant Molec. Biol. 42 (1991), 205-225 (und darin zitierte Literaturstellen)] oder Säugerzellen exprimiert werden. Geeignete Wirtszellen werden ferner erörtert in Goeddel, Gene Expression Technology: Methods in Enzymology 185, Academic Press, San Diego, CA (1990). Der rekombinante Expressionsvektor kann alternativ, zum Beispiel unter Verwendung von T7-Promotor-Regulationssequenzen und T7-Polymerase, in vitro transkribiert und translatiert werden.

Die Expression von Proteinenin Prokaryoten erfolgt meist mit Vektoren, die konstitutive oder induzierbare Promotoren enthalten, welche die Expression von Fusions- oder nicht-Fusionsproteinen steuern. Fusionsvektoren fügen eine Reihe von Aminosäuren an ein darin codiertes Protein an, gewöhnlich am Aminoterminus des rekombinanten Proteins, aber auch am C-Terminus oder fusioniert innerhalb geeigneter Bereiche in den Proteinen. Diese Fusionsvektoren haben gewöhnlich drei Aufgaben: 1) die Verstärkung der Expression von rekombinantem Protein; 2) die Erhöhung der Löslichkeit des rekombinanten Proteins und 3) die Unterstützung der Reinigung des rekombinanten Proteins durch Wirkung als Ligand bei der Affinitätsreinigung beispielsweise über sogenannte His-Tags. Bei Fusions-Expressionsvektoren wird oft eine proteolytische Spaltstelle an der Verbindungsstelle der Fusionseinheit und des rekombinanten Proteins eingebracht, so daß die Abtrennung des rekombinanten Proteins von der Fusionseinheit nach der Reinigung des Fusionsproteins möglich ist. Diese Enzyme und ihre entsprechenden Erkennungssequenzen umfassen Faktor Xa, Thrombin und Enterokinase.

Typische Fusions-Expressionsvektoren sind u.a.pGEX [Pharmacia Biotech Inc; Smith, D.B., und Johnson, K.S. (1988) Gene 67:31-40], pMAL [New England Biolabs, Beverly, MA] und pRIT5 [Pharmacia, Piscataway, NJ], bei denen Glutathion-S-Transferase (GST), Maltose E-bindendes Protein bzw. Protein A an das rekombinante Zielprotein fusioniert wird. Bei einer Ausführungsform ist die PSE-codierende Sequenz in einen pGEX-Expressionsvektor kloniert, so daß ein Vektor erzeugt wird, der ein Fusionsprotein codiert, das vom N-Terminus zum C-Terminus GST-Thrombin-Spaltstelle-X-Protein umfaßt. Das Fusionsprotein kann durch Affinitätschromatographie unter Verwendung von Glutathion-Agarose-Harz gereinigt werden. Rekombinante PSE, die nicht an GST fusioniert ist, kann durch Spaltung des Fusionsproteins mit Thrombin gewonnen werden.

Beispiele für geeignete induzierbare nicht-Fusions-E. coli-Expressionsvektoren sind u.a. pTrc (Amann et al. (1988) Gene 69:301-315) und pET 11d (Studier et al., Gene Expression Technology: Methods in Enzymology 185, Academic Press, San Diego, Kalifornien (1990) 60-89). Die Zielgenexpression vom pTrc-Vektor beruht auf der Transkription durch Wirts-RNA-Polymerase von einem Hybrid-trp-lac-Fusionspromotor. Die Zielgenexpression aus dem pET 11d-Vektor beruht auf der Transkription von einem T7-gn10-lac-Fusions-Promotor, die von einer coexprimierten viralen RNA-Polymerase (T7 gnl) vermittelt wird. Diese virale Polymerase wird von den Wirtsstämmen BL21 (DE3) oder HMS174 (DE3) von einem residenten λ-Prophagen bereitgestellt, der ein T7 gnl-Gen unter der Transkriptionskontrolle des lacUV 5-Promotors birgt.

Andere in prokaryotischen Organismen geeignete Vektoren sind dem Fachmann bekannt, diese Vektoren sind beispielsweise in E. coli pLG338, pACYC184, die pBR-Reihe, wie pBR322, die pUC-Reihe, wie pUC18 oder pUC19, die M113mp-Reihe, pKC30, pRep4, pHS1, pHS2, pPLc236, pMBL24, pLG200, pUR290, pIN-III¹¹³-B1, pgt11 or pBdCI, in Streptomyces pIJ101, pIJ364, pIJ702 oder pIJ361, in Bacillus pUB110, pC194 oder pBD214, in Corynebacterium pSA77 oder pAJ667.

Eine Strategie zur Maximierung der Expression von rekombinantem Protein ist die Expression des Proteins in einem Wirtsbakterium, dessen Fähigkeit zur proteolytischen Spaltung des rekombinanten Proteins gestört ist [Gottesman, S., Gene Expression Technology: Methods in Enzymology 185, Academic Press, San Diego, Kalifornien (1990) 119-128]. Eine weitere Strategie ist die Veränderung der Nukleinsäuresequenz der in einen Expressionsvektor zu inserierenden Nukleinsäure, so daß die einzelnen Codons für jede Aminosäure diejenigen sind, die vorzugsweise in einem zur Expression ausgewählten Bakterium, wie C. *glutamicum,* verwendet werden [Wada et al. (1992) Nucleic Acids Res. 20:2111-2118]. Diese Veränderung der erfindungsgemäßen Nukleinsäuresequenzen erfolgt durch Standard-DNA-Synthesetechniken.

Bei einer weiteren Ausführungsform ist der PSE-Expressionsvektor ein Hefe-Expressionsvektor. Beispiele für Vektoren zur Expression in der Hefe S. cerevisiae umfassen pYepSec1 [Baldari et al. (1987) Embo J. 6:229-234], pMFa [Kurjan und Herskowitz (1982) Cell 30:933-943], pJRY88 [Schultz et al. (1987) Gene 54:113-123] sowie pYES2 [Invitrogen Corporation, San Diego, CA]. Vektoren und Verfahren zur Konstruktion von Vektoren, die sich zur Verwendung in anderen Pilzen, wie den filamentösen Pilzen, eignen, umfassen diejenigen, die eingehend beschrieben sind in: van den Hondel, C.A.M.J.J., & Punt, P.J. (1991) "Gene transfer systems and vector development for filamentous fungi, in: Applied Molecular Genetics of fungi, J.F. Peberdy et al., Hrsgb., S. 1-28, Cambridge University Press: Cambridge, oder in: More Gene Manipulations in Fungi [J.W. Bennet & L.L. Lasure, Hrsgb., S. 396-428: Academic Press: San Diego]. Weitere geeignete Hefevektoren sind beispielsweise pAG-1, YEp6, YEp13 oder pEMBLYe23.

Alternativ können die erfindungsgemäßen PSEs in Insektenzellen unter Verwendung von Baculovirus-Expressionsvektoren exprimiert werden. Baculovirus-Vektoren, die zur Expression von Proteinen in gezüchteten Insektenzellen (z.B. Sf9-Zellen) verfügbar sind, umfassen die pAc-Reihe [Smith et al. (1983) Mol. Cell Biol.. 3:2156-2165] und die pVL-Reihe [Lucklow und Summers (1989) Virology 170:31-39].

Die oben genannten Vektoren bieten nur einen kleinen Überblick über mögliche geeignete Vektoren. Weitere Plasmide sind dem Fachmann bekannt und sind zum Beispiel beschrieben in: Cloning Vectors (Hrsgb. Pouwels, P.H., et al., Elsevier, Amsterdam-New York-Oxford, 1985, ISBN 0 444 904018).

Bei noch einer weiteren Ausführungsform wird eine erfindungsgemäße Nukleinsäure in Säugerzellen unter Verwendung eines Säuger-Expressionsvektors exprimiert. Beispiele für Säuger-Expressionsvektoren umfassen pCDM8 [Seed, B. (1987) Nature 329:840] und pMT2PC [Kaufman et al. (1987) EMBO J. 6:187-195]. Bei der Verwendung in Säugerzellen werden die Kontrollfunktionen des Expressionsvektors oft von viralen Regulationselementen bereitgestellt. Üblicherweise verwendete Promotoren stammen z.B. aus Polyoma, Adenovirus2, Cytomegalievirus und Simian Virus 40. Weitere geeignete Expressionssysteme für prokaryotische und eukaryotische Zellen siehe in den Kapiteln 16 und 17 von Sambrook, J. , Fritsch, E. F. , und Maniatis, T., Molecular Cloning: A Laboratory Manual, 2. Auflage, Cold Spring Harbor Laboratory, Cold Spring Harbor Laboratory Press, Cold Spring Harbor, NY, 1989.

Bei einer anderen Ausführungsform kann der rekombinante Säuger-Expressionsvektor die Expression der Nukleinsäure vorzugsweise in einem bestimmten Zelltyp steuern (z.B. werden gewebespezifische Regulationselemente zur Expression der Nukleinsäure verwendet). Gewebespezifische Regulationselemente sind im Fachgebiet bekannt. Nicht beschränkende Beispiele für geeignete gewebespezifische Promotoren sind u.a. der Albuminpromotor [leberspezifisch; Pinkert et al. (1987) Genes Dev. 1:268-277], lymphoidspezifische Promotoren [Calame und Eaton (1988) Adv. Immunol. 43:235-275], insbesondere Promotoren von T-Zellrezeptoren [Winoto und Baltimore (1989) EMBO J. 8:729-733] und Immunglobulinen [Banerji et al. (1983) Cell 33:729-740; Queen und Baltimore (1983) Cell 33:741-748], neuronspezifische Promotoren [z.B. Neurofilament-Promotor; Byrne und Ruddle (1989) PNAS 86:5473-5477], pankreasspezifische Promotoren [Edlund et al., (1985) Science 230:912-916] und milchdrüsenspezifische Promotoren [z.B. Milchserum-Promotor; US 4,873,316 und EP-A-0 264 166). Auch entwicklungsregulierte Promotorensind umfaßt, z.B. die hox-Promotoren der Maus [Kessel und Gruss (1990) Science 249:374-379] und der Fetoprotein-Promotor [Campes undTilghman (1989) Genes Dev. 3:537-546].

Bei einer weiteren Ausführungsform können die erfindungsgemäßen PSEs in einzelligen Pflanzenzellen (wie Algen), siehe Falciatore et al., 1999, Marine Biotechnology 1 (3):239-251 und darin zitierte Literaturangaben, und Pflanzenzellen aus höheren Pflanzen (z.B. Spermatophyten, wie Feldfrüchten) exprimiert werden. Beispiele für Pflanzen-Expressionsvektoren umfassen solche, die eingehend beschrieben sind in: Becker, D., Kemper, E., Schell, J., und Masterson, R. (1992) "New plant binary vectors with selectable markers located proximal to the left border", Plant Mol. Biol. 20:1195-1197; und Bevan, M.W. (1984) "Binary Agrobacterium vectors for plant transformation", Nucl. Acids Res. 12:8711-8721; Vectors for Gene Transfer in Higher Plants; in: Transgenic Plants, Bd. 1, Engineering and Utilization, Hrsgb. : KungundR. Wu, Academic Press, 1993, S. 15-38.Weiteregeeignete pflanzliche Vektoren werden unter anderem in "Methods in Plant Molecular Biology and Biotechnology" (CRC Press), Kap. 6/7, S.71-119beschrieben.

Vorteilhafte Vektoren sind sog. shuttle-Vektoren oder binäre Vektoren, die in E. coli und Agrobacterium replizieren.

Eine Pflanzen-Expressionskassette enthält vorzugsweise Regulationssequenzen, welche die Genexpression in Pflanzenzellen steuern können und funktionsfähig verbunden sind, so daß jede Sequenz ihre Funktion, wie Termination der Transkription, erfüllen kann, beispielsweise Polyadenylierungssignale. Bevorzugte Polyadenylierungssignale sind diejenigen, die aus Agrobacterium tumefaciens-t-DNA stammen, wie das als Octopinsynthase bekannte Gen 3 des Ti-Plasmids pTiACH5 [Gielen et al., EMBO J. 3 (1984) 835ff.] oder funktionelle Äquivalente davon, aber auch alle anderen in Pflanzen funktionell aktiven Terminatoren sind geeignet.

Da die Pflanzengenexpression sehr oft nicht auf Transkriptionsebenen beschränkt ist, enthält eine Pflanzen-Expressionskassette vorzugsweise andere funktionsfähig verbunden Sequenzen, wie Translationsenhancer, beispielsweise die Overdrive-Sequenz, welche die 5'-untranslatierte Leader-Sequenz aus Tabakmosaikvirus, die das Protein/RNA-Verhältnis erhöht, enthält [Gallie et al., 1987, Nucl. Acids Research 15:8693-8711].

Die Pflanzengenexpression muss funktionsfähig mit einem geeigneten Promotor verbunden sein, der die Genexpression auf rechtzeitige, zell- oder gewebespezifische Weise durchführt. Bevorzugt sind Promotoren, welche die konstitutive Expression herbeiführen [Benfey et al., EMBO J. 8 (1989) 2195-2202], wie diejenigen, die von Pflanzenviren stammen, wie 35S CAMV [Franck et al., Cell 21 (1980) 285-294], 19S CaMV (siehe auch US 5,352,605 und WO 84/02913) oder Pflanzenpromotoren, wie der in US 4,962,028 beschriebene der kleinen Untereinheit der Rubisco.

Andere bevorzugte Sequenzen für die Verwendung zur funktionsfähigen Verbindung in Pflanzengenexpressions-Kassetten sind Targeting-Sequenzen, die zur Steuerung des Genproduktes in sein entsprechendes Zellkompartiment notwendig sind [siehe eine Übersicht inKermode, Crit. Rev. Plant Sci. 15, 4 (1996) 285-423 und darin zitierte Literaturstellen], beispielsweise in die Vakuole, den Zellkern, alle Arten von Plastiden, wie Amyloplasten, Chloroplasten, Chromoplasten, den extrazellulären Raum, die Mitochondrien, das Endoplasmatische Retikulum, Ölkörper, Peroxisomen und andere Kompartimente von Pflanzenzellen.

Die Pflanzengenexpression läßt sich auch über einen chemisch induzierbaren Promotor erleichtern [siehe eine Übersicht in Gatz 1997, Annu. Rev. Plant Physiol. Plant Mol. Biol., 48:89-108]. Chemisch induzierbare Promotoren eignen sich besonders, wenn gewünscht wird, daß die Genexpression auf zeitspezifische Weise erfolgt. Beispiele für solche Promotoren sind ein Salicylsäure-induzierbarer Promotor (WO 95/19443), ein Tetracyclin-induzierbarer Promotor [Gatz et al. (1992) Plant J. 2, 397-404] und ein Ethanol-induzierbarer Promotor.

Auch Promotoren, die auf biotische oder abiotische Streßbedingungen reagieren, sind geeignete Promotoren, beispielsweise der pathogeninduzierte PRP1-Gen-Promotor [Ward et al., Plant. Mol. Biol. 22 (1993) 361-366], der hitzeinduzierbare hsp80-Promotor aus Tomate (US 5,187,267), der kälteinduzierbare Alphaamylase-Promotor aus Kartoffel (WO 96/12814) oder der durch Wunden induzierbare pinII-Promotor (EP-A-0 375 091).

Es sind insbesondere solche Promotoren bevorzugt, welche die Genexpression in Geweben und Organen herbeiführen, in denen die Lipid-und Ölbiosynthese stattfindet, in Samenzellen, wie den Zellen des Endosperms und des sich entwickelnden Embryos. Geeignete Promotoren sind der Napingen-Promotor aus Raps (US 5,608,152), der USP-Promotor aus Vicia faba [Baeumlein et al., Mol Gen Genet, 1991, 225 (3) : 459-67], der Oleosin-Promotor aus Arabidopsis (WO 98/45461), der Phaseolin-Promotor aus Phaseolus vulgaris (US 5,504,200), der Bce4-Promotor aus Brassica (WO 91/13980) oder der Legumin-B4-Promotor [LeB4; Baeumlein et al., 1992, Plant Journal, 2 (2):233-9] sowie Promotoren, welche die samenspezifische Expression in Monokotyledonen-Pflanzen, wie Mais, Gerste, Weizen, Roggen, Reis usw. herbeiführen. Geeignete beachtenswerte Promotoren sind der lpt2- oder lpt1-Gen-Promotor aus Gerste (WO 95/15389 und WO 95/23230) oder die in WO 99/16890 beschriebenen (Promotoren aus dem Gersten-Hordein-Gen, dem Reis-Glutelin-Gen, dem Reis-Oryzin-Gen, dem Reis-Prolamin-Gen, dem Weizen-Gliadin-Gen, Weizen-Glutelin-Gen, dem Mais-Zein-Gen, dem Hafer-Glutelin-Gen, dem Sorghum-Kasirin-Gen, dem Roggen-Secalin-Gen).

Ebenfalls besonders geeignet sind Promotoren, welche die plastidenspezifische Expression herbeiführen, da Plastiden das Kompartiment sind, in dem die Vorläufer sowie einige Endprodukte der Lipidbiosynthese synthetisiert werden. Geeignete Promotoren, wie der virale RNA-Polymerase-Promotor, sind beschrieben in WO 95/16783 und WO 97/06250, und der clpP-Promotor aus Arabidopsis, beschrieben in WO 99/46394.

Die Erfindung stellt zudem einen rekombinanten Expressionsvektor bereit, umfassend ein erfindungsgemäßesDNA Molekül, das in Antisense-Richtung in den Expressionsvektor kloniert ist. d.h. das DNA-Molekül ist derart mit einer regulatorischen Sequenz funktionsfähig verbunden, daß die Expression (durch Transkription des DNA-Moleküls) eines RNA-Moleküls, das zur PSE-mRNA "Antisense" ist, ermöglicht wird. Es können Regulationssequenzen ausgewählt werden, die funktionsfähig mit einer in Antisense-Richtung klonierten Nukleinsäure verbunden sind und die kontinuierliche Expression des Antisense-RNA-Moleküls in einer Vielzahl von Zelltypen steuern, zum Beispiel können virale Promotoren und/oder Enhancer oder Regulationssequenzen ausgewählt werden, welche die konstitutive, gewebespezifische oder zelltypspezifische Expression von Antisense-RNA steuern. Der Antisense-Expressionsvektor kann in Form eines rekombinanten Plasmids, Phagemids oder attenuierten Virus vorliegen, in dem Antisense-Nukleinsäuren unter der Kontrolle eines hochwirksamen regulatorischen Bereichs produziert werden, dessen Aktivität durch den Zelltyp bestimmt werden kann, in den der Vektor eingebracht worden ist. Eine Erläuterung der Regulation der Genexpression mittels Antisense-Genen siehe in Weintraub, H., et al., Antisense-RNA as a molecular tool for genetic analysis, Reviews - Trends in Genetics, Bd. 1(1) 1986.

Ein weiterer Aspekt der Erfindung betrifft Wirtszellen, in die ein erfindungsgemäßer rekombinanter Expressionsvektor eingebracht worden ist. Die Begriffe "Wirtszelle" und "rekombinante Wirtszelle" werden hier untereinander austauschbar verwendet. Selbstverständlich betreffen diese Begriffe nicht nur die bestimmte Zielzelle, sondern auch die Nachkommen oder potentiellen Nachkommen dieser Zelle. Da in aufeinanderfolgenden Generationen aufgrund von Mutation oder Umwelteinflüssen bestimmte Modifikationen auftreten können, sind diese Nachkommen nicht unbedingt mit der Parentalzelle identisch, sind jedoch immer noch vom Umfang des Begriffs, wie hier verwendet, umfaßt.

Eine Wirtszelle kann eine prokaryotische oder eukaryotischeZelle sein. Zum Beispiel kann eine PSE in Bakterienzellen, wie C. glutamicum, Insektenzellen, Pilzzellen oder Säugerzellen (wie Chinesischer Hamster-Ovarzellen (CHO) oder COS-Zellen), Algen, Ciliaten, Pflanzenzellen, Pilzen oder anderen Mikroorganismen, wie C. glutamicum, exprimiert werden. Andere geeignete Wirtszellen sind dem Fachmann geläufig.

Vektor-DNA läßt sich in prokaryotische oder eukaryotische Zellen über herkömmliche Transformations- oder Transfektionstechniken einbringen. Die Begriffe "Transformation" und "Transfektion", Konjugation und Transduktion, wie hier verwendet, sollen eine Vielzahl von im Stand der Technik bekannten Verfahren zum Einbringen fremder Nukleinsäure (z.B. DNA) in eine Wirtszelle, einschließlich Calciumphosphat- oder Calciumchlorid-Copräzipitation, DEAE-Dextran-vermittelte Transfektion, Lipofektion, natürliche Kompetenz, chemisch vermittelter Transfer, Elektroporation oder Teilchenbeschuß, umfassen. Geeignete Verfahren zur Transformation oder Transfektion von Wirtszellen, einschließlich Pflanzenzellen, lassen sich finden in Sambrook et al. (Molecular Cloning: A Laboratory Manual., 2. Aufl., Cold Spring Harbor Laboratory, Cold Spring Harbor Laboratory Press, Cold Spring Harbor, NY, 1989) und anderen Labor-Handbüchern, wie Methods in Molecular Biology, 1995, Bd. 44, Agrobacterium protocols, Hrsgb: Gartland und Davey, Humana Press, Totowa, New Jersey.

Über die stabile Transfektion von Säugerzellen ist bekannt, daß je nach verwendetem Expressionsvektor und verwendeter Transfektionstechnik nur ein kleiner Teil der Zellen die fremde DNA in ihr Genom integriert. Zur Identifikation und Selektion dieser Integranten wird gewöhnlich ein Gen, das einen selektierbaren Marker (z.B. Resistenz gegen Antibiotika) codiert, zusammen mit dem Gen von Interesse in die Wirtszellen eingebracht. Bevorzugte selektierbare Marker umfassen solche, welche Resistenz gegen Medikamente, wie G418, Hygromycin und Methotrexat, verleihen, oder in Pflanzen solche, welche Resistenz gegen ein Herbizid, wie Glyphosphat oder Glufosinat, verleihen. Weitere geeignete Marker sind beispielsweise Marker, welche Gene codieren, die an Biosynthesewegen von zum Beispiel Zuckern oder Aminosäuren beteiligt sind, wie β-Galactodsidase, ura3 oder ilv2. Marker, welche Gene, wie Luziferase, gfp oder andere Fluoreszenzgene codieren, sind ebenfalls geeignet. Diese Marker lassen sich in Mutanten verwenden, in denen diese Gene nicht funktionell sind, da sie beispielsweise mittels herkömmlicher Verfahren deletiert worden sind. Ferner können Marker, welche eine Nukleinsäure codieren, die einen selektierbaren Marker codiert, in eine Wirtszelle auf dem gleichen Vektor eingebracht werden, wie derjenige, der eine PSE codiert, oder können auf einem gesonderten Vektor eingebracht werden. Zellen, die mit der eingebrachten Nukleinsäure stabil transfiziert worden sind, können zum Beispiel durch Medikamentenselektion identifiziert werden (z. B. überleben Zellen, die den selektierbaren Marker integriert haben, wohingegen die anderen Zellen absterben).

Zur Erzeugung eines homolog rekombinierten Mikroorganismus wird ein Vektor hergestellt, der zumindest einen Abschnitt eines PSE-Gens enthält, in den eine Deletion, Addition oder Substitution eingebracht worden ist, um dadurch das PSE-Gen zu verändern, z.B. funktionell zu disrumpieren. Dieses PSE-Gen ist vorzugsweise ein Physcomitrella, Phytophthora-, oder Thraustochytrium PSE-Gen, es kann jedoch ein Homologon oder Analogon aus anderen Organismen, sogar aus einer Säuger- Pilz- oder Insektenquelle verwendet werden. Bei einer bevorzugten Ausführungsform ist der Vektor so gestaltet, daß das endogene PSE-Gen bei homologer Rekombination funktionell disruptiert wird (d.h. nicht länger ein funktionelles Protein codiert, auch als Knock-out-Vektor bezeichnet). Alternativ kann der Vektor so gestaltet sein, daß das endogene PSE-Gen bei homologer Rekombination mutiert oder anderweitig verändert wird, aber immer noch ein funktionelles Protein codiert (z.B. kann der stromaufwärts gelegene regulatorische Bereich so verändert sein, daß dadurch die Expression der endogenen PSE verändert wird) . Zur Erzeugung einer Punktmutation über homologe Rekombination können auch als Chimeraplasty bekannte DNA-RNA-Hybride verwendet werden, die aus Cole-Strauss et al., 1999, Nucleic Acids Research 27(5):1323-1330 und Kmiec, Gene therapy, 19999, American Scientist, 87(3):240-247 bekannt sind.

Im Vektor für die homologe Rekombination ist der veränderte Abschnitt des PSE-Gens an seinem 5' - und 3'-Ende von zusätzlicher Nukleinsäure des PSE-Gens flankiert, so daß homologe Rekombination zwischen dem exogenen PSE-Gen, das auf dem Vektor vorliegt, und einem endogenen PSE-Gen in einem Mikroorganismus oder einer Pflanze möglich ist. Die zusätzliche flankierende PSE-Nukleinsäure ist für eine erfolgreiche homologe Rekombination mit dem endogenen Gen hinreichend lang. Gewöhnlich sind im Vektor mehrere hundert Basenpaare bis zu Kilobasen flankierende DNA (sowohl am 5'- als auch am 3'-Ende) enthalten [eine Beschreibung von Vektoren zur homologen Rekombination siehe z.B. in Thomas, K.R., und Capecchi, M.R. (1987) Cell 51:503 oder der Rekombination in Physcomitrella patens auf cDNA-Basis in Strepp et al., 1998, Proc. Natl. Acad. Sci. USA 95 (8):4368-4373]. Der Vektor wird in einen Mikroorganismus oder eine Pflanzenzelle (z.B. mittels Polyethylenglycol-vermittelter DNA) eingebracht, und Zellen, in denen das eingebrachte PSE-Gen mit dem endogenen PSE-Gen homolog rekombiniert ist, werden unter Verwendung im Fachgebiet bekannter Techniken selektiert.

Bei einer anderen Ausführungsform können rekombinante Organismen, wie Mikroorganismen der Pflanzen, hergestellt werden, die ausgewählte Systeme enthalten, welche eine regulierte Expression des eingebrachten Gens ermöglichen. Der Einschluß eines PSE-Gens in einem Vektor, wobei es unter die Kontrolle des lac-Operons gebracht wird, ermöglicht z.B. die Expression des PSE-Gens nur in Gegenwart von IPTG. Diese Regulationssysteme sind im Fachgebiet bekannt.

Eine erfindungsgemäße Wirtszelle, wie eine prokaryotische oder eukaryotische Wirtszelle, in Kultur oder auf einem Feld wachsend, kann zur Produktion (d.h. Expression) einer PSE verwendet werden. In Pflanzen kann zusätzlich ein alternatives Verfahren durch direkten Transfer von DNA in sich entwickelnde Blüten über Elektroporation oder Gentransfer mittels Agrobacterium angewendet werden. Die Erfindung stellt folglich ferner Verfahren zur Produktion von PSEs unter Verwendung der erfindungsgemäßen Wirtszellen bereit. Bei einer Ausführungsform umfaßt das Verfahren die Anzucht der erfindungsgemäßen Wirtszelle (in die ein rekombinanter Expressionsvektor, der eine PSE codiert, eingebracht worden ist, oder in deren Genom ein Gen eingebracht worden ist, das eine Wildtyp- oder veränderte PSE codiert) in einem geeigneten Medium, bis die PSE produziert worden ist. Das Verfahren umfaßt bei einer weiteren Ausführungsform das Isolieren der PSEs aus dem Medium oder der Wirtszelle.

Wirtszellen, die im Prinzip zum Aufnehmen der erfindungsgemäßen Nukleinsäure, des erfindungsgemäßen neuen Genproduktes oder des erfindungsgemäßen Vektors geeignet sind, sind alle prokaryotischen oder eukaryotischen Organismen. Die vorteilhafterweise verwendeten Wirtsorganismen sind Organismen, wie Bakterien, Pilze, Hefen, Tier- oder Pflanzenzellen. Weitere vorteilhafte Organismen sind Tiere oder vorzugsweise Pflanzen oder Teile davon. Unter dem Begriff "Tier" wird hier verstanden, daß Menschen nicht umfaßt sind. Pilze, Hefen oder Pflanzen werden vorzugsweise verwendet, besonders bevorzugt Pilze oder Pflanzen, ganz besonders bevorzugt Pflanzen, wie Ölfruchtpflanzen, die große Mengen an Lipidverbindungen enthalten, wie Raps, Nachtkerze, Rizinus, Canola, Erdnuß, Lein, Soja, Safflor, Sonnenblume, Borretsch, Ölpalme, Kokosnuß oder Pflanzen, wie Mais, Weizen, Roggen, Hafer, Triticale, Reis, Gerste, Baumwolle, Maniok, Pfeffer, Tagetes, Solanaceen-Pflanzen, wie Kartoffel, Tabak, Aubergine und Tomate, Vicia-Arten, Erbse, Alfalfa, Buschpflanzen (Kaffee, Kakao, Tee), Salix-Arten, Bäume (Ölplame, Kokosnuß) sowie ausdauernde Gräser und Futterfeldfrüchte. Besonders bevorzugte erfindungsgemäße Pflanzen sind Ölfruchtpflanzen, wie Soja, Erdnuß, Raps, Canola, Rizinus, Lein, Nachtkerze, Sonnenblume, Safflor, Bäume (Ölpalme, Kokosnuß).

In einem besonders bevorzugten Aspekt betrifft die Erfindung eine Pflanzenzelle, die das erfindungsgemäße Polynukleotid oder den erfindungsgemäßen Vektor umfaßt. Weiter bevorzugt sind transgene Pflanzen oder Pflanzengewebe, die die erfindungsgemäße Pflanzenzelle umfassen. In einem weiteren Aspekt betrifft die vorliegende Erfindung jene Teile der erfindungsgemäßen Pflanzen, die man ernten kann, und jenes Material, das sich für die Vermehrung der transgenen Pflanzen gemäß der Erfindung eignet und welche entweder erfindungsgemäßen Pflanzenzellen, die die erfindungsgemäße Nukleinsäure exprimieren, oder Zellen enthalten, die einen erhöhten Level des erfindungsgemäßen Proteins besitzen. Erntebar sind prinzipiell alle Teile einer Pflanze, insbesondere Blüten, Pollen, Früchte, Sämlinge, Wurzeln, Blätter, Samen, Knollen, Stengel usw.. Vermehrungsmaterial umfaßt z.B. Samen, Früchte, Sämlinge, Knollen, Schnitte und Wurzelstöcke.

### D. Isolierte PSE

Ein weiterer Aspekt der Erfindung betrifft isolierte PSEs und biologisch aktive Teile davon. Ein "isoliertes" oder "gereinigtes" Protein oder ein biologisch aktiver Teil davon ist im wesentlichen frei von zellulärem Material, wenn es durch DNA-Rekombinationstechniken produziert wird, oder von chemischen Vorstufen oder andern Chemikalien, wenn es chemisch synthetisiert wird. Der Begriff "im wesentlichen frei von zellulärem Material" umfaßt PSE-Präparationen, in denen das Protein von zellulären Komponenten der Zellen, in denen es natürlich oder rekombinant produziert wird, getrennt ist. Bei einer Ausführungsform umfaßt der Ausdruck "im wesentlichen frei von zellulärem Material" PSE-Präparationen mit weniger als etwa 30 % (bezogen auf das Trockengewicht) nicht-PSE (hier auch als "verunreinigendes Protein" bezeichnet), stärker bevorzugt weniger als etwa 20 % nicht-PSE, noch stärker bevorzugt weniger als etwa 10 % nicht-PSE und am stärksten bevorzugt weniger als etwa 5 % nicht-PSE. Wenn die PSE oder ein biologisch aktiver Teil davon rekombinant hergestellt worden ist, ist sie/er auch im wesentlichen frei von Kulturmedium, d.h. das Kulturmedium macht weniger als etwa 20 %, stärker bevorzugt weniger als etwa 10 % und am stärksten bevorzugt weniger als etwa 5 % des Volumens der Proteinpräparation aus. Der Begriff "im wesentlichen frei von chemischen Vorstufen oder anderen Chemikalien" umfaßt PSE-Präparationen, in denen das Protein von chemischen Vorstufen oder anderen Chemikalien getrennt ist, die an der Synthese des Proteins beteiligt sind. Bei einer Ausführungsform umfaßt der Begriff "im wesentlichen frei von chemischen Vorstufen oder anderen Chemikalien" PSE-Präparationen mit weniger als etwa 30 % (bezogen auf das Trockengewicht) chemischen Vorstufen oder nicht-PSE-Chemikalien, stärker bevorzugt weniger als etwa 20 % chemischen Vorstufen oder nicht-PSE-Chemikalien, noch stärker bevorzugt weniger als etwa 10 % chemischen Vorstufen oder nicht-PSE-Chemikalien und am stärksten bevorzugt weniger als etwa 5 % chemischen Vorstufen oder nicht-PSE-Chemikalien. Bei bevorzugten Ausführungsformen weisen isolierte Proteine oder biologisch aktive Teile davon keine verunreinigenden Proteine aus dem gleichen Organismus auf, aus dem die PSE stammt. Im Falle des erfindungsgemäßen Proteins, das die Sequenz, die in SEQ ID NO: 10 gezeigt wird, oder das von einem Gen, das SEQ IDNO: 9 umfasst, codiertwird, ist jedoch zu berücksichtigen, dass die Sequenz mit zwei Met beginnt. Dies kann dazu führen, dass bei der Translation einer entsprechenden codierenden Nukleinsäuresequenz zwei Derivate des erfindungsgemäßen Proteins exprimiert werden, beginnend am ersten oder zweiten Met. Das Expressionsverhältnis zwischen beiden Derivaten kann je nach Expressionsart oder Wirtsorganismus zwischen 0 und 1 schwanken. Umfasst ist somit PSE, das beide genannte Derivate oder nur eins der Derivate enthält. Die beiden Derivate können unterschiedliche Aktivitäten, Lokalisationen, Halbwertszeiten, Regulationsmechanismen etc. aufweisen. Diese Proteine werden gewöhnlich durch rekombinante Expression zum Beispiel Physcomitrella-, Phytophthora-, Crypthecodinium- oder Thraustochytrium-PSE in Pflanzen wie Physcomitrella patens bzw. o.g. oder Mikroorganismen, beispielsweise Bakterien, wie E. coli, Bacillus subtilis, C. glutamicum, Pilzen, wie Mortierella, Hefe, wie Saccharomyces, oder Ciliaten wie Colpidium oder Algen wie Phaeodactylum hergestellt.

Eine erfindungsgemäße isolierte PSE oder ein Teil davon kann auch am Stoffwechsel von zum Aufbau von Zellmembranen in Physcomitrella, Phytophthora, oder Thraustochytrium notwendigen Verbindungen oder am Transport von Molekülen über diese Membranen teilnehmen. Bei bevorzugten Ausführungsformen umfaßt das Protein oder der Teil davon eine Aminosäuresequenz, die ausreichend homolog zu einer Aminosäuresequenz der SEQ ID NO:2, SEQ ID NO:4, und SEQ ID NO:10 ist, daß das Protein oder der Teil davon die Fähigkeit, am Stoffwechsel von zum Aufbau von Zellmembranen in Physcomitrella, Phytophthora, oder Thraustochytrium notwendigen Verbindungen oder am Transport von Molekülen über diese Membranen teilzunehmen, beibehält. Der Teil des Proteins ist vorzugsweise ein biologisch aktiver Teil, wie hier beschrieben. Bei einer weiteren bevorzugten Ausführungsform hat eine erfindungsgemäße PSE eine der in SEQ ID NO:2, SEQ ID NO:4, und SEQ ID NO:10 gezeigten Aminosäuresequenzen. Bei einer weiteren bevorzugten Ausführungsform hat die PSE eine Aminosäuresequenz, die von einer Nukleotidsequenz codiert wird, die, zum Beispiel unter stringenten Bedingungen, an eine Nukleotidsequenz der SEQ ID NO:1, SEQ ID NO:3, und SEQ ID NO:9 hybridisiert. Bei noch einer weiteren bevorzugten Ausführungsform hat die PSE eine Aminosäuresequenz, die von einer Nukleotidsequenz codiert wird, die mindestens etwa 50 bis 60 %, vorzugsweise mindestens etwa 60 bis 70 %, stärker bevorzugt mindestens etwa 70 bis 80 %, 80 bis 90 %, 90 bis 95 % und noch stärker bevorzugt mindestens etwa 96 %, 97 %, 98 %, 99 % oder noch homologer zu einer der Aminosäuresequenzen der SEQ ID NO:2, SEQ ID NO:4, und SEQ ID NO:10 ist. Die erfindungsgemäße bevorzugte PSE besitzt vorzugsweise auch mindestens eine der hier beschriebenen PSE-Aktivitäten. Zum Beispiel umfaßt eine erfindungsgemäße bevorzugte PSE eine Aminosäuresequenz, die von einer Nukleotidsequenz codiert wird, die, zum Beispiel unter stringenten Bedingungen, an eine Nukleotidsequenz der SEQ ID NO:1, SEQ ID NO:3, und SEQ ID NO:9 hybridisiert und am Stoffwechsel von zum Aufbau von Zellmembranen in Physcomitrella, Phytophthora, Crypthecodinium oder Thraustochytrium notwendigen Verbindungen oder am Transport von Molekülen über diese Membranen teilnehmen kann oder eine oder mehrere polyungesättigte Fettsäuren mit wenigstens zwei Doppelbindungen und einer Kettenlänge von C₁₆ oder C₁₈ verlängern kann.

Bei anderen Ausführungsformen ist die PSE im wesentlichen homolog zu einer Aminosäuresequenz der SEQ ID NO:2, SEQ ID NO:4, und SEQ ID NO:10 und behält die funktionelle Aktivität des Proteins einer der sequenzen der SEQ ID NO:2, SEQ ID NO:4, und SEQ ID NO:10 bei, ihre Aminosäuresequenz unterscheidet sich jedoch aufgrund von natürlicher Variation oder Mutagenese, wie eingehend im obigen Unterabschnitt I beschrieben. Bei einer weiteren Ausführungsform ist die PSE folglich ein Protein, das eine Aminosäuresequenz umfaßt, die mindestens etwa 50 bis 60 %, vorzugsweise mindestens etwa 60 bis 70 % und stärker bevorzugt mindestens etwa 70 bis 80 %, 80 bis 90 %, 90 bis 95 % und am stärksten bevorzugt mindestens etwa 96 %, 97 %, 98 %, 99 % oder noch homologer zu einer vollständigen Aminosäuresequenz der SEQ ID NO:2, SEQ ID NO:4, und SEQ ID NO:10 ist und zumindest eine der hier beschriebenen PSE-Aktivitäten aufweist. Bei einer anderen Ausführungsform betrifft die Erfindung ein vollständiges Physcomitrella-, Phytophthora-, oder Thraustochytrium -Protein, das im wesentlichen homolog zu einer vollständigen Aminosäuresequenz der SEQ ID NO:2, SEQ ID NO:4, und SEQ ID NO:10 ist.

Biologisch aktive Teile einer PSE umfassen Peptide, umfassend Aminosäuresequenzen, die von der Aminosäuresequenz einer PSE hergeleitet sind, z.B. eine in SEQ ID NO:2, SEQ ID NO:4, und SEQ ID NO:10 gezeigte Aminosäuresequenz oder die Aminosäuresequenz eines Proteins, das zu einer PSE homolog ist, welche weniger Aminosäuren als die Vollängen-PSE oder das Vollängenprotein aufweisen, das zu einer PSE homolog ist, und zumindest eine Aktivität einer PSE aufweisen. Gewöhnlich umfassen biologisch aktive Teile (Peptide, z.B. Peptide, die zum Beispiel 5, 10, 15, 20, 30, 35, 36, 37, 38, 39, 40, 50, 100 oder mehr Aminosäuren lang sind) eine Domäne oder ein Motiv mit mindestens einer Aktivität einer PSE. Über dies können andere biologisch aktive Teile, in denen andere Bereiche des Proteins deletiert sind, durch rekombinante Techniken hergestellt und bezüglich einer oder mehrerer der hier beschriebenen Aktivitäten untersucht werden. Die biologisch aktiven Teile einer PSE umfassen vorzugsweise ein/eine oder mehrere ausgewählte Domänen/Motive oder Teile davon mit biologischer Aktivität.

Solche Domänen und Motive können zum Teil durch Sequenzanalyse, z.B. mithilfe von EDV-gestützten Methoden, identifiziert werden.

In den erfindungsgemäßen Sequenzen wurden z.B. sogenannte KK-Methoden gefunden.

Kermode 1996, Critical Reviews in Plant Sciences 15 (4):285-423 beschreibt KK-Motive, ein Doppel-Lysin, das vornehmlich als KKXX oder K X K XXX Motiv gefunden wird und ein Recycling vom ER zum Golgi Apparat und damit die Verweilzeit des Proteins und seiner Enzymaktivität an einem bestimmten Ort, insbesondere dem ER beeinflusst.

Doppel-Lysin-Motive wurden z.B. auch in Δ12-Desaturasen gefunden (Arondel et al. 1992, Science 258:1353) und liegen auch in erfindungsgemäßen Elongasen vor. Insbesondere sind solche Motive C-terminal lokalisierbar beschrieben. In erfindungsgemäßen und hier beschriebenen Sequenzen findet sich eine auffällige Anhäufung von Lysinen am C-Terminus.

| | |
|---|---|
| Moos Elongase PSE1: C-Terminus | KQKGAKTE |
| SEQ ID NO 2: | KTKKA |
| SEQ ID NO 4 | KKSTPAAKKTN |
| SEQ ID NO 6: | KHLK |

Es könnte sich hiermit um eine mögliche Genvariation handeln.

Es gibt Lys Reste, die das Targeting, die Adressierung und Lokalisation am oder im ER beeinflussen. Eine Maskierung dieser Sequenz, Modifizierung oder räumlich Modifikation, der Nähe zum Ende des C-Terminus z.B. durch Fusion mit GFP "green fluorescent protein" kann zur Beeinflussung der Kompartimentierung genutzt werden.

PSEs werden vorzugsweise durch DNA-Rekombinationstechniken hergestellt. Zum Beispiel wird ein das Protein codierendes Nukleinsäuremolekül in einen Expressionsvektor (wie vorstehend beschrieben) kloniert, der Expressionsvektor wird in eine Wirtszelle (wie vorstehend beschrieben) eingebracht, und die PSE wird in der Wirtszelle exprimiert. Die PSE kann dann durch ein geeignetes Reinigungsschema mittels Standard-Proteinreinigungstechniken aus den Zellen isoliert werden. Alternativ zur rekombinanten Expression kann eine PSE, ein -Polypeptid, oder -Peptid mittels Standard-Peptidsynthesetechniken chemisch synthetisiert werden. Überdies kann native PSE aus Zellen (z.B. Endothelzellen) z.B. unter Verwendung eines Anti-PSE-Antikörpers isoliert werden, der durch Standardtechniken produziert werden kann, wobei eine erfindungsgemäße PSE oder ein Fragment davon verwendet wird.

Die Erfindung stellt auch chimäre PSE-Proteine oder PSE-Fusionsproteine bereit. Wie hier verwendet, umfaßt ein "chimäres PSE-Protein" oder "PSE-Fusionsprotein" ein PSE-Polypeptid, das funktionsfähig an ein nicht-PSE-Polypeptid gebunden ist. Ein "PSE-Polypeptid" betrifft ein Polypeptid mit einer Aminosäuresequenz, die einer PSE entspricht, wohingegen ein "nicht-PSE-Polypeptid" ein Polypeptid mit einer Aminosäuresequenz betrifft, die einem Protein entspricht, das im wesentlichen nicht homolog zu der PSE ist, z.B. ein Protein, das sich vom der PSE unterscheidet und aus dem gleichen oder einem anderen Organismus stammt. Innerhalb des Fusionsproteins soll der Begriff "funktionsfähig verbunden" bedeuten, daß das PSE-Polypeptid und das nicht-PSE-Polypeptid so miteinander fusioniert sind, daß beide Sequenzen die vorhergesagte, der verwendeten Sequenz zugeschriebene Funktion erfüllen. Das nicht-PSE-Polypeptid kann an den N-Terminus oder den C-Terminus des PSE-Polypeptids fusioniert sein. Bei einer Ausführungsform ist das Fusionsprotein zum Beispiel ein GST-PSE-Fusionsprotein, bei dem die PSE-Sequenzen an den C-Terminus der GST-Sequenzen fusioniert sind. Diese Fusionsproteine können die Reinigung der rekombinanten PSEs erleichtern. Bei einer weiteren Ausführungsform ist das Fusionsprotein eine PSE, die eine heterologe Signalsequenz an ihrem N-Terminus aufweist. In bestimmten Wirtszellen (z.B. Säuger-Wirtszellen) kann die Expression und/oder Sekretion einer PSE durch Verwendung einer heterologen Signalsequenz gesteigert werden.

Ein erfindungsgemäßes chimäres PSE-Protein oder PSE-Fusionsprotein wird durch Standard-DNA-Rekombinationstechniken hergestellt. Zum Beispiel werden DNA-Fragmente, die unterschiedliche Polypeptidsequenzen codieren, gemäß herkömmlicher Techniken im Leseraster aneinander ligiert, indem beispielsweise glatte oder überhängende Enden zur Ligation, Restriktionsenzymspaltung zur Bereitstellung geeigneter Enden, Auffüllen kohäsiver Enden, wie erforderlich, Behandlung mit alkalischer Phosphatase, um ungewollte Verknüpfungen zu vermeiden, und enzymatische Ligation eingesetzt werden. Bei einer weiteren Ausführungsform kann das Fusionsgen durch herkömmliche Techniken, einschließlich DNA-Syntheseautomaten, synthetisiert werden. Alternativ kann eine PCR-Amplifizierung von Genfragmenten unter Verwendung von Ankerprimern durchgeführt werden, die komplementäre Überhänge zwischen aufeinanderfolgenden Genfragmenten erzeugen, die anschließend miteinander hybridisiert und reamplifiziert werden können, so daß eine chimäre Gensequenz erzeugt wird (siehe zum Beispiel Current Protocols in Molecular Biology, Hrsgb. Ausubel et al., John Wiley & Sons: 1992) . Überdies sind viele Expressionsvektoren kommerziell erhältlich, die bereits eine Fusionseinheit (z.B. ein GST-Polypeptid) codieren. Eine PSE-codierende Nukleinsäure kann in einen solchen Expressionsvektor kloniert werden, so daß die Fusionseinheit im Leseraster mit dem PSE-Protein verbunden ist.

Homologe der PSE können durch Mutagenese, z.B. durch spezifische Punktmutation oder Verkürzung der PSE, erzeugt werden. Der Begriff "Homologe", wie hier verwendet, betrifft eine variante Form der PSE, die als Agonist oder Antagonist der PSE-Aktivität wirkt. Ein Agonist der PSE kann im wesentlichen die gleiche Aktivität wie die oder einen Teil der biologischen Aktivitäten der PSE beibehalten. Ein Antagonist der PSE kann eine oder mehrere Aktivitäten der natürlich vorkommenden Form der PSE durch zum Beispiel kompetitive Bindung an ein stromabwärts oder -aufwärts gelegenes Element der Stoffwechselkaskade für Zellmembrankomponenten, welche die PSE umfaßt, oder durch Bindung an eine PSE, welche den Transport von Verbindungen über Zellmembranen vermittelt, hemmen, wodurch die Translokation gehemmt wird.

Bei einer alternativen Ausführungsform können Homologe der PSE durch Sichten kombinatorischer Banken von Mutanten, z.B. Verkürzungsmutanten, der PSE hinsichtlich PSE-Agonisten- oder -Antagonisten-Aktivität identifiziert werden. Bei einer Ausführungsform wird eine variegierte Bank von PSE-Varianten durch kombinatorische Mutagenese auf Nukleinsäureebene erzeugt und durch eine variegierte Genbank codiert. Eine variegierte Bank von PSE-Varianten kann z.B. durch enzymatische Ligation eines Gemisches von synthetischen Oligonukleotiden in Gensequenzen hergestellt werden, so daß sich ein degenerierter Satz potentieller PSE-Sequenzen als individuelle Polypeptide oder alternativ als Satz größerer Fusionsproteine (z.B.für das Phage-Display), die diesen Satz von PSE-Sequenzen enthalten, exprimieren läßt. Es gibt eine Vielzahl von Verfahren, die zur Herstellung von Banken potentieller PSE-Homologen aus einer degenerierten Oligonukleotidsequenz verwendet werden können. Die chemische Synthese einer degenerierten Gensequenz kann in einem DNA-Syntheseautomaten durchgeführt und das synthetische Gen dann in einen geeigneten Expressionsvektor ligiert werden. Die Verwendung eines degenerierten Satzes von Genen ermöglicht die Bereitstellung sämtlicher Sequenzen, die den gewünschten Satz an potentiellen PSE-Sequenzen codieren, in einem Gemisch. Verfahren zur Synthese degenerierter Oligonukleotide sind im Fachgebiet bekannt [siehe z.B. Narang, S.A. (1983) Tetrahedron 39:3; Itakura et al. (1984) Annu. Rev. Biochem. 53:323; Itakura et al., (1984) Science 198:1056; Ike et al. (1983) Nucleic Acids Res. 11:477].

Zusätzlich können Banken von PSE-Fragmenten zur Herstellung einer variegierten Population von PSE-Fragmenten für das Sichten und für die anschließende Selektion von Homologen einer PSE verwendet werden. Bei einer Ausführungsform kann eine Bank von Fragmenten der codierenden Sequenz durch Behandeln eines doppelsträngigen PCR-Fragmentes einer codierenden PSE-Sequenz mit einer Nuklease unter Bedingungen, unter denen Doppelstrangbrüche nur etwa einmal pro Molekül erfolgen, Denaturieren der doppelsträngigen DNA, Renaturieren der DNA unter Bildung doppelsträngiger DNA, welche Sense/Antisense-Paare von verschiedenen Produkten mit Doppelstrangbrüchen umfassen kann, Entfernen einzelsträngiger Abschnitte aus neu gebildeten Duplices durch Behandlung mit S1-Nuklease und Ligieren der resultierenden Fragmentbank in einen Expressionsvektor erzeugt werden. Mit diesem Verfahren kann eine Expressionsbank hergeleitet werden, die N-terminale, C-terminale und interne Fragmente der PSE verschiedener Größen codiert.

Im Fachgebiet sind mehrere Techniken für das Sichten von Genprodukten in kombinatorischen Banken, die durch Punktmutationen oder Verkürzung hergestellt worden sind, und für das Sichten von cDNA-Banken nach Genprodukten mit einer ausgewählten Eigenschaft bekannt. Diese Techniken lassen sich an das schnelle Sichtung der Genbanken anpassen, die durch kombinatorische Mutagenese von PSE-Homologen erzeugt worden sind. Die am häufigsten verwendeten Techniken zum Sichtung großer Genbanken, die einer Analyse mit hohem Durchsatz unterworfen werden können, umfassen gewöhnlich das Klonieren der Genbank in replizierbare Expressionsvektoren, Transformieren von geeigneten Zellen mit der resultierenden Vektorenbank und Exprimieren der kombinatorischen Gene unter Bedingungen, unter denen der Nachweis der gewünschten Aktivität die Isolation des Vektors, der das Gen codiert, dessen Produkt nachgewiesen wurde, erleichtert. Recursive-Ensemble-Mutagenese (REM), eine neue Technik, die die Häufigkeit funktioneller Mutanten in den Banken erhöht, kann in Kombination mit den Sichtungstests zur Identifikation von PSE-Homologen verwendet werden [Arkin und Yourvan (1992) Proc. Natl. Acad. Sci. USA 89:7811-7815; Delgrave et al. (1993) Protein Engineering 6(3):327-331]. Es können auch vorteilhaft Kombinationen der oben genannten Methoden verwendet werden.

Eine weitere bekannte Technik zur Veränderung von katalytischen Eigenschaften von Enzymen bzw. deren codierenden Genen ist das sogenannte "Gen-Shuffling" (siehe z.B. WO 97/20078 oder WO 98/13487), das eine Kombination von Genfragmenten darstellt, wobei diese Neukombination zusätzlich noch durch fehlerhafte Polymerasekettenreaktionen variiert werden kann und somit eine hohe zu testende Sequenzdiversität schafft. Voraussetzung für den Einsatz eines solchen Ansatzes ist jedoch ein geeignetes Screeningsystem, um die erstellte Gendiversität auf Funktionalität zu überprüfen. Insbesondere für die Sichtung von Elongaseaktivitäten ist ein Sichtungsverfahren Voraussetzung, das PUFA-abhängig Enzymaktivität(en) erfaßt. Bzgl. Elongaseaktivitäten mit Spezifität für PUFAs kann man in Mucor-Species, die durch bekannte Transformationsverfahren mit gewünschten Genkonstrukten transformierbar sind, die Toxizität von Arachidonsäure in Anwesenheit eines toxischen Metaboliten (hier: Salicylsäure oder Salicylsäurederivate) nutzen (Eroshin et al., Mikrobiologiya, Vol. 65, No. 1, 1996, Seiten 31-36), um eine wachstumsbasierte Erstsichtung durchzuführen. Resultierende Klone können dann einer Analyse ihrer Lipidinhaltstoffe mittels Gaschromatographie und Massenspektroskopie unterzogen werden, um Edukte und Produkte in Art und Menge zu erfassen.

Bei einer weiteren Ausführungsform können Tests auf Zellbasis zur Analyse einer variegierten PSE-Bank unter Verwendung von weiteren im Fachgebiet bekannten Verfahren ausgenutzt werden.

In einer weiteren Ausführungsform betrifft die vorliegende Erfindung einen Antikörper, der spezifisch mit dem Polypeptid der vorliegenden Erfindung oder Teilen, z.B. Epitopen, eines solchen Proteins bindet. Der erfindungsgemäße Antikörper kann verwendet werden, um andere Elongasen, insbesondere PSEs, zu identifizieren und zu isolieren. Diese Antikörper können monoklonale Antikörper, polyklonale Antikörper oder synthetische Antikörper sowie Fragmente dieser Antikörper, wie z.B. Fab-, Fv- oderscFV-Fragmente usw., sein. Monoklonale Antikörper können z.B. nach Verfahren, wie ursprünglich beschrieben bei Köhler und Milstein, Nature 256 (1975), 485, und Galfrö, Meth. Enzymol. 73 (1981), hergestellt werden. Antikörper und Fragmente davon können auch hergestelltwerdennachz.B. in Harlow & Lane, "Antibodies, aLaboratory Manual", CSH, Press, Cold Spring Harbor, 1988. Diese Antikörper können verwendet werden, um z.B. die erfindungsgemäßen Proteine zu präzipitieren und lokalisieren oder um die Synthese dieser Proteine z.B. in rekombinanten Organismen zu überprüfen sowie für die Identifikation von Verbindungen, die mit den erfindungsgemäßen Proteinen interagieren. In vielen Fällen ist die BindungvonAntikörpern mit Antigenen äquivalent zu der Bindung anderer Liganden und Antiliganden.

Weiterhin beschreibt die vorliegende Erfindung ein Verfahren zur Identifikation eines Agonisten oder Antagonisten von Elongasen, insbesondere PSEs, umfassend
a) in Kontaktbringen der Zellen, die das Polypeptid der vorliegenden Erfindung exprimieren, mit einem Kandidatenstoff;
b) Testen der PSE-Aktivität;
c) Vergleichen der PSE-Aktivität mit einer Standardaktivität in Abwesenheit des Kandidatenstoffs, wobei ein Anstieg der PSE-Aktivität über den Standard anzeigt, daß der Kandidatenstoff ein Agonist und ein Verringerung der PSE-Aktivität anzeigt, daß der Kandidatenstoff ein Antagonist ist.

Der genannte Kandidatenstoff kann ein chemisch synthetisierter oder mirkobiologisch produzierter Stoff sein und z.B. in Zellextrakten von z.B. Pflanzen, Tieren oder Mikroorganismen auftreten. Weiterhin kann der genannte Stoff zwar im Stand der Technik bekannt sein, aber bisher nicht bekannt sein als die Aktivität der PSEs steigernd oder repremierend. Das Reaktionsgemisch kann ein zellfreier Extrakt sein oder eine Zelle oder Zellkultur umfassen. Geeignete Methoden sind dem Fachmann bekannt und werden z.B. allgemein beschrieben in Alberts, Molecular Biology the cell, 3rd Edition (1994), z.B. Kapitel 17. Die genannten Stoffe können z.B. zu dem Reaktionsgemisch oder dem Kulturmedium zugegeben werden oder den Zellen injiziert werden oder auf eine Pflanze gesprüht werden.

Wenn eine Probe, die ein nach der beschriebenen Methode aktiven Stoff beinhaltet, identifiziert wurde, dann ist es entweder möglich, den Stoff direkt von der ursprünglichen Probe zu isolieren oder man kann die Probe in verschiedene Gruppen teilen, z.B. wenn sie aus einer Vielzahl von verschiedenen Komponenten besteht, um so die Zahl der verschiedenen Substanzen pro Probe zu reduzieren und dann das beschriebene Verfahren mit einer solchen "Unterprobe" der ursprünglichen Probe zu wiederholen. Abhängig von der Komplexität der Probe können die oben beschriebenen Schritte mehrmals wiederholt werden, vorzugsweise bis die gemäß der beschriebenen Methoden identifizierte Probe nur noch eine geringe Anzahl von Substanzen oder nur noch eine Substanz umfaßt. Vorzugsweise wird der gemäß der beschriebenen Methode identifizierte Stoff oder Derivate davon weiter formuliert, so, daß er für die Anwendung in der Pflanzenzüchtung oder Pflanzenzell- oder Gewebekultur geeignet ist.

Die Stoffe, die gemäß dem beschriebenen Verfahren getestet und identifiziert wurden, können sein: Expressionsbibliotheken, z.B. cDNA-Expressionsbibliotheken, Peptide, Proteine, Nukleinsäuren, Antikörper, kleine organische Stoffe, Hormone, PNAs oder ähnliches (Milner, NatureMedicin (1995), 879-880; Hupp, Cell. 83 (1995), 237-245; Gibbs, Cell. 79 (1994), 193-198 und darin zitierte Referenzen) . Diese Stoffe könne auch funktionelle Derivate oder Analogon der bekannten Inhibitoren oder Aktivatoren sein. Verfahren zur Herstellung von chemischen Derivaten oder Analogon sind dem Fachmann bekannt. Die genannten Derivate und Analogon können gemäß Verfahren nach dem Stand der Technik getestet werden. Weiterhin kann computergestütztes Design oder Peptidomimetics zur Herstellung geeigneter Derivate und Analogon verwendet werden. Die Zelle oder das Gewebe, die/das für das erfindungsgemäße Verfahren verwendet werden kann, ist vorzugsweise eine erfindungsgemäße Wirtszelle, Pflanzenzelle oder ein Pflanzengewebe, wie in den oben genannten Ausführungsformen beschrieben.

Entsprechend beschreibt die vorliegende Erfindung auch einen Stoff, der gemäß den vorstehenden erfindungsgemäßen Verfahrenidentifiziert wurde. Der Stoff ist z.B. ein Homolog der erfindungsgemäßen PSE. Homologe der PSEs können durch Mutagenese, z.B. durch Punktmutation oder Deletion der PSE, erzeugt werden. Hierin verwendet wird der Begriff "Homolog" als eine variante Form der PSEs, die als Agonist oder Antagonist für die Aktivität der PSEs wirkt. Ein Agonist kann die im wesentlichen gleiche oder einen Teil der biologischen Aktivität der PSEs haben. Ein Antagonist der PSEs kann eine oder mehr Aktivitäten der natürlich vorkommenden Formen der PSEs inhibieren, z.B. kompetitiv an ein *Downstream* oder *Upstream* gelegenes Mitglied der Fettsäuresynthese-Stoffwechselwege, die die PSEs einschließen, binden oder an PSEs binden und dabei die Aktivität reduzieren oder inhibieren.

Folglich betrifft die vorliegende Erfindung auch ein Antikörper oder ein Fragment davon, wie sie hierin beschrieben werden, der die Aktivität der erfindungsgemäßen PSEs inhibiert.

Bei einem Aspekt beschreibt die vorliegende Erfindung ein Antikörper, der spezifisch den oben beschriebenen Agonisten oder Antagonisten erkennt bzw. bindet.

Ein weiterer Aspekt betrifft eine Zusammensetzung, die den Antikörper, den nach dem erfindungsgemäßen Verfahren identifizierten Stopp oder das Antisense-Molekül umfaßt.

### E. Erfindungsgemäße Verwendungen und Verfahren

Die hier beschriebenen Nukleinsäuremoleküle, Proteine, Proteinhomologen, Fusionsproteine, Antikörper, Primer, Vektoren und Wirtszellen können bei einem oder mehreren der nachstehenden Verfahren verwendet werden: Identifikation Physcomitrella patens, Crypthecodinium, Phytophthora infestans oder Thraustochytrium und verwandten Organismen, Kartierung der Genome von Organismen, die mit Physcomitrella, Phytophthora, Crypthecodinium oder Thraustochytrium verwandt sind, Identifikation und Lokalisierung von Physcomitrella-, Phytophthora-, Crypthecodinium- oder Thraustochytrium -Sequenzen von Interesse, Evolutionsstudien, Bestimmung von PSE-Proteinbereichen, die für die Funktion notwendig sind, Modulation einer PSE-Aktivität; Modulation des Stoffwechsels einer oder mehrerer Zellmembrankomponenten; Modulation des Transmembrantransports einer oder mehrerer Verbindungen sowie Modulation der zellulären Produktion einer gewünschten Verbindung, wie einer Feinchemikalie. Die erfindungsgemäßen PSE-Nukleinsäuremoleküle haben eine Vielzahl von Verwendungen. Sie können zunächst zur Identifikation eines Organismus als Physcomitrella, Phytophthora, oder Thraustochytrium oder als naher Verwandter davon verwendet werden. Sie können auch zur Identifikation des Vorliegens von Physcomitrella, Phytophthora oder Thraustochytrium oder eines Verwandten davon in einer Mischpopulation von Mikroorganismen verwendet werden. Die Erfindung stellt die Nukleinsäuresequenzen einer Reihe von Physcomitrella-, Phytophthora-, oder Thraustochytrium -Genen bereit; durch Sondieren der extrahierten genomischen DNA einer Kultur einer einheitlichen oder gemischten Population von Mikroorganismen unter stringenten Bedingungen mit einer Sonde, die einen Bereich eines Physcomitrella-, Phytophthora- oder Thraustochytrium -Gens oder von Teilen davon überspannt, das für diesen Organismus einzigartig ist, kann man bestimmen, ob dieser Organismus vorliegt. Physcomitrella, Phytophthora oder Thraustochytrium selbst werden zur kommerziellen Produktion mehrfach ungesättigter Säuren verwendet. Die erfindungsgemäßen Nukleinsäuren eignen sich darüber hinaus zur PUFA-Produktion auch in anderen Organismen insbesondere wenn erreicht werden soll, daß resultierende PUFAs auch in die Triacylglycerolfraktion eingebaut werden sollen.

Ferner können die erfindungsgemäßen Nukleinsäure- und Proteinmoleküle als Marker für spezifische Bereiche des Genoms dienen. Diese sind nicht nur zur Kartierung des Genoms, sondern auch für funktionelle Studien von Physcomitrella-, Phytophthora-, oder Thraustochytrium -Proteinen geeignet. Zur Identifikation des Genombereichs, an den ein bestimmtes DNA-bindendes Protein von Physcomitrella, Phytophthora oder Thraustochytrium bindet, könnte das Physcomitrella-, Phytophthora- oder Thraustochytrium -Genom zum Beispiel gespalten werden und die Fragmente mit dem DNA-bindenden Protein inkubiert werden. Diejenigen, die das Protein binden, können zusätzlich mit den erfindungsgemäßen Nukleinsäuremolekülen, vorzugsweise mit leicht nachweisbaren Markierungen, sondiert werden; die Bindung eines solchen Nukleinsäuremoleküls an das Genomfragment ermöglicht die Lokalisierung des Fragments auf der Genomkarte von Physcomitrella, Phytophthora, oder Thraustochytrium und erleichtert, wenn dies mehrmals mit unterschiedlichen Enzymen durchgeführt wird, eine rasche Bestimmung der Nukleinsäuresequenz, an die das Protein bindet. Die erfindungsgemäßen Nukleinsäuremoleküle können zudem ausreichend homolog zu den Sequenzen verwandter Arten sein, daß diese Nukleinsäuremoleküle als Marker für die Konstruktion einer genomischen Karte bei verwandten Pilzen oder Algen dienen können.

Die erfindungsgemäßen PSE-Nukleinsäuremoleküle eignen sich auch für Evolutions- und Proteinstruktur-Untersuchungen. Die Stoffwechsel- und Transportprozesse, an denen die erfindungsgemäßen Moleküle beteiligt sind, werden von vielen prokaryotischen und eukaryotischen Zellen genutzt; durch Vergleich der Sequenzen der erfindungsgemäßen Nukleinsäuremoleküle mit solchen, die ähnliche Enzyme aus anderen Organismen codieren, kann der Evolutions-Verwandschaftsgrad der Organismen bestimmt werden. Entsprechend ermöglicht ein solcher Vergleich die Bestimmung, welche Sequenzbereiche konserviert sind und welche nicht, was bei der Bestimmung von Bereichen des Proteins hilfreich sein kann, die für die Enzymfunktion essentiell sind. Dieser Typ der Bestimmung ist für Proteinengineering-Untersuchungen wertvoll und kann einen Hinweis darauf geben, wieviel Mutagenese das Protein tolerieren kann, ohne die Funktion zu verlieren.

Die Manipulation der erfindungsgemäßen PSE-Nukleinsäuremoleküle kann zur Produktion von PSEs mit funktionellen Unterschieden zu den Wildtyp-PSEs führen. Die Effizienz oder Aktivität dieser Proteine kann verbessert sein, sie können in größeren Anzahlen als gewöhnlich in der Zelle zugegen sein, oder ihre Effizienz oder Aktivität kann verringert sein. Verbesserte Effizienz oder Aktivität bedeutet beispielsweise, daß das Enzym eine höhere Selektivität und/oder Aktivität, vorzugsweise eine mindestens 10 % höhere, besonders bevorzugt eine mindestens 20 % höhere Aktivität, ganz besonders bevorzugt eine mindestens 30 % höhere Aktivität als das ursprüngliche Enzym aufweist.

Es gibt eine Reihe von Mechanismen, durch die die Veränderung einer erfindungsgemäßen PSE die Ausbeute, Produktion und/oder Effizienz der Produktion einer Feinchemikalie, welche ein solches verändertes Protein enthält, direkt beeinflussen kann. Die Gewinnung von Feinchemikalien-Verbindungen aus Kulturen von Ciliaten, Algen oder Pilzen im großen Maßstab ist signifikant verbessert, wenn die Zelle die gewünschten Verbindungen sezerniert, da diese Verbindungen aus dem Kulturmedium (im Gegensatz zur Extraktion aus der Masse der gezüchteten Zellen) leicht gereinigt werden können. Ansonsten läßt sich die Reinigung verbessern, wenn die Zelle in vivo Verbindungen in einem spezialisierten Kompartiment mit einer Art Konzentrationsmechanismus speichert. Bei Pflanzen, die PSEs exprimieren, kann ein gesteigerter Transport zu besserer Verteilung innerhalb des Pflanzengewebes und der -organe führen. Durch Vergrößern der Anzahl oder der Aktivität von Transportermolekülen, welche Feinchemikalien aus der Zelle exportieren, kann es möglich sein, die Menge der produzierten Feinchemikalie, die im extrazellulären Medium zugegen ist, zu steigern, wodurch Ernte und Reinigung oder bei Pflanzen eine effizientere Verteilung erleichtert werden. Zur effizienten Überproduktion einer oder mehrerer Feinchemikalien sind dagegen erhöhte Mengen an Cofaktoren, Vorläufermolekülen und Zwischenverbindungen für die geeigneten Biosynthesewege erforderlich. Durch Vergrößern der Anzahl und/oder der Aktivität von Transporterproteinen, die am Import von Nährstoffen, wie Kohlenstoffquellen (d.h. Zuckern), Stickstoffquellen (d.h. Aminosäuren, Ammoniumsalzen), Phosphat und Schwefel, beteiligt sind, kann man die Produktion einer Feinchemikalie aufgrund der Beseitigung aller Einschränkungen des Nährstoffangebots beim Biosyntheseprozeß verbessern. Fettsäuren, wie PUFAs, und Lipide, die PUFAs enthalten, sind selbst wünschenswerte Feinchemikalien; durch Optimieren der Aktivität oder Erhöhen der Anzahl einer oder mehrerer erfindungsgemäßer PSEs, die an der Biosynthese dieser Verbindungen beteiligt sind, oder durch Stören der Aktivität einer oder mehrerer PSEs, die am Abbau dieser Verbindungen beteiligt sind, kann man somit die Ausbeute, Produktion und/oder Effizienz der Produktion von Fettsäure- und Lipidmoleküle in Ciliaten, Algen, Pflanzen, Pilzen, Hefen oder anderen Mikroorganismen steigern.

Die Manipulation eines oder mehrerer erfindungsgemäßer PSE-Gene kann ebenfalls zu PSEs mit veränderten Aktivitäten führen, welche die Produktion einer oder mehrerer gewünschter Feinchemikalien aus Algen, Pflanzen, Ciliaten oder Pilzen indirekt beeinflussen. Die normalen biochemischen Stoffwechselprozesse führen z.B. zur Produktion einer Vielzahl an Abfallprodukten (z.B. Wasserstoffperoxid und andere reaktive Sauerstoffspezies), die diese Stoffwechselprozesse aktiv stören können [z.B. nitriert Peroxynitrit bekanntlich Tyrosin-Seitenketten, wodurch einige Enzyme mit Tyrosin im aktiven Zentrum inaktiviert werden, Groves, J.T. (1999) Curr. Opin. Chem. Biol. 3(2);226-235]. Diese Abfallprodukte werden zwar üblicherweise ausgeschieden, aber die zur fermentativen Produktion im großen Maßstab verwendeten Zellen werden für die Überproduktion einer oder mehrerer Feinchemikalien optimiert und können somit mehr Abfallprodukte produzieren als für eine Wildtypzelle üblich. Durch Optimieren der Aktivität einer oder mehrerer erfindungsgemäßer PSEs, die am Export von Abfallmolekülen beteiligt sind, kann man die Lebensfähigkeit der Zelle verbessern und eine effiziente Stoffwechselaktivität aufrechterhalten. Auch das Vorliegen hoher intrazellulärer Mengen der gewünschten Feinchemikalie kann tatsächlich für die Zelle toxisch sein, so daß man durch Steigern der Fähigkeit der Zelle zur Sekretion dieser Verbindungen die Lebensfähigkeit der Zelle verbessern kann.

Die erfindungsgemäßen PSEs können ferner so manipuliert sein, daß die relativen Mengen verschiedener Lipid- und Fettsäuremoleküle verändert werden. Dies kann eine entscheidende Auswirkung auf die Lipidzusammensetzung der Zellmembran haben. Da jeder Lipidtyp unterschiedliche physikalische Eigenschaften hat, kann die Veränderung der Lipidzusammensetzung einer Membran die Membranfluidität signifikant verändern. Änderungen der Membranfluidität können den Transport von Molekülen über die Membran beeinflussen, was, wie vorstehend erläutert, den Export von Abfallprodukten oder der produzierten Feinchemikalie oder den Import notwendiger Nährstoffe modifizieren kann. Diese Änderungen der Membranfluidität können auch die Integrität der Zelle entscheidend beeinflussen; Zellen mit vergleichsweise schwächeren Membranen sind anfälliger gegenüber abiotischen und biotischen Streßbedingungen, welche die Zelle beschädigen oder abtöten können. Durch Manipulieren von PSEs, die an der Produktion von Fettsäuren und Lipiden für den Membranaufbau beteiligt sind, so daß die resultierende Membran eine Membranzusammensetzung hat, die für die in den Kulturen, die zur Produktion von Feinchemikalien verwendet werden, herrschenden Umweltbedingungen empfänglicher sind, sollte ein größerer Anteil der Zellen überleben und sich vermehren. Größere Mengen an produzierenden Zellen sollten sich in größeren Ausbeuten, höherer Produktion oder Effizienz der Produktion der Feinchemikalie aus der Kultur manifestieren.

Die vorstehend genannten Mutagenesestrategien für PSEs, die zu erhöhten Ausbeuten einer Feinchemikalie führen sollen, sollen nicht beschränkend sein; Variationen dieser Strategien sind dem Fachmann leicht ersichtlich. Unter Verwendung dieser Mechanismen und mit Hilfe der hier offenbarten Mechanismen können die erfindungsgemäßen Nukleinsäure- und Proteinmoleküle zur Erzeugung von Algen, Ciliaten, Pflanzen, Tieren, Pilzen oder anderen Mikroorganismen, wie C. glutamicum, verwendet werden, die mutierte PSE-Nukleinsäure- und Proteinmoleküle exprimieren, so daß die Ausbeute, Produktion und/oder Effizienz der Produktion einer gewünschten Verbindung verbessert wird. Diese gewünschte Verbindung kann ein beliebiges natürliches Produkt von Algen, Ciliaten, Pflanzen, Tieren, Pilzen oder Bakterien sein, welches die Endprodukte von Biosynthesewegen und Zwischenprodukte natürlich vorkommender Stoffwechselwege umfaßt, sowie Moleküle, die im Stoffwechsel dieser Zellen nicht natürlich vorkommen, die jedoch von den erfindungsgemäßen Zellen produziert werden.

Eine weitere erfindungsgemäße Ausführungsform ist ein Verfahren zur Produktion von PUFAs, wobei das Verfahren das Züchten eines nicht-menschliche Organismus, der eine erfindungsgemäße Nukleinsäure, ein erfindungsgemäßes Genkonstrukt oder einen erfindungsgemäßen Vektor enthält, welche für ein Polypeptid codieren, das C₁₆-, C₁₈- oder C₂₀-Fettsäuren mit mindestens zwei Doppelbindungen im Fettsäuremolekül um mindestens zwei Kohlenstoffatome unter Bedingungen, unter denen PUFAs in dem Organismus produziert werden, verlängert, umfaßt. Durch dieses Verfahren hergestellte PUFAs lassen sich durch Ernten der Organismen entweder aus der Kultur, in der sie wachsen, oder von dem Feld, Aufbrechen und/oder Extrahieren des geernteten Materials mit einem organischen Lösungsmittel isolieren. Aus diesem Lösungsmittel kann das Öl, das Lipide, Phospholipide, Sphingolipide, Glycolipide, Triacylglycerine und/oder freie Fettsäuren mit höherem Gehalt an PUFAs enthält, isoliert werden. Durch basische oder saure Hydrolyse der Lipide, Phospholipide, Sphingolipide, Glycolipide, Triacylglycerine können die freien Fettsäuren mit höherem Gehalt an PUFAs isoliert werden. Ein höherer Gehalt an PUFAs bedeutet mindestens 5 %, vorzugsweise 10 %, besonders bevorzugt 20 %, ganz besonders bevorzugt 40 % mehr PUFAs als der ursprüngliche Organismus, der keine zusätzliche Nukleinsäure, die für die erfindungsgemäße Elongase codiert, besitzt.

Vorzugsweise sind die durch dieses Verfahren produzierten PUFAs C₁₈-, C₂₀- oder C₂₂-Fettsäuremoleküle mit mindestens zwei Doppelbindungen im Fettsäuremolekül, vorzugsweise drei, vier, fünf oder sechs Doppelbindungen, besonders bevorzugt drei oder fünf Doppelbindungen. Diese C₁₈-, C₂₀- oder C₂₂-Fettsäuremoleküle lassen sich aus dem Organismus in Form eines Öls, Lipids oder einer freien Fettsäure isolieren. Geeignete Organismen sind beispielsweise die vorstehend erwähnten. Bevorzugte Organismen sind Mikroorganismen, Tiere oder Pflanzen, besonders bevorzugt Pflanzen oder Algen, ganz besonders bevorzugt transgene Pflanzen.

Die Erfindung beschreibt Öle, Lipide oder Fettsäuren oder Fraktionen davon, die durch das oben beschriebene Verfahren hergestellt worden sind, besonders bevorzugt Öl, Lipid oder eine Fettsäurezusammensetzung, die PUFAs umfassen und von transgenen Pflanzen herrühren.

Die Erfindung beschreibt Öle, Lipide oder Fettsäuren, die durch das erfindungsgemäße Verfahren hergestellt wurden. Die Erfindung beschreibt auch Öl-, Lipid- oder Fettsäurezusammensetzungen, die PUFAs hergestellt nach dem erfindungsgemäßen Verfahren enthalten und von transgenen Pflanzen stammen, welche eine erfindungsgemäße Nukleinsäure, ein Genkonstrukt oder Vektor enthalten.

Die Erfindung beschreibt die Verwendung des Öls, Lipids oder der Fettsäurezusammensetzung in Futtermitteln, Nahrungsmitteln, Kosmetika oder Pharmazeutika.

Die vorliegende Erfindung beschreibt ein Kit, umfassend die erfindungsgemäße Nukleinsäure, das erfindungsgemäße Genkonstrukt, die erfindungsgemäße Aminosäuresequenz, das erfindungsgemäße Antisense-Nukleinsäuremolekül, den erfindungsgemäßen Antikörper und/oder Zusammensetzung, einen Antagonisten oder Agonisten, der nach dem beschriebenen Verfahren hergestellt wurde, und/oder Öle, Lipide und/oder Fettsäuren oder eine Fraktion davon. Ebenso kann das Kit die erfindungsgemäßen Wirtszellen, Organismen, Pflanzen oder Teile davon, erntbare Teile der erfindungsgemäßen Pflanzen oder Vermehrungsmaterial oder aber auch den beschriebenen Antagonisten oder Agonisten umfassen. Die Komponenten des Kits der vorliegenden Erfindung können in geeigneten Containern, beispielsweise mit oder in Puffern oder anderen Lösungen verpackt sein. Ein oder mehr der genannten Komponenten können in ein und demselben Container verpackt sein. Zusätzlich oder alternativ können ein oder mehr der genannten Komponenten z.B. auf einer festen Oberfläche adsorbiert sein, z.B. Nitrozellulosefilter, Glasplatten, Chips, Nylonmembranen oder Mikrotiterplatten. Das Kit kann für jede der hierin beschriebenen Methoden und Ausführungsformen verwendet werden, z.B. für die Produktion von Wirtszellen, transgenen Pflanzen, zur Detektion von homologen Sequenzen, zur Identifikation von Antagonisten oder Agonisten usw. Weiterhin kann das Kit Anleitungen für die Verwendung des Kits für eine der genanntenAnwendungen enthalten.

Diese Erfindung wird durch die nachstehenden Beispiele weiter veranschaulicht, die nicht als beschränkend aufgefaßt werden sollten.

### Beispiele

### Beispiel 1: Allgemeine Verfahren

### a) Allgemeine Klonierungsverfahren:

Klonierungsverfahren, wie beispielsweise Restriktionsspaltungen, Agarosegelelektrophorese, Reinigung von DNA-Fragmenten, Transfer von Nukleinsäuren auf Nitrocellulose- und Nylonmembranen, Verbindung von DNA-Fragmenten,Transformation von Escherichia coli- und Hefe-Zellen, Anzucht von Bakterien und Sequenzanalyse rekombinanter DNA, wurden durchgeführt wie beschrieben in Sambrook et al. [(1989), Cold Spring Harbor Laboratory Press: ISBN 0-87969-309-6] oder Kaiser, Michaelis und Mitchell [(1994), "Methods in Yeast Genetics", Cold Spring Harbor Laboratory Press: ISBN 0-87969-451-3]. Die Transformation und Anzucht von Algen, wie Chlorella oder Phaeodactylum werden durchgeführt wie beschrieben von E1-Sheekh [(1999), Biologia Plantarum 42:209-216] oder und mit Hilfe der hier offenbarten Mechanismen können die erfindungsgemäßen Nukleinsäure- und Proteinmoleküle zur Erzeugung von Algen, Ciliaten, Pflanzen, Tieren, Pilzen oder anderen Mikroorganismen, wie C. glutamicum, verwendet werden, die mutierte PSE-Nukleinsäure- und Proteinmoleküle exprimieren, so daß die Ausbeute, Produktion und/oder Effizienz der Produktion einer gewünschten Verbindung verbessert wird. Diese gewünschte Verbindung kann ein beliebiges natürliches Produkt von Algen, Ciliaten, Pflanzen, Tieren, Pilzen oder Bakterien sein, welches die Endprodukte von Biosynthesewegen und Zwischenprodukte natürlich vorkommender Stoffwechselwege umfaßt, sowie Moleküle, die im Stoffwechsel dieser Zellen nicht natürlich vorkommen, die jedoch von den erfindungsgemäßen Zellen produziert werden.

Eine weitere erfindungsgemäße Ausführungsform ist ein Verfahren zur Produktion von PUFAs, wobei das Verfahren das Züchten eines Organismus, der eine erfindungsgemäße Nukleinsäure, ein erfindungsgemäßes Genkonstrukt oder einen erfindungsgemäßen Vektor enthält, welche für ein Polypeptid codieren, das C₁₆-, C₁₈- oder C₂₀-Fettsäuren mit mindestens zwei Doppelbindungen im Fettsäuremolekül um mindestens zwei Kohlenstoffatome unter Bedingungen, unter denen PUFAs in dem Organismus produziert werden, verlängert, umfaßt. Durch dieses Verfahren hergestellte PUFAs lassen sich durch Ernten der Organismen entweder aus der Kultur, in der sie wachsen, oder von dem Feld, Aufbrechen und/oder Extrahieren des geernteten Materials mit einem organischen Lösungsmittel isolieren. Aus diesem Lösungsmittel kann das Öl, das Lipide, Phospholipide, Sphingolipide, Glycolipide, Triacylglycerine und/oder freie Fettsäuren mit höherem Gehalt an PUFAs enthält, isoliert werden. Durch basische oder saure Hydrolyse der Lipide, Phospholipide, Sphingolipide, Glycolipide, Triacylglycerine können die freien Fettsäuren mit höherem Gehalt an PUFAs isoliert werden. Ein höherer Gehalt an PUFAs bedeutet mindestens 5 %, vorzugsweise 10 %, besonders bevorzugt 20 %, ganz besonders bevorzugt 40 % mehr PUFAs als der ursprüngliche Organismus, der keine zusätzliche Nukleinsäure, die für die erfindungsgemäße Elongase codiert, besitzt.

Vorzugsweise sind die durch dieses Verfahren produzierten PUFAs C₁₈-, C₂₀- oder C₂₂-Fettsäuremoleküle mit mindestens zwei Doppelbindungen im Fettsäuremolekül, vorzugsweise drei, vier, fünf oder sechs Doppelbindungen, besonders bevorzugt drei oder fünf Doppelbindungen. Diese C₁₈-, C₂₀- oder C₂₂-Fettsäuremoleküle lassen sich aus dem Organismus in Form eines Öls, Lipids oder einer freien Fettsäure isolieren. Geeignete Organismen sind beispielsweise die vorstehend erwähnten. Bevorzugte Organismen sind Mikroorganismen, Tiere oder Pflanzen, besonders bevorzugt Pflanzen oder Algen, ganz besonders bevorzugt transgene Pflanzen.

Eine erfindungsgemäße Ausführungsform sind Öle, Lipide oder Fettsäuren oder Fraktionen davon, die durch das oben beschriebene Verfahren hergestellt worden sind, besonders bevorzugt Öl, Lipid oder eine Fettsäurezusammensetzung, die PUFAs umfassen und von transgenen Pflanzen herrühren.

Eine Ausführungsform der Erfindung sind Öle, Lipide oder Fettsäuren, die durch das erfindungsgemäße Verfahren hergestellt wurden. Weitere Ausführungsformen der Erfindung sind Öl-, Lipid- oder Fettsäurezusammensetzungen, die PUFAs hergestellt nach dem erfindungsgemäßen Verfahren enthalten und von transgenen Pflanzen stammen, welche eine erfindungsgemäße Nukleinsäure, ein Genkonstrukt oder Vektor enthalten.

Eine weitere erfindungsgemäße Ausführungsform ist die Verwendung des Öls, Lipids oder der Fettsäurezusammensetzung in Futtermitteln, Nahrungsmitteln, Kosmetika oder Pharmazeutika.

In einer weiteren Ausführungsform betrifft die vorliegende Erfindung ein Kit, umfassend die erfindungsgemäße Nukleinsäure, das erfindungsgemäße Genkonstrukt, die erfindungsgemäße Aminosäuresequenz, das erfindungsgemäße Antisense-Nukleinsäuremolekül, den erfindungsgemäßen Antikörper und/oder Zusammensetzung, einen Antagonisten oder Agonisten, der nach dem erfindungsgemäßen Verfahren hergestellt wurde, und/oder erfindungsgemäße Öle, Lipide und/oder Fettsäuren oder eine Fraktion davon. Ebenso kann das Kit die erfindungsgemäßen Wirtszellen, Organismen, Pflanzen oder Teile davon, erntbare Teile der erfindungsgemäßen Pflanzen oder Vermehrungsmaterial oder aber auch den erfindungsgemäßen Antagonisten oder Agonisten umfassen. Die Komponenten des Kits der vorliegenden Erfindung können in geeigneten Containern, beispielsweise mit oder in Puffern oder anderen Lösungen verpackt sein. Ein oder mehr der genannten Komponenten können in ein und demselben Container verpackt sein. Zusätzlich oder alternativ können ein oder mehr der genannten Komponenten z.B. auf einer festen Oberfläche adsorbiert sein, z.B. Nitrozellulosefilter, Glasplatten, Chips, Nylonmembranen oder Mikrotiterplatten. Das Kit kann für jede der hierin beschriebenen Methoden und Ausführungsformen verwendet werden, z.B. für die Produktion von Wirtszellen, transgenen Pflanzen, zur Detektion von homologen Sequenzen, zur Identifikation von Antagonisten oder Agonisten usw. Weiterhin kann das Kit Anleitungen für die Verwendung des Kits für eine der genannten Anwendungen enthalten.

Diese Erfindung wird durch die nachstehenden Beispiele weiter veranschaulicht, die nicht als beschränkend aufgefaßt werden sollten. Der Inhalt sämtlicher in dieser Patentanmeldung zitierten Literaturstellen, Patentanmeldungen, Patente und veröffentlichten Patentanmeldungen ist hier durch Bezugnahme aufgenommen.

### Beispiele

### Beispiel 1: Allgemeine Verfahren

### a) Allgemeine Klonierungsverfahren:

Klonierungsverfahren, wie beispielsweise Restriktionsspaltungen, Agarosegelelektrophorese, Reinigung von DNA-Fragmenten, Transfer von Nukleinsäuren auf Nitrocellulose- und Nylonmembranen, Verbindung von DNA-Fragmenten, Transformation von Escherichia coli- und Hefe-Zellen, Anzucht von Bakterien und Sequenzanalyse rekombinanter DNA, wurden durchgeführt wie beschrieben in Sambrook et a1. [(1989), Cold Spring Harbor Laboratory Press: ISBN 0-87969-309-6] oder Kaiser, Michaelis und Mitchell [(1994), "Methods in Yeast Genetics", Cold Spring Harbor Laboratory Press: ISBN 0-87969-451-3]. Die Transformation und Anzucht von Algen, wie Chlorella oder Phaeodactylum werden durchgeführt wie beschrieben von E1-Sheekh [(1999), Biologia Plantarum 42:209-216]oder Apt et al. [(1996) Molecular and General Genetics 252 (5):872-9].

### b) Chemikalien

Die verwendeten Chemikalien wurden, wenn im Text nicht anders angegeben, in p. A.-Qualität von den Firmen Fluka (Neu-Ulm), Merck (Darmstadt), Roth (Karlsruhe), Serva (Heidelberg) und Sigma (Deisenhofen) bezogen. Lösungen wurden unter Verwendung von reinem pyrogenfreiem Wasser, im nachstehenden Text als H₂O bezeichnet, aus einer Milli-Q-Wassersystem-Wasserreinigungsanlage (Millipore, Eschborn) hergestellt. Restriktionsendonukleasen, DNA-modifizierende Enzyme und molekularbiologische Kits wurden bezogen von den Firmen AGS (Heidelberg), Amersham (Braunschweig), Biometra (Göttingen), Boehringer (Mannheim), Genomed (Bad Oeynhausen), New England Biolabs (Schwalbach/Taunus), Novagen (Madison, Wisconsin, USA), Perkin-Elmer (Weiterstadt), Pharmacia (Freiburg), Qiagen (Hilden) und Stratagene (Amsterdam, Niederlande). Wenn nicht anders angegeben, wurden sie nach den Anweisungen des Herstellers verwendet.

### c) Zellmaterial

Für diese Untersuchungen wurde eine Thraustochytrium-Stamm verwendet, der über die American Type Culture Collection (ATCC-Sammlung) mit der Stammnummer ATCC26185 (Thraustochytrium) verfügbar ist, bzw im Fall von Crypthecodinium vom Provasoli-Guillard National Center for Culture of Marine Phytoplancton ((CCMP) West Boothbay Harbour, ME, USA) mit der Stammkultur-Nr. CCMP316 zugänglich ist. Die Algen wurden bei 25 Grad Celsius im Dunkeln kultiviert in Glasröhren, die von unten mit Luft begast wurden. Die Anzucht von Thraustochytrium erfolgte alternativ wie detailliert beschrieben bei Singh & Ward (1997, Advances in Microbiology, 45, 271-311).

Als Kulturmedium für Crypthecodinium wurde das f/2 Kulturmedium mit 10% organischen Medium nach Guillard, R.R.L. verwendet [1975; Culture of phytoplankton for feeding marine invertebrates. In:Smith, W.L. and Chanley, M.H. (Eds.) Culture of marine Invertebrate animals, NY Plenum Press, pp. 29-60.]: Es enthält
995,5 ml Seewasser (artifiziell)
1 ml NaNO₃ (75 g/l), 1 ml NaH₂PO₄ (5 g/l), 1 ml Trace metal solution, 1 ml Tris/Cl pH 8.0, 0.5 ml f /2 vitamin solution
Trace metal solution: Na₂EDTA (4,36 g/l), FeCl₃ (3,15 g/l), Primary Trace metals: CuSO₄ (10 g/l), ZnSO₄ (22 g/l), CoCl₂ (10 g/l), MnCl₂ (18 g/l), NaMoO₄ (6,3 g/l)
f/2 vitamin solution: Biotin: 10 mg/l, Thiamin 200 mg/l, Vit B12 0,1 mg/l
org-Medium: Na-Acetat (1 g/l), Glucose (6 g/l), Na-Succinat (3 g/l), Bacto-Trypton (4 g/l), Hefe-Extrakt (2 g/l)

### d) Moosmaterial (= Pflanzenmaterial)

Für diese Untersuchung wurden Pflanzen der Art Physcomitrella patens (Hedw.) B.S.G. aus der Sammlung der Abteilung für Genetische Untersuchungen der Universität Hamburg verwendet. Sie stammen von dem Stamm 16/14, der von H.L.K. Whitehouse in Gransden Wood, Huntingdonshire (England) gesammelt und von Engel (1968, Am J Bot 55, 438-446) aus einer Spore subkultiviert wurde. Die Proliferation der Pflanzen erfolgte mittels Sporen und mittels Regeneration der Gametophyten. Das Protonema entwickelte sich aus der haploiden Spore zu chloroplastenreichem Chloronema und chloroplastenarmem Caulonema, woraus sich nach etwa 12 Tagen Knospen bildeten. Diese wuchsen zu Gametophoren mit Antheridien und Archegonien. Nach der Befruchtung entstand der diploide Sporophyt mit kurzer-Seta und Sporenkapsel, in der die Meiosporen reifen.

### e) Pflanzenanzucht

Die Anzucht erfolgte in einer klimatisierten Kammer bei einer Lufttemperatur von 25°C und einer Lichtintensität von 55 Mikromol-1m-2 (Weißlicht; Phillips TL 65W/25 Fluoreszenzröhre) und einem Licht-Dunkel-Wechsel von 16/8 Stunden. Das Moos wurde entweder in Flüssigkultur unter Verwendung von Knop-Medium nach Reski und Abel (1985, Planta 165, 354-358) modifiziert oder auf festem Knop-Medium unter Verwendung von 1 % Oxoidagar (Unipath, Basingstoke, England) angezogen.

Die zur RNA- und DNA-Isolation verwendeten Protonemata wurden in belüfteten Flüssigkulturen gezüchtet. Die Protonemata wurden alle 9 Tage zerkleinert und in frisches Kulturmedium überführt.

### f) Kultivierung von Phytophthora infestans

Zunächst wurde eine cDNA Bank von Phytophthora infestans erstellt. Hierzu kann der Stamm ATCC 48886 von der American Type Culture Collection Rockville, USA erhalten werden. Abweichend vom Anzuchtprotokoll beschrieben für den Stamm ATCC 48886 wurden Phytophthora Sporen mit kaltem bidestillierten Wasser gewaschen und für 6 .Stunden im Kühlschrank zur Sporulation gebracht. Danach wurde das Material in Erbsenmedium überführt. Hierzu wurden 150 g Tiefkühlerbsen (Iglu, erhältlich von lokalen Supermärkten) in 1 Liter Wasser für 20 Minuten steril autoklaviert. Das Material wird in 100 ml Kolben bei Raumtemperatur auf einem Kreisschüttler angezogen (200 rpm). Nach zwei Tagen wurden je 2 Kolben und in den darauffolgenden 4 Tagen ebenfalls je 2 Kolben abfiltriert und in Flüssigstickstoff zermörsert.

### Beispiel 2: Isolierung von Gesamt-DNA aus Pflanzen und Mikroorganismen wie Thraustochytrium und Crypthecodinium für Hybridisierungsexperimente

Die Einzelheiten der Isolation von Gesamt-DNA betreffen die Aufarbeitung von Pflanzenmaterial mit einem Frischgewicht von einem Gramm.

CTAB-Puffer: 2 % (Gew./Vol.) N-Acetyl-N,N,N-trimethylammoniumbromid (CTAB); 100 mM Tris-HCl, pH 8,0; 1,4 M NaCl; 20 mM EDTA. N-Laurylsarkosin-Puffer: 10 % (Gew./Vol.) N-Laurylsarkosin; 100 mM Tris-HCl, pH 8,0; 20 mM EDTA.

Das Pflanzenmaterial oder Crypthecodinium- oder Thraustochytrium-Zellmaterial wurde unter flüssigem Stickstoff in einem Mörser verrieben, so daß ein feines Pulver erhalten wurde, und in 2-ml-Eppendorfgefäße überführt. Das gefrorene Pflanzenmaterial wurde dann mit einer Schicht von 1 ml Zersetzungspuffer (1 ml CTAB-Puffer, 100 ml N-Laurylsarkosin-Puffer, 20 ml β-Mercaptoethanol und 10 ml Proteinase K-Lösung, 10 mg/ml) überschichtet und eine Stunde unter kontinuierlichem Schütteln bei 60°C inkubiert. Das erhaltene Homogenat wurde in zwei Eppendorfgefäße (2 ml) aufgeteilt und zweimal durch Schütteln mit dem gleichen Volumen Chloroform/Isoamylalkohol (24:1) extrahiert. Zur Phasentrennung wurde eine Zentrifugation bei 8000 x g und RT (= Raumtemperatur = ~ 23°C) jeweils 15 min lang durchgeführt. Die DNA wurde dann 30 min unter Verwendung von eiskaltem Isopropanol bei -70°C gefällt. Die gefällte DNA wurde bei 10000 g 30 min bei 4°C sedimentiert und in 180 ml TE-Puffer (Sambrook et al., 1989, Cold Spring Harbor Laboratory Press: ISBN 0-87969-309-6) resuspendiert. Zur weiteren Reinigung wurde die DNA mit NaCl (1,2 M Endkonzentration) behandelt und erneut 30 min unter Verwendung des zweifachen Volumens an absolutem Ethanol bei -70°C gefällt. Nach einem Waschschritt mit 70 % Ethanol wurde die DNA getrocknet und anschließend in 50 ml H₂O + RNAse (50 mg/ml Endkonzentration) aufgenommen. Die DNA wurde über Nacht bei 4°C gelöst und die RNAse-Spaltung wurde anschließend 1 Std. bei 37°C durchgeführt. Die Aufbewahrung der DNA erfolgte bei 4°C.

### Beispiel 3: Isolierung von Gesamt-RNA und poly (A)⁺-RNA aus Pflanzen und Mikroorganismen (Crypthecodinium und Thraustochytrium)

Die Isolierung von Gesamt-RNA aus Pflanzen wie Lein und Raps etc. erfolgt nach einer bei Logemann et al beschriebenen Methode (1987, Anal. Biochem. 163, 21) isoliert. Aus Moos kann die Gesamt-RNA Protonema-Gewebe nach dem GTC-Verfahren (Reski et al., 1994, Mol. Gen. Genet., 244:352-359) gewonnen werden.

RNA Isolation aus Crypthecodinium und Thraustochytrium:

Tiefgefrorene Algenproben (- 70°C) werden in einem eiskaltem Mörser unter Flüssigstickstoff zu feinem Pulver zerreiben. 2 Volumen Homogenisationsmedium (12,024 g Sorbitol, 40,0 ml 1M Tris-HCl, pH 9 (0,2 M); 12,0 ml 5 M NaCl (0,3 M), 8,0 ml 250 mM EDTA, 761,0 mg EGTA, 40,0 ml 10% SDS werden auf 200 ml mit H₂O aufgefüllt und der pH auf 8,5 eingestellt) und 4 Volumen Phenol mit 0,2% Mercaptoethanol werden bei 40-50°C unter gutem Mischen zu gefrorenem Zellpulver gegeben. Danach werden 2 Volumen Chloroform hinzufügen und für 15 min kräftig gerührt. Es wird 10 min bei 10000g zentrifugiert und die wässrige Phase mit Phenol/Chloroform (2Vol/2Vol) und abschließend mit Chloroform extrahiert.

Das erhaltene Volumen der wässrigen Phase wird mit 1/20 Vol 4 M Na-Acetat (pH 6) und 1 Vol Isopropanol (eiskalt) versetzet und die Nukleinsäuren bei -20°C ÜN gefällt. Es wird 30 min bei 10000 g zentrifugiert und der Überstand abgesogen. Es folgt ein Waschschritt mit 70 % EtOH und erneute Zentrifugation. Das Sediment wird in Tris-Borat-Puffer (80 mM Tris-Borat-Puffer, 10 mM EDTA, pH 7,0). Dann wird der Überstand mit 1/3 Vol 8 M LiCl versetzt, gemischt 30 min bei 4°C inkubiert. Nach erneutem zentrifugieren wird das Sediment mit 70 % Ethanol gewaschen, zentrifugiert und das Sediment in RNAse-freiem Wasser gelöst.

Die Isolierung von poly(A)⁺-RNA erfolgt unter Verwendung von Dyna Beads^{®} (Dynal, Oslo, Finnland) nach den Anweisungen im Protokoll des Herstellers.

Nach der Bestimmung der RNA- oder poly(A)⁺-RNA-Konzentration wird die RNA durch Zugabe von 1/10 Volumina 3 M Natriumacetat, pH 4,6, und 2 Volumina Ethanol gefällt und bei -70°C aufbewahrt.

Für die Analyse werden jeweils 20 µg RNA in einem Formaldehydhaltigen 1,5%igen Agarosegel aufgetrennt und auf Nylon Membranen (Hybond, Amersham) überführt. Der Nachweis spezifischer Transkripte wird wie bei Amasino beschrieben durchgeführt ((1986) Anal. Biochem. 152, 304)).

Isolierung von Gesamt-RNA und poly-(A)+ RNA aus Phytophthora infestans:

Gesamt-RNA wurde mit dem RNeasy Plant Total RNA Kit (Qiagen, Hilden) und dem darin enthaltenen RLT-Puffer nach Angaben des Herstellers gewonnen. Aus der gewonnenen Gesamt-RNA wurde die poly-(A)+ RNA mit dem Poly Attract mRNA Isolation System III der Firma Promega (Heidelberg) nach Angaben des Herstellers isoliert.

### Beispiel 4: Konstruktion der cDNA-Bank

Zur Konstruktion der cDNA-Bank aus Physcomitrella, Crypthecodinium bzw. Thraustochytrium wurde die Erststrangsynthese unter Verwendung von Reverser Transkriptase aus Maus-Leukämie-Virus (Roche, Mannheim, Deutschland) und Oligo-d(T)-Primern, die Zweitstrangsynthese durch Inkubation mit DNA-Polymerase I, Klenow-Enzym und RNAse H-Spaltung bei 12°C (2 Std.), 16°C (1 Std.) und 22°C (1 STd.) erzielt. Die Reaktion wurde durch Inkubation bei 65°C (10 min) gestoppt und anschließend auf Eis überführt. Doppelsträngige DNA-Moleküle wurde mit T4-DNA-Polymerase (Roche, Mannheim) bei 37°C (30 min) mit glatten Enden versehen. Die Nukleotide wurden durch Phenol/Chloroform-Extraktion und Sephadex-G50-Zentrifugiersäulen entfernt. EcoRI/XhoI-Adapter (Pharmacia, Freiburg, Deutschland) wurden mittels T4-DNA-Ligase (Roche, 12°C, über Nacht) an die cDNA-Enden ligiert, mit XhoI nachgeschnitten und durch Inkubation mit Polynukleotidkinase (Roche, 37°C, 30 min) phosphoryliert. Dieses Gemisch wurde der Trennung auf einem Low-Melting-Agarose-Gel unterworfen. DNA-Moleküle über 300 Basenpaaren wurden aus dem Gel eluiert, Phenolextrahiert, auf Elutip-D-Säulen (Schleicher und Schüll, Dassel, Deutschland) konzentriert und an Vektorarme ligiert und in lambda-ZAPII-Phagen oder lambda-ZAP-Express-Phagen unter Verwendung des Gigapack Gold-Kits (Stratagene, Amsterdam, Niederlande) verpackt, wobei Material des Herstellers verwendet und seine Anweisungen befolgt wurden.

Die Konstruktion einer cDNA Bank für Phytophthora infestans erfolgte wie oben beschrieben.

### Beispiel 5: DNA-Sequenzierung und Computeranalyse

cDNA-Banken, wie im Beispiel 4 beschrieben, wurden zur DNA-Sequenzierung nach Standardverfahren, insbesondere durch das Kettenterminationsverfahren unter Verwendung des ABI PRISM Big Dye Terminator Cycle Sequencing Ready Reaction-Kit (Perkin-Elmer, Weiterstadt, Deutschland), verwendet. Die Sequenzierung zufälliger, vereinzelter Klone wurde anschließend an die präparative Plasmidgewinnung aus cDNA-Banken über in vivo-Massenexcision und Retransformation von DH10B auf Agarplatten durchgeführt (Einzelheiten zu Material und Protokoll von Stratagene, Amsterdam, Niederlande). Plasmid-DNA wurde aus über Nacht gezüchteten E. coli-Kulturen, die in Luria-Brühe mit Ampicillin (siehe Sambrook et al. (1989) (Cold Spring Harbor Laboratory Press: ISBN 0-87969-309-6)) gezüchtet worden waren, an einem Qiagen-DNA-Präparations-Roboter (Qiagen, Hilden) nach den Protokollen des Herstellers präpariert. Sequenzierprimer mit den folgenden Nukleotidsequenzen wurden verwendet:
5'-CAGGAAACAGCTATGACC-3'
5'-CTAAAGGGAACAAAAGCTG-3'
5'-TGTAAAACGACGGCCAGT-3'

Die Sequenzen wurden unter Verwendung des Standard-Softwarepakets EST-MAX, das kommerziell von Bio-Max (München, Deutschland) geliefert wird, prozessiert und annotiert. Durch Nutzung von Vergleichsalgorithmen und unter Verwendung einer Suchsequenz wurde mithilfe des BLAST-Programms nach homologen Genen gesucht (Altschul et al. (1997) "Gapped BLAST and PSI-BLAST: a new generation of protein database search programs", Nucleic Acids Res. 25:3389-3402.). Eine Sequenz aus Crypthecodinium und Thraustochytrium mit Homologien zur Suchsequenz der Mooselongase aus Physcomitrella patens wurden eingehender charakterisiert.

### Beispiel 6a: Identifizierung des Tc_PSE1 und Tc_PSE2-Gens (Tc = Thraustochytrium) sowie des Cc_PSE1 und Cc_PSE2-Gens (Cc = Crypthecodinium cohnii) durch Vergleich mit dem Pp_PSE1-Gen aus Physcomitrella patens.

Die Vollängensequenz der erfindungsgemäßen Mooselongase Pp_PSE1 (Bezeichnung siehe auch Tabelle 2) wurde für die Sequenzvergleiche im TBLASTN Suchalgorythmus eingesetzt:

Die vollständige Nukleotidsequenz der Mooselongase Pp_PSE1 cDNA besteht aus etwa 1200 bp. Sie enthält einen offenen Leserahmen von 873 bp, der 290 Aminosäure mit einer berechneten Molekülmasse von 33,4 Da codiert. Die Proteinsequenz besitzt nur 38,5 % Identität und 48,3 % Ähnlichkeit mit einem, z.B. PSE1-, Genprodukt von Saccharomyces cerevisiae, das zur Elongation von Fettsäuren mit mittlerer Kettenlänge in der Hefe erforderlich ist (Toke & Martin, 1996, Isolation and characterization of a gene affecting fatty acid elongation in Saccharomyces cerevisiae. Journal of Biological Chemistry 271, 18413-18422).

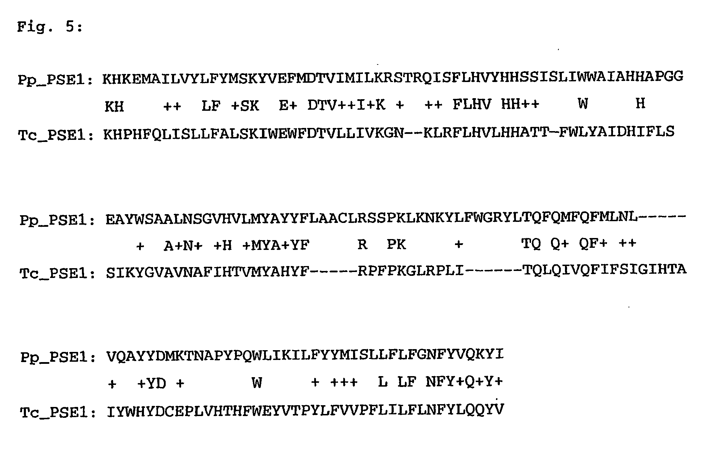

Die EST-Sequenz CC001042041R sowie TC002034029R und TC002014093R wurden zunächst aufgrund schwacher Homologien mit der Elongase aus Physcomitrella patens (siehe Tabelle 2), dem PSE1 Gen unter anderen Kandidatengenen als Zielgen in Betracht gezogen. In Figur 5 ist das Ergebnis des Vergleiches der Pp_PSE1 Peptidsequenz mit der gefundenen Sequenz dargestellt. Sie ist Teil der erfindungsgemäßen Nukleinsäure aus Seq ID NO:3. (Genname: Tc_PSE1, eigene Datenbank Nr. der Erfinder TC002034029R). Buchstaben zeigen identische Aminosäuren an, während das Pluszeichen eine chemisch ähnliche Aminosäure bedeutet. Die Identitäten bzw. Homologien aller erfindungsgemäß gefundener Sequenzen gehen aus Tabelle 3 zusammenfassend hervor.

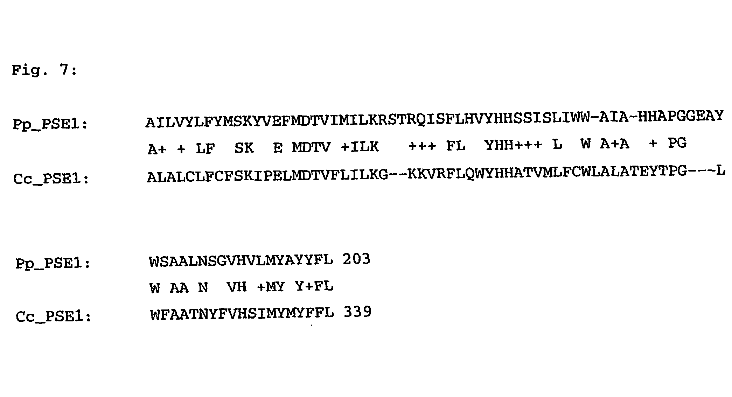

Die Sequenzierung des vollständigen cDNA Fragmentes aus Klon TC002034029R ergab eine 693 Basenpaare lange Sequenz beginnend mit erster Base im offenen Leseraster. Die Sequenz codiert für ein Polypeptid von 195 Aminosäuren dargestellt in Seq ID NO:4 mit einem Stopcodon in Basenpaarposition übersetzt aus SEQ ID NO:3 in Basenpaarposition 586-588. Der Klon TC002014093R beinhaltet ein nahezu vollständiges Elongase-Polypeptid, wie aus dem Sequenzvergleich in Figur 7 zu ersehen ist. Striche bedeuten identische Aminosäuren während Doppelpunkte und Einzelpunkte chemisch austauschbare, d.h. chemisch äquivalente Aminosäuren darstellen. Der Vergleich wurde mit der BLOSUM62 Austauschmatrix für Aminsoäuren nach Henikoff & Henikoff durchgeführt ((1992) Amino acid substitution matrices from protein blocks. Proc. Natl. Acad. Sci. USA 89: 10915-10919).Verwendete Parameter: Gap Weight: 8; Average Match: 2.912, Length Weight: 2, Average Mismatch: -2.003.

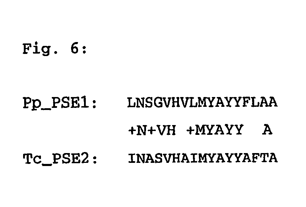

Weiterhin wurde durch den Sequenzvergleich ein zweiter est identifiziert. Dargestellt in Figur 6 ist der Vergleich der Pp_PSE1 Peptidsequenz mit der gefundenen Sequenz. Wenngleich sich die Homologie unter gewählten Parametern auf wenige Aminosäuren beschränkt, so bezieht sich dies auf einen stark konservierten Bereich der PUFA-spezifischen Elongasen. Es wurde daher die Sequenz des vollständigen klonierten Fragmentes ermittelt.

Die Sequenzierung des vollständigen cDNA Fragmentes aus Klon TC002014093R ergab eine 955 Basenpaare lange Sequenz beginnend mit erster Base im offenen Leseraster. Diese ist erfindungsgemäß mit SEQ ID NR: 5 angegeben. Die Sequenz codiert für ein Polypeptid von 297 Aminosäuren mit einem Stopcodon in Basenpaarposition 892-894 erfindungsgemäß dargestellt in SEQ ID NO: 6.

Mithilfe der Sequenz PpPSE1 wurde der est CC001042041R aus Crypthecodinium cohnii codierend für das Gen Cc_PSE1 identifiziert. Der isolierte est CC001042041R, erfindungsgemäß dargestellt als SEQ ID NR: 7 ist 708 Basenpaaren lang mit einem offenen Leseraster ab erster Base von 642 Basenpaaren codierend für 214 Aminosäuren und mit einem Stopcodon in Position 643-645. Die Aminosäuresequenz bis zum Stopcodon ist erfindungsgemäß dargestellt in SEQ ID NR:8.

Neben der Ähnlichkeit mit dem PSE1-Genprodukt kann auch die Ähnlichkeit zur Elongase aus *Saccharomyces cerevisiae (sce elo 1P)* herangezogen werden, die zur Elongation von Fettsäuren mit mittlerer Kettenlänge in der Hefe erforderlich ist (Toke & Martin, 1996, Isolation and characterization of a gene affecting fatty acid elongation in Saccharomyces cerevisiae. Journal of Biological Chemistry 271, 18413-18422). In Tabelle 3 sind die Identitäten und Homologien erfindungsgemäßer Elongasen untereinander und mit den Elongasen aus Physcomitrella patens und aus Hefe dargestellt. Die Angaben wurden mithilfe des Programms GAP als Teilprogramm folgender Software erhalten: Wisconsin Package Version 10.0 (Genetics Computer Group (GCG), Madison, Wisc., USA).

**Tabelle 3:**

| Identity/ homology | TC_PSE1 | TC_PSE2 | Pp_PSE1 | Sce elo 1P |
|---|---|---|---|---|
| Cc_PSE1 | 47,1% / 40,2% | 50,6% / 43,5% | 38,5% / 29,4% | 45,1% / 33,5% |
| Tc_PSE1 | 100 / 100 | n.d. | 43,2% / 32,7% | 41,9% / 29,9% |
| Tc_PSE2 | 41,7% / 29,5% | 100 / 100 | 39,2% / 30,0 | 35,4% / 27,8% |

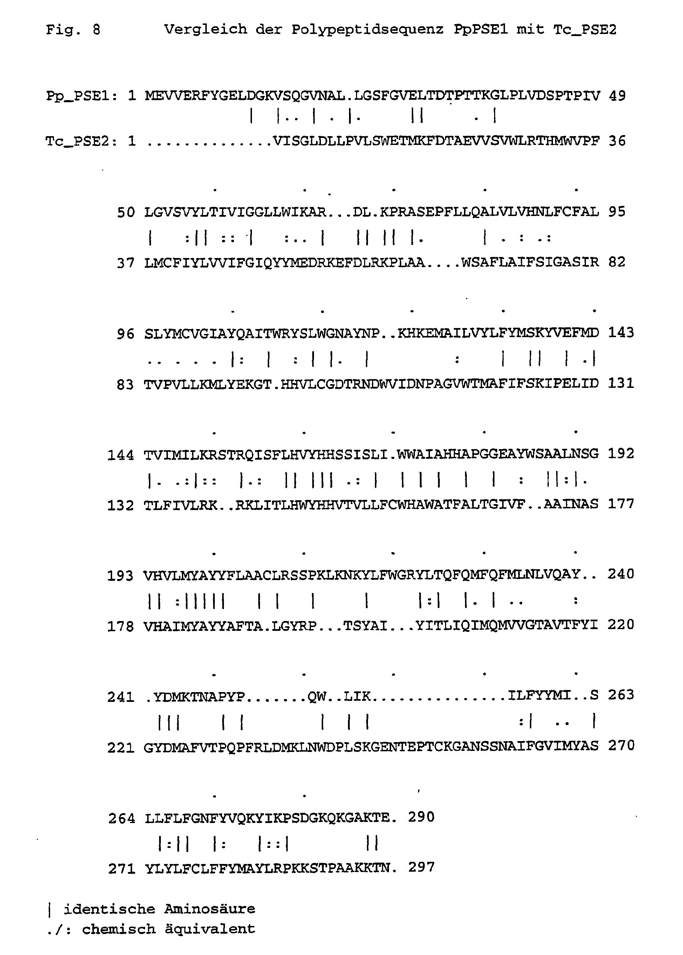

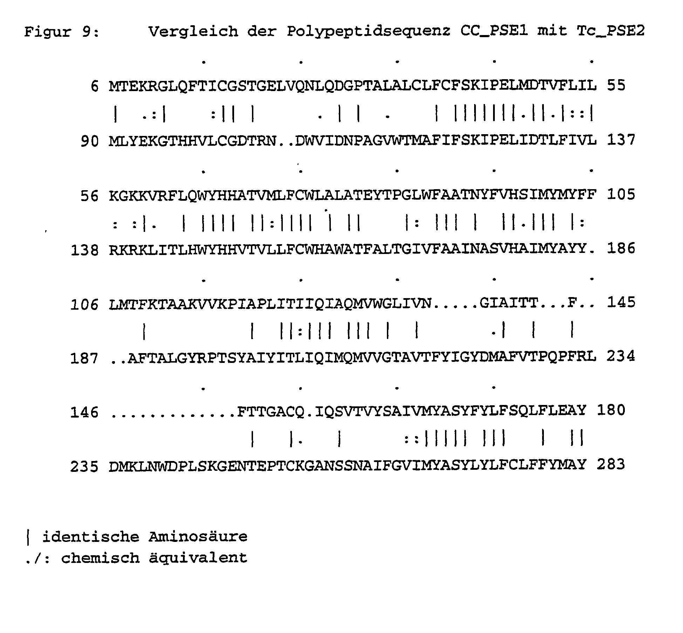

Insbesondere lassen sich aus Figuren 5 bis 10 die folgenden Sequenzmotive als Bereiche hoher Homologie und entsprechende daraus abgeleitete Consensussequenzen ableiten, die durch Rückübersetzung der Aminsoäuren in Drei-Basenpaar-Codons zu Oligonukleotiden führen, die zur Isolation neuer Elongasen mittels Polymerasekettenreaktion genutzt werden können. Dies sind insbesondere die in Figur 10 dargestellten Sequenzmotive. Aus diesen Motiven lassen sich Oligonukleotide ableiten und in Kombination mit zwei Oligonukleotiden in PCR-Experimenten zur Isolation weiterer Elongasefragmente einsetzen. Dabei wird zweckmäßigerweise ein Oligonukleotid passend für den konventionell definierten 5'-3'Strang und ein zweites mit einem strangabwärts für den 3'-5'Strang passenden Oligonukleotid konstruiert und synthetisiert. Dabei ergibt sich eine definierbare Anzahl an Primerkombinationen, die durch Permutation der möglichen Varianten begrenzt ist.

Dabei können auch Oligo-dT Primer und Varianten davon genutzt werden, z.B. indem die letzte Base Spezifität für einen Pool von Transkripten zuläßt wie z.B: Oligo dT (12-20) X, wobei X ein G, C oder T sein kann. Auch läßt sich eine zweite Base Oligo dT (12-20) XY nutzen, wobei X ein G, C oder A sein kann, während das Y ein A, G, C, oder T sein kann.

Aus oben definierten Sequenzen lassen sich 17- bis 20mere Oligonukleotide ableiten, die unter Variation der Primerkombinationen und Versuchsparameter wie Temperaturprogramm, Mg-Ionenkonzentration etc. zur Isolation von Genfragmenten genutzt werden können. Die so erhaltenen Fragmente können in geeignete Vektoren kloniert werden und die Sequenz erhaltener Klone zur Identifizierung neuer Elongasen nach gängigen verfahren ermittelt werden.

Beispiel 6b: Isolation des cDNA Klons aus Pythophtora infestans

Der cDNA Klon mit der Bezeichnung PI001002014R aus Phytophthora infestans wurde aus zufällig sequenzierten cDNAs aufgrund von Homologien zur PUFA Elongase aus dem Moos Physcomitrella patens identifiziert (ATCCC 48886). Der Klon enthält das in Abbildung 8 dargestellte Konsensus Motiv MYxYYF, wobei abweichend von bisher identifizierten PUFA Elongasen ein Threoninrest als variabe Aminosäure x gefunden wurde. Diese weitere Variation kann zur Ableitung von PCR Primern genutzt werden.

### Beispiel 7: Identifikation von Genen mittels Hybridisierung (TC002034029R-11 iGenTc-PCE1)

Gensequenzen lassen sich zur Identifikation homologer oder heterologer Gene aus cDNA- oder genomischen Banken verwenden.

Homologe Gene (d.h. Volllängen-cDNA-Klone, die homolog sind, oder Homologen) lassen sich über Nukleinsäurehybridisierung unter Verwendung von beispielsweise cDNA-Banken isolieren: Insbesondere zur Isolation von funktionell aktiven Voll-Längengenen der in SEQ ID NO:1, SEQ ID NO:3, SEQ ID NO:5, SEQ ID NO:7, SEQ ID NO:9 und SEQ ID NO:11 kann die Methode genutzt werden. Je nach der Häufigkeit des Gens von Interesse werden 100000 bis zu 1000000 rekombinante Bakteriophagen plattiert und auf eine Nylonmembran überführt. Nach der Denaturierung mit Alkali wird die DNA auf der Membran z.B. durch UV-Vernetzung immobilisiert. Die Hybridisierung erfolgt bei hoch-stringenten Bedingungen. In wäßriger Lösung werden die Hybridisierung und die Waschschritte bei einer Ionenstärke von 1 M NaCl und einer Temperatur von 68°C durchgeführt. Hybridisierungssonden wurden z.B. durch Markierung mittels radioaktiver (³²P-) Nicktranskription (High Prime, Roche, Mannheim, Deutschland) hergestellt. Die Signale werden mittels Autoradiographie nachgewiesen.

Partiell homologe oder heterologe Gene, die verwandt, aber nicht identisch sind, lassen sich analog zum oben beschriebenen Verfahren unter Verwendung niedrig-stringenter Hybridisierungs- und Waschbedingungen identifizieren. Für die wäßrige Hybridisierung wurde die Ionenstärke gewöhnlich bei 1 M NaCl gehalten, wobei die Temperatur nach und nach von 68 auf 42°C gesenkt wurde.

Die Isolation von Gensequenzen, die nur zu einer einzelnen Domäne von beispielsweise 10 bis 20 Aminosäuren Homologien aufweisen, läßt sich unter Verwendung synthetischer, radioaktiv markierter Oligonukleotidsonden durchführen. Radioaktiv markierte Oligonukleotide werden mittels Phosphorylierung des 5'-Endes zweier komplementärer Oligonukleotide mit T4-Polynukleotidkinase hergestellt. Die komplementären Oligonukleotide werden aneinander hybridisiert und ligiert, so daß Konkatemere entstehen. Die doppelsträngigen Konkatemere werden beispielsweise durch Nicktranskription radioaktiv markiert. Die Hybridisierung erfolgt gewöhnlich bei niedrig-stringenten Bedingungen unter Verwendung hoher Oligonukleotidkonzentrationen.

### Oligonukleotid-Hybridisierungslösung:

6 x SSC
0,01 M Natriumphosphat
1 mM EDTA (pH 8)
0,5 % SDS
100 mikrog/ml denaturierte Lachssperma-DNA
0,1 % fettarme Trockenmilch

Während der Hybridisierung wird die Temperatur schrittweise auf 5 bis 10°C unter die berechnete Oligonukleotid-Tm oder bis auf Raumtemperatur (bedeutet RT = ~ 23°C in allen Experimenten, wenn nicht anders angegeben) gesenkt, gefolgt von Waschschritten und Autoradiographie. Das Waschen wird mit extrem niedriger Stringenz durchgeführt, zum Beispiel 3 Waschschritte unter Verwendung von 4 x SSC. Weitere Einzelheiten sind wie von Sambrook, J., et al. (1989), "Molecular Cloning: A Laboratory Manual", Cold Spring Harbor Laboratory Press, oder Ausubel, F.M., et al. (1994) "Current Protocols in Molecular Biology", John Wiley & Sons, beschrieben.

Der Klon TC002034029R-11 mit dem Gennamen Tc_PCE1_1 ist eine Vollängensequenz einer Elongase aus Thraustochytrium und somit länger als Klon TC002034029R aus Seq. ID No. 3 und Seq. ID No. 4. Die Isolation des Klones erfolgte über Hybridisierungsverfahren wie oben (Beispiel 7) beschrieben. Es handelt sich um eine 1054 Basenpaare lange DNA Sequenz codierend für 271 Aminosäuren mit einem Startcodon in Basenpaarposition 43-45 und einem Stopcodon in Basenpaarposition 856-858.

### Beispiel 8: Identifikation von Zielgenen durch Sichtung von Expressionsbanken mit Antikörpern

Es werden cDNA-Sequenzen zur Herstellung von rekombinantem Protein zum Beispiel in E. coli verwendet (z.B. Qiagen QIAexpress pQE-System). Die rekombinanten Proteine werden dann gewöhnlich über Ni-NTA-Affinitätschromatographie (Qiagen) affinitätsgereinigt. Die rekombinanten Proteine werden dann zur Herstellung spezifischer Antikörper beispielsweise unter Verwendung von Standardtechniken zur Immunisierung von Kaninchen verwendet. Die Antikörper werden dann unter Verwendung einer Ni-NTA-Säule, die mit rekombinantem Antigen vorgesättigt wird, affinitätsgereinigt, wie von Gu et al., (1994) BioTechniques 17:257-262 beschrieben. Der Antikörper kann dann zur Durchmusterung von Expressions-cDNA-Banken mittels immunologischem Sichtung verwendet werden (Sambrook, J., et al. (1989), "Molecular Cloning: A Laboratory Manual", Cold Spring Harbor Laboratory Press, oder Ausubel, F.M., et al. (1994) "Current Protocols in Molecular Biology", John Wiley & Sons).

### Beispiel 9: Plasmide für die Pflanzentransformation

Zur Pflanzentransformation können binäre Vektoren, wie pBinAR verwendet werden (Höfgen und Willmitzer, Plant Science 66 (1990) 221-230). Die Konstruktion der binären Vektoren kann durch Ligation der cDNA in Sense- oder Antisense-Orientierung in T-DNA erfolgen. 5' der cDNA aktiviert ein Pflanzenpromotor die Transkription der cDNA. Eine Polyadenylierungssequenz befindet sich 3' von der cDNA.

Die gewebespezifische Expression läßt sich unter Verwendung eines gewebespezifischen Promotors erzielen. Beispielsweise kann die samenspezifische Expression erreicht werden, indem der Napin- oder der LeB4- oder der USP-Promotor 5' der cDNA einkloniert wird. Auch jedes andere samenspezifische Promotorelement kann verwendet werden. Zur konstitutiven Expression in der ganzen Pflanzen läßt sich der CaMV-35S-Promotor verwenden.

Das exprimierte Protein kann unter Verwendung eines Signalpeptids, beispielsweise für Plastiden, Mitochondrien oder das Endoplasmatische Retikulum, in ein zelluläres Kompartiment dirigiert werden (Kermode, Crit. Rev. Plant Sci. 15, 4 (1996) 285-423). Das Signalpeptid wird 5' im Leseraster mit der cDNA einkloniert, um die subzelluläre Lokalisierung des Fusionsprotein zu erreichen.

### Beispiel 10: Transformation von Agrobacterium

Die Agrobacterium-vermittelte Pflanzentransformation kann zum Beispiel,unter Verwendung des GV3101- (pMP90-) (Koncz und Schell, Mol. Gen. Genet. 204 (1986) 383-396) oder LBA4404- (Clontech) Agrobacterium tumefaciens-Stamms durchgeführt werden. Die Transformation kann durch Standard-Transformationstechniken durchgeführt werden (Deblaere et al., Nucl. Acids. Tes. 13 (1984), 4777-4788).

### Beispiel 11: Pflanzentransformation

Die Agrobacterium-vermittelte Pflanzentransformation kann unter Verwendung von Standard-Transformations- und Regenerationstechniken durchgeführt werden (Gelvin, Stanton B., Schilperoort, Robert A., Plant Molecular Biology Manual, 2. Aufl., Dordrecht: Kluwer Academic Publ., 1995, in Sect., Ringbuc Zentrale Signatur: BT11-P ISBN 0-7923-2731-4; Glick, Bernard R., Thompson, John E., Methods in Plant Molecular Biology and Biotechnology, Boca Raton: CRC Press, 1993, 360 S., ISBN 0-8493-5164-2).

Beispielsweise kann Raps mittels Kotyledonen- oder Hypokotyltransformation transformiert werden (Moloney et al., Plant Cell Report 8 (1989) 238-242; De Block et al., Plant Physiol. 91 (1989) 694-701). Die Verwendung von Antibiotika für die Agrobacterium- und Pflanzenselektion hängt von dem für die Transformation verwendeten binären Vektor und Agrobacterium-Stamm ab. Die Rapsselektion wird gewöhnlich unter Verwendung von Kanamycin als selektierbarem Pflanzenmarker durchgeführt.

Der Agrobacterium-vermittelte Gentransfer in Lein (Linum usitatissimum) läßt sich unter Verwendung von beispielsweise einer von Mlynarova et al. (1994) Plant Cell Report 13:282-285 beschriebenen Technik durchführen. Die Transformation von Soja kann unter Verwendung von beispielsweise einer in EP-A-0 0424 047 (Pioneer Hi-Bred International) oder in EP-A-0 0397 687, US 5,376,543, US 5,169,770 (University Toledo) beschriebenen Technik durchgeführt werden.

Die Pflanzentransformation unter Verwendung von Teilchenbeschuß, Polyethylenglycol-vermittelter DNA-Aufnahme oder über die Siliziumcarbonatfaser-Technik ist beispielsweise beschrieben von Freeling und Walbot "The maize handbook" (1993) ISBN 3-540-97826-7, Springer Verlag New York).

### Beispiel 12: Plasmide für die Pflanzentransformation

Zur Pflanzentransformation können binäre Vektoren, wie pBinAR verwendet werden (Höfgen und Willmitzer, Plant Science 66 (1990) 221-230). Die Konstruktion der binären Vektoren kann durch Ligation der cDNA in Sense- oder Antisense-Orientierung in T-DNA erfolgen. 5' der cDNA aktiviert ein Pflanzenpromotor die Transkription der cDNA. Eine Polyadenylierungssequenz befindet sich 3' von der cDNA.
Die gewebespezifische Expression läßt sich unter Verwendung eines gewebespezifischen Promotors erzielen. Beispielsweise kann die samenspezifische Expression erreicht werden, indem der Napin- oder der LeB4- oder der USP-Promotor 5' der cDNA einkloniert wird. Auch jedes andere samenspezifische Promotorelement kann verwendet werden. Zur konstitutiven Expression in der ganzen Pflanzen läßt sich der CaMV-35S-Promotor verwenden. Insbesondere lassen sich Gene codierend für Elongasen und Desaturasen durch Konstruktion mehrerer Expressionskassetten hintereinander in einen binären Vektor klonieren, um den Stoffwechselweg in der Pflanzen nachzubilden.

Innerhalb einer Expressionskassette kann das zu exprimierende Protein unter Verwendung eines Signalpeptids, beispielsweise für Plastiden, Mitochondrien oder das Endoplasmatische Retikulum, in ein zelluläres Kompartiment dirigiert werden (Kermode, Crit. Rev. Plant Sci. 15, 4 (1996) 285-423). Das Signalpeptid wird 5' im Leseraster mit der cDNA einkloniert, um die subzelluläre Lokalisierung des Fusionsprotein zu erreichen.

### Beispiel 13: In vivo-Mutagenese

Die in vivo-Mutagenese von Mikroorganismen kann mittels Passage der Plasmid- (oder einer anderen Vektor-) DNA durch E. coli oder andere Mikroorganismen (z.B. Bacillus spp. oder Hefen, wie Saccharomyces cerevisiae), bei denen die Fähigkeiten, die Unversehrtheit ihrer genetischen Information aufrechtzuerhalten, gestört ist, erfolgen. Übliche Mutator-Stämme haben Mutationen in den Genen für das DNA-Reparatursystem (z.B. mutHLS, mutD, mutT usw.; als Literaturstelle siehe Rupp, W.D. (1996) DNA repair mechanisms, in: Escherichia coli and Salmonella, S. 2277-2294, ASM: Washington). Diese Stämme sind dem Fachmann bekannt. Die Verwendung dieser Stämme ist beispielsweise in Greener, A., und Callahan, M. (1994) Strategies 7:32-34, erläutert. Der Transfer mutierter DNA-Moleküle in Pflanzen erfolgt vorzugsweise nach Selektion und Test der Mikrooganismen. Transgene Pflanzen werden nach verschiedenen Beispielen im Beispielteil dieses Dokumentes erzeugt.

### Beispiel 14: Untersuchung der Expression eines rekombinanten Genproduktes in einem transformierten Organismus

Die Aktivität eines rekombinanten Genproduktes im transformierten Wirtsorganismus wurde auf der Transkriptions- und/oder der Translationsebene gemessen.

Ein geeignetes Verfahren zur Bestimmung der Menge an Transkription des Gens (ein Hinweis auf die Menge an RNA, die für die Translation des Genproduktes zur Verfügung steht) ist die Durchführung eines Northern-Blots wie unten ausgeführt (als Bezugsstelle siehe Ausubel et al. (1988) Current Protocols in Molecular Biology, Wiley: New York, oder den oben erwähnten Beispielteil), wobei ein Primer, der so gestaltet ist, daß er an das Gen von Interesse bindet, mit einer nachweisbaren Markierung (gewöhnlich radioaktiv oder chemilumineszent) markiert wird, so daß, wenn die Gesamt-RNA einer Kultur des Organismus extrahiert, auf einem Gel aufgetrennt, auf eine stabile Matrix transferiert und mit dieser Sonde inkubiert wird, die Bindung und das Ausmaß der Bindung der Sonde das Vorliegen und auch die Menge der mRNA für dieses Gen anzeigt. Diese Information zeigt den Grad der Transkription des transformierten Gens an. Zelluläre Gesamt-RNA kann aus Zellen, Geweben oder Organen mit mehreren Verfahren, die alle im Fachgebiet bekannt sind, wie zum Beispiel das von Bormann, E.R., et al. (1992) Mol. Microbiol. 6:317-326 beschriebene, präpariert werden.

### Northern-Hybridisierung:

Für die RNA-Hybridisierung wurden 20 ∝g Gesamt-RNA oder 1 ∝g poly(A)⁺-RNA mittels Gelelektrophorese in Agarosegelen mit einer Stärke von 1,25 % unter Verwendung von Formaldehyd, wie beschrieben in Amasino (1986, Anal. Biochem. 152, 304) aufgetrennt, mittels Kapillaranziehung unter Verwendung von 10 x SSC auf positiv geladene Nylonmembranen (Hybond N+, Amersham, Braunschweig) übertragen, mittels UV-Licht immobilisiert und 3 Stunden bei 68°C unter Verwendung von Hybridisierungspuffer (10 % Dextransulfat Gew./Vol., 1 M NaCl, 1 % SDS, 100 mg Heringssperma-DNA) vorhybridisiert. Die Markierung der DNA-Sonde mit dem Highprime DNA labeling-Kit (Roche, Mannheim, Deutschland) erfolgte während der Vorhybridisierung unter Verwendung von alpha-³²P-dCTP (Amersham, Braunschweig, Deutschland). Die Hybridisierung wurde nach Zugabe der markierten DNA-Sonde im gleichen Puffer bei 68°C über Nacht durchgeführt. Die Waschschritte wurden zweimal für 15 min unter Verwendung von 2 X SSC und zweimal für 30 min unter Verwendung von 1 X SSC, 1 % SDS, bei 68°C durchgeführt. Die Exposition der verschlossenen Filter wurde bei -70°C für einen Zeitraum von 1 bis 14 T durchgeführt.

Zur Untersuchung des Vorliegens oder der relativen Menge an von dieser mRNA translatiertem Protein können Standardtechniken, wie ein Western-Blot, eingesetzt werden (siehe beispielsweise Ausubel et al. (1988) Current Protocols in Molecular Biology, Wiley: New York). Bei diesem Verfahren werden die zellulären Gesamt-Proteine extrahiert, mittels Gelelektrophorese aufgetrennt, auf eine Matrix, wie Nitrozellulose, übertragen und mit einer Sonde, wie einem Antikörper, der spezifisch an das gewünschte Protein bindet, inkubiert. Diese Sonde ist gewöhnlich mit einer chemilumineszenten oder kolorimetrischen Markierung versehen, die sich leicht nachweisen läßt. Das Vorliegen und die Menge der beobachteten Markierung zeigt das Vorliegen und die Menge des gewünschten, in der Zelle vorliegenden mutierten Proteins an.

### Beispiel 15: Analyse der Auswirkung der rekombinanten Proteine auf die Produktion des gewünschten Produktes

Die Auswirkung der genetischen Modifikation in Pflanzen, Pilzen, Algen, Ciliaten oder auf die Produktion einer gewünschten Verbindung (wie einer Fettsäure) kann bestimmt werden, indem die modifizierten Mikroorganismen oder die modifizierte Pflanze unter geeigneten Bedingungen (wie den vorstehend beschriebenen) gezüchtet werden und das Medium und/oder die zellulären Komponenten auf die erhöhte Produktion des gewünschten Produktes (d.h. von Lipiden oder einer Fettsäure) untersucht wird. Diese Analysetechniken sind dem Fachmann bekannt und umfassen Spektroskopie, Dünnschichtchromatographie, Färbeverfahren verschiedener Art, enzymatische und mikrobiologische Verfahren sowie analytische Chromatographie, wie Hochleistungs-Flüssigkeitschromatographie (siehe beispielsweise Ullman, Encyclopedia of Industrial Chemistry, Bd. A2, S. 89-90 und S. 443-613, VCH: Weinheim (1985); Fallon, A., et al., (1987) "Applications of HPLC in Biochemistry" in: Laboratory Techniques in Biochemistry and Molecular Biology, Bd. 17; Rehm et al. (1993) Biotechnology, Bd. 3, Kapitel III: "Product recovery and purification", S. 469-714, VCH: Weinheim; Belter, P.A., et al. (1988) Bioseparations: downstream processing for Biotechnology, John Wiley and Sons; Kennedy, J.F., und Cabral, J.M.S. (1992) Recovery processes for biological Materials, John Wiley and Sons; Shaeiwitz, J.A., und Henry, J.D. (1988) Biochemical Separations, in: Ullmann's Encyclopedia of Industrial Chemistry, Bd. B3; Kapitel 11, S. 1-27, VCH: Weinheim; und Dechow, F.J. (1989) Separation and purification techniques in biotechnology, Noyes Publications).

Neben den oben erwähnten Verfahren werden Pflanzenlipide aus Pflanzenmaterial wie von Cahoon et al. (1999) Proc. Natl. Acad. Sci. USA 96 (22):12935-12940, und Browse et al. (1986) Analytic Biochemistry 152:141-145, beschrieben extrahiert. Die qualitative und quantitative Lipid- oder Fettsäureanalyse ist beschrieben bei Christie, William W., Advances in Lipid Methodology, Ayr/Scotland: Oily Press (Oily Press Lipid Library; 2); Christie, William W., Gas Chromatography and Lipids. A Practical Guide - Ayr, Scotland: Oily Press, 1989, Repr. 1992, IX, 307 S. (Oily Press Lipid Library; 1); "Progress in Lipid Research, Oxford: Pergamon Press, 1 (1952) - 16 (1977) u.d.T.: Progress in the Chemistry of Fats and Other Lipids CODEN.

Zusätzlich zur Messung des Endproduktes der Fermentation ist es auch möglich, andere Komponenten der Stoffwechselwege zu analysieren, die zur Produktion der gewünschten Verbindung verwendet werden, wie Zwischen- und Nebenprodukte, um die Gesamteffizienz der Produktion der Verbindung zu bestimmen. Die Analyseverfahren umfassen Messungen der Nährstoffmengen im Medium (z.B. Zucker, Kohlenwasserstoffe, Stickstoffquellen, Phosphat und andere Ionen), Messungen der Biomassezusammensetzung und des Wachstums, Analyse der Produktion üblicher Metabolite von Biosynthesewegen und Messungen von Gasen, die während der Fermentation erzeugt werden. Standardverfahren für diese Messungen sind in Applied Microbial Physiology; A Practical Approach, P.M. Rhodes und P.F. Stanbury, Hrsgb., IRL Press, S. 131-163 und 165-192 (ISBN: 0199635773) und darin angegebenen Literaturstellen beschrieben.

Ein Beispiel ist die Analyse von Fettsäuren (Abkürzungen: FAME, Fettsäuremethylester; GC-MS, Gas-Flüssigkeitschromatographie-Massenspektrometrie; TAG, Triacylglycerin; TLC, Dünnschichtchromatographie).

Der unzweideutige Nachweis für das Vorliegen von Fettsäureprodukten kann mittels Analyse rekombinanter Organismen nach Standard-Analyseverfahren erhalten werden: GC, GC-MS oder TLC, wie verschiedentlich beschrieben von Christie und den Literaturstellen darin (1997, in: Advances on Lipid Methodology, Vierte Auf1.: Christie, Oily Press, Dundee, 119-169; 1998, Gaschromatographie-Massenspektrometrie-Verfahren, Lipide 33:343-353).

Das zu analysierende Material kann durch Ultraschallbehandlung, Mahlen in der Glasmühle, flüssigen Stickstoff und Mahlen oder über andere anwendbare Verfahren aufgebrochen werden. Das Material muss nach dem Aufbrechen zentrifugiert werden. Das Sediment wird in Aqua dest. resuspendiert, 10 min bei 100°C erhitzt, auf Eis abgekühlt und erneut zentrifugiert, gefolgt von Extraktion in 0,5 M Schwefelsäure in Methanol mit 2 % Dimethoxypropan für 1 Std. bei 90°C, was zu hydrolysierten Öl- und Lipidverbindungen führt, die transmethylierte Lipide ergeben. Diese Fettsäuremethylester werden in Petrolether extrahiert und schließlich einer GC-Analyse unter Verwendung einer Kapillarsäule (Chrompack, WCOT Fused Silica, CP-Wax-52 CB, 25 mikrom, 0,32 mm) bei einem Temperaturgradienten zwischen 170°C und 240°C für 20 min und 5 min bei 240°C unterworfen. Die Identität der erhaltenen Fettsäuremethylester muss unter Verwendung von Standards, die aus kommerziellen Quellen erhältlich sind (d.h. Sigma), definiert werden.

Bei Fettsäuren, für die keine Standards verfügbar sind, muss die Identität über Derivatisierung und anschließende GC-MS-Analyse gezeigt werden. Beispielsweise muss die Lokalisierung von Fettsäuren mit Dreifachbindung über GC-MS nach Derivatisierung mit 4,4-Dimethoxyoxazolin-Derivaten (Christie, 1998, siehe oben) gezeigt werden.

### Beispiel 16: Expressionskonstrukte in heterologen mikrobiellen Systemen

### Stämme, Wachstumsbedingungen und Plasmide

Der Escherichia coli-Stamm XL1 Blue MRF' kan (Stratagene) wird zur Subklonierung der neuen Elongasen wie PpPSE1 aus Physcomitrella patens verwendet. Für die funktionelle Expression dieses Gens verwenden wir den Saccharomyces cerevisiae-Stamm INVSc 1 (Invitrogen Co.). E. coli wird in Luria-Bertini-Brühe (LB, Duchefa, Haarlem, Niederlande) bei 37°C kultiviert. Wenn nötig, wird Ampicillin (100 mg/Liter) zugegeben, und 1,5 % Agar (Gew./Vol.) wird für feste LB-Medien hinzugefügt. S. cerevisiae wird bei 30°C entweder in YPG-Medium oder in komplettem Minimalmedium ohne Uracil (CMdum; siehe in: Ausubel, F.M., Brent, R., Kingston, R.E., Moore, D.D., Seidman, J.G., Smith, J.A., Struhl, K., Albright, L.B., Coen, D.M., und Varki, A. (1995) Current Protocols in Molecular Biology, John Wiley & Sons, New York) mit entweder 2 % (Gew./Vol.) Raffinose oder Glucose kultiviert. Für feste Medien werden 2 % (Gew./Vol.) Bacto^{™}-Agar (Difco) hinzugefügt. Die zur Klonierung und Expression verwendeten Plasmide sind pUC18 (Pharmacia) und pYES2 (Invitrogen Co.).

### Beispiel 17: Klonierung und Expression PUFA-spezifischer Elongasen aus Physcomitrella, Crypthecodinium und Thraustochytrium.

Die Vollängengene erfindungsgemäßer Sequenzen können wie in Beispiel 7 beschrieben isoliert und wie nachfogend ausgeführt weiter bearbeitet werden. Ausführungsweise werden konkrete Expressionsbeispiele dargestellt.

### A) Elongation von Fettsäuren durch die Mooselongase Pp_PSE1:

Für die Expression in Hefe wurde der P. patens-cDNA-Klon PpPSE1 (früherer Datenbanksequenzname: 08_ck19_b07, neuer Name: pp001019019f), der das PUFA-spezifische Elongase- (PSE1-) Gen codiert, zuerst so modifiziert, daß eine BamHI-Restriktionsstelle und die Hefe-Konsensussequenz für hoch-effiziente Translation (Kozak, M. (1986) Point mutations define a sequence flanking the AUG initiator codon that modulates translation by eukaryotic ribosomes, Cell 44, 283-292) neben dem Startcodon und eine BamHI-Restriktionsstelle, die das Stopcodon flankierte, erhalten wurden. Zur Amplifikation des offenen Leserahmens wurde ein Primerpaar, das komplementär zu dessen 5'- und 3'-Enden war, synthetisiert.

Das Gen erfindungsgemäßer Sequenz dargestellt in SEQ ID NO:1 wurde mittels Polymerase-Kettenreaktion in pYES kloniert und das Plasmid pYPp_PSE1 erhalten:

Folgende Oligonukleotide wurden für das PCR-Experiment eingesetzt:

| | |
|---|---|
| Ppex6: | ggatccacataatggaggtcgtggagagattc |
| Ppex6r: | ggatcctcactcagttttagctccttttgc |

Die PCR-Reaktion wurde mit Plasmid-DNA des Klones PP001019019F als Matrize in einem Thermocycler (Biometra) mit der Pfu-DNA-(Stratagene) Polymerase und dem folgenden Temperaturprogramm durchgeführt: 3 min bei 96°C, gefolgt von 25 Zyklen mit 30 s bei 96°C, 30 s bei 55°C und 1 min bei 72°C, 1 Zyklus mit 10 min bei 72°C.

Die korrekte Größe des amplifizierten DNA-Fragments wurde mittels Agarose-TBE-Gelelektrophorese bestätigt. Die amplifizierte DNA wurde aus dem Gel mit dem QIAquick-Gelextraktionskit (QIAGEN) extrahiert und unter Verwendung des Sure Clone Ligation Kit (Pharmacia)zunächst in pUC18 kloniert. Das so klonierte Fragment wurde mit BamHI geschnitten, in pYES ligiert, wobei pYPp_PSE1 erhalten wurde. Die Fragmentorientierung wurde mittels HindIII überprüft. Nach der Transformation von E. coli XL1 Blue MRF' kan wurde eine DNA-Minipräparation (Riggs, M.G., & McLachlan, A. (1986) A simplified screening procedure for large numbers of plasmid mini-preparation. BioTechniques 4, 310-313) von Transformanten durchgeführt, und positive Klone mittels BamHI-Restriktionsanalyse identifiziert. Die Sequenz des klonierten PCR-Produktes wurde mittels Resequenzierung unter Verwendung des ABI PRISM Big Dye Terminator Cycle Sequencing Ready Reaction Kit (Perkin-Elmer, Weiterstadt) bestätigt.

Ein Klon wurde für die DNA-Maxipräparation mit dem Nucleobond^{®} AX 500 Plasmid-DNA-Extraktionskit (Macherey-Nagel, Düringen) angezogen.

Saccharomyces INVSc1 wurde mit pYPp_PSE1 bzw. mit pYES2 als Kontrolle mittels eines modifizierten PEG/Lithiumacetat-Protokolls transformiert (Ausubel et al., 1995). Nach der Selektion auf CMdum-Agarplatten mit 2 % Glucose wurden Transformanten und eine pYES2-Transformante zur weiteren Anzucht und funktionellen Expression wie bereits ausgeführt ausgewählt und mit verschiedenen Fettsäuren im Medium gefüttert.
i) Lipidmuster von Hefen, die mit dem pYES Plasmid ohne Fragmentinsertion transformiert sind oder das Pp-PSE1 Gen exprimieren (Angaben in mol%) nach Fütterung mit mit 250 mikrom Hexadekatriensäure (16:3^{Δ7c,10c,13c}).

**Tabelle 4:**

| PYES2 | PYES2 | pYPp_PSE1 | PYPp_PSE1 |
|---|---|---|---|
| 16:0 | 11,8% | 16:0 | 11,1% |
| 16:1 | 28,7% | 16:1 | 23,9% |
| 16:3^{Δ7c,10c,13c} | 9,2% | 16: 3^{Δ7c,10c,13c} | 12,0% |
| 18:0 | 10,6% | 18:0 | 8,6% |
| 18:1^{Δ9c} | 34,9% | 18:1^{Δ9c} | 20,6% |
| 18:1^{Δ11c} | 1,1% | 18 : 1^{Δ11c} | 1,4% |
| 18:3^{Δ9c,12c,15c} | 3,7% | 18 : 3^{Δ9c,12c,15c} | 21,4% |

ii) Lipidmuster von Hefen, die mit dem pYES Plasmid ohne Fragmentinsertion transformiert sind oder das Pp-PSE1 Gen exprimieren (Angaben in mo1%) nach Fütterung mit 500 mikrom Pinolensäure (18:3^{Δ6c,9c,12c}).

**Tabelle 5:**

| PYES2 | PYES2 | pYPp_PSE1 | PYPp_PSE1 |
|---|---|---|---|
| 16:0 | 18,3% | 16:0 | 16,9% |
| 16:1 ^{Δ9c} | 16,0% | 16:1 ^{Δ69c} | 15,3% |
| 18:0 | 8,6% | 18:0 | 8,4% |
| 18:1 ^{Δ9c} | 16,7% | 18:1 ^{Δ9c} | 17,5% |
| 18:1 ^{Δ11c} | 0,7% | 18:1 ^{Δ11c} | 2,0% |
| 18:3 ^{Δ3c,9c,12c} | 39,8% | 18:3 ^{Δ3c,9c,12c} | 32,6% |
| 20:3 ^{Δ7c,11c,14c} | 0% | 20:3 ^{Δ7c,11c,14c} | 5,1% |

iii)Lipidmuster von Hefen, die mit dem pYES Plasmid ohne Fragmentinsertion transformiert sind oder das Pp-PSE1 Gen exprimieren (Angaben in mol%) nach Fütterung mit 500 mikrom Stearidonsäure (18:4 ^{Δ6c,9c,12c,15c).}

**Tabelle 6:**

| PYES2 | pYES2 | pYPp__PSE1 | PYPp_PSE1 |
|---|---|---|---|
| 16:0 | 15,2% | 16:0 | 15,6% |
| 16:1 ^{Δ9c} | 13,1% | 16:1 ^{Δ9c} | 14,9% |
| 18:0 | 12,3% | 18:0 | 10,7% |
| 18:1 ^{Δ9c} | 12,9% | 18:1 ^{Δ9c} | 14,0% |
| 18:1 ^{Δ11c} | 0,7% | 18:1 ^{Δ11c} | 1,2% |
| 18:3 ^{Δ6c,9c,12c,15c} | 45,4% | 18 : 3 ^{Δ6c,9c,12c,15c} | 23,8% |
| 20:4 ^{Δ8c, 11c, 14c, 17c} | 0,4% | 20:4 | 19,8% |

iv) Lipidmuster von Hefen, die mit dem pYES Plasmid ohne Fragmentinsertion transformiert sind oder das Pp-PSE1 Gen exprimieren (Angaben in mol%) nach Fütterung mit 500 mikrom Linolsäure (18:2 ^{Δ9c,12c}).

**Tabelle 7:**

| pYES2 | pYES2 | pYPp_PSE1 | PYPp_PSE1 |
|---|---|---|---|
| 16:0 | 7,9% | 16:0 | 8,7% |
| 16:1 ^{Δ9c} | 1,2% | 16:1 ^{Δ9c} | 1,3% |
| 18:0 | 5,3% | 18:0 | 5,1% |
| 18:1 ^{Δ9c} | 1,3% | 18:1 ^{Δ9c} | 1,3% |
| 18:2 ^{Δ9c,12c} | 83,9% | 18:2 ^{Δ9c,12c} | 80,4% |
| 20: 2^{Δ11c, 14c} | 0,5% | 20:2 ^{Δ11c,14c} | 3,2% |

### B) Elongation von Fettsäuren durch eine Elongase aus Thraustochytrium

Für die Expression in Hefe wird der Thraustochytrium-cDNA-Klon aus SEQ ID NR: 3 (Tc_PSE2), der ein PUFA-spezifisches Elongase-(PSE)-Gen codiert, zuerst so modifiziert, daß es ein funktionell aktives Polypeptid darstellt. Zu diesem Zweck wird der N-Terminus des Proteins auf DNA-Ebene durch die wenigen fehlenden Basen aus der Physcomitrella patens Elongase um 42 Basenpaare verlängert. Es kann aber auch lediglich ein Startcodon im passenden Leseraster zur Sequenz hinzugefügt werden.
Folgende Oligonukleotide werden für das PCR-Experiment eingesetzt: pTCPSE2-3': aaggatccctgagttttagctcccttttgctttcc

Zusätzlich ist in beiden Oligonukleotiden eine BamHI-Restriktionsstelle und die Hefe-Konsensussequenz für hoch-effiziente Translation enthalten (Kozak, M. (1986) Point mutations define a sequence flanking the AUG initiator codon that modulates translation by eukaryotic ribosomes, Cell 44, 283-292).

Die PCR-Reaktion wird mit Plasmid-DNA als Matrize in einem Thermocycler (Biometra) mit der Pfu-DNA- (Stratagene) Polymerase und dem folgenden Temperaturprogramm durchgeführt: 3 min bei 96°C, gefolgt von 25 Zyklen mit 30 s bei 96°C, 30 s bei 55°C und 3 min bei 72°C, 1 Zyklus mit 10 min bei 72°C und Stop bei 4°C.

Die korrekte Größe des amplifizierten DNA-Fragments wird mittels Agarose-TBE-Gelelektrophorese bestätigt. Die amplifizierte DNA wird aus dem Gel mit dem QIAquick-Gelextraktionskit (QIAGEN) extrahiert und in die SmaI-Restriktionsstelle des dephosphorylierten Vektors pUC18 unter Verwendung des Sure Clone Ligation Kit (Pharmacia) ligiert, wobei pUC-hybrid-Tc_PSE2 erhalten wird. Nach der Transformation von E. coli XL1 Blue MRF' kan wurde eine DNA-Minipräparation (Riggs, M.G., & McLachlan, A. (1986) A simplified screening procedure for large numbers of plasmid mini-preparation. BioTechniques 4, 310-313) an 24 ampicillinresistenten Transformanten durchgeführt, und positive Klone wurden mittels BamHI-Restriktionsanalyse identifiziert. Die Sequenz des klonierten PCR-Produktes wurde mittels Resequenzierung unter Verwendung des ABI PRISM Big Dye Terminator Cycle Sequencing Ready Reaction Kit (Perkin-Elmer, Weiterstadt) bestätigt.

Die Plasmid-DNA von pUC-PSE1 sowie pUC- hybrid-Tc_PSE2 wurden zudem mit BamHI gespalten und die erhaltene Fragmente wurden in die BamHI-Restriktionsstelle des dephosphorylierten Hefe-E. coli-Shuttlevektors pYES2 ligiert, wobei pY2 hybrid-Tc_PSE2 erhalten wird. Nach der Transformation von E. coli und DNA-Minipräparation aus den Transformanten wurde die Orientierung des DNA-Fragments im Vektor durch HindIII-Spaltung überprüft. Ein Klon wurde jeweils für die DNA-Maxipräparation mit dem Nucleobond^{®} AX 500 Plasmid-DNA-Extraktionskit (Macherey-Nagel, Düringen) angezogen. Saccharomyces INVSc1 wird mit pY2PSE1, pYES2, pY2-hybrid-Tc_PSE2 und pYES2 mittels eines modifizierten PEG/Lithiumacetat-Protokolls transformiert (Ausubel et al., 1995). Nach der Selektion auf CMdum-Agarplatten mit 2 % Glucose werden je vier pY2PSE1-Transformanten (pY2PSEla-d), pY2-hybrid-Tc_PSE2 -Transformanten (pY2-hybrid-Tc_PSE2 1a-d) und eine pYES2-Transformante zur weiteren Anzucht und funktionellen Expression ausgewählt.

### Funktionelle Expression einer Elongaseaktivität in Hefe

### Vorkultur:

20 ml CMdum-Flüssigmedium mit 2 % (Gew./Vol.) Raffinose wurden mit den transgenen Hefeklonen (pY2- hybrid-Tc_PSE2 1a-d, pYES2) angeimpft und 3 T bei 30°C, 200 rpm gezüchtet, bis eine optische Dichte bei 600 nm (OD₆₀₀) von 1,5-2 erreicht wurde.

### Hauptkultur:

Für die Expression wurden 20 ml CMdum-Flüssigmedium mit 2 % Raffinose und 1 % (Vol./Vol.) Tergitol NP-40 mit Fettsäuresubstraten auf eine Endkonzentration von 0,003 % (Gew./Vol.) angereichert. Die Medien werden mit den Vorkulturen auf eine oD₆₀₀ von 0,05 angeimpft. Die Expression wurde bei einer OD₆₀₀ von 0,2 mit 2 % (Gew./Vol.) Galaktose für 16 Std. induziert, wonach die Kulturen eine OD₆₀₀ von 0,8-1,2 geerntet wurden.

### Fettsäureanalyse:

Die Gesamt-Fettsäuren wurden aus Hefekulturen extrahiert und mittels Gaschromatographie analysiert. Davon wurden Zellen von 5 ml Kultur mittels Zentrifugation (1000 x g, 10 min, 4°C) geerntet und einmal mit 100 mM NaHCO₃, pH 8,0, gewaschen, um restliches Medium und Fettsäuren zu entfernen. Zur Herstellung des Fettsäuremethylester (FAMES) wurden die Zellsedimente mit 1M methanolischer H₂SO₄ und 2% (Vol./Vol.) Dimethoxypropan für 1 Std. bei 80°C behandelt. Die FAMES wurden zweimal mit 2 ml Petrolether extrahiert, einmal mit 100 mM NaHCO₃ pH 8,0, und einmal mit destilliertem Wasser gewaschen und mit Na₂SO₄ getrocknet. Das organische Lösungsmittel wurde unter einem Argonstrom verdampft, und die FAMES wurden in 50 mikrol Petrolether gelöst. Die Proben wurden auf einer ZEBRON-ZB-Wax-Kapillarsäule (30 m, 0,32 mm, 0,25 mikro m; Phenomenex) in einem Hewlett Packard-6850-Gaschromatograph mit einem Flammenionisationsdetektor aufgetrennt. Die Ofentemperatur wurde von 70°C (1 min halten) bis 200°C mit einer Rate von 20°C/min, dann auf 250°C (5 min halten) mit einer Rate von 5°C/min und schließlich auf 260°C mit einer Rate von 5°C/min programmiert. Stickstoff wurde als Trägergas verwendet (4,5 ml/min bei 70°C). Die Fettsäuren wurden durch Vergleich mit Retentionszeiten von FAME-Standards (SIGMA) identifiziert.

Die Fettsäuremuster von fünf transgenen Hefestämmen in Mol.-% sind in Tabelle 1 gezeigt.

Die Verhältnisse der zugegebenen und aufgenommenen g-Linolensäure sind durch fettgedruckte Zahlen hervorgehoben, die der elongierten Produkte durch rote Zahlen und die der elongierten g-Linolensäure durch fettgefruckte Zahlen (letzte Zeile).

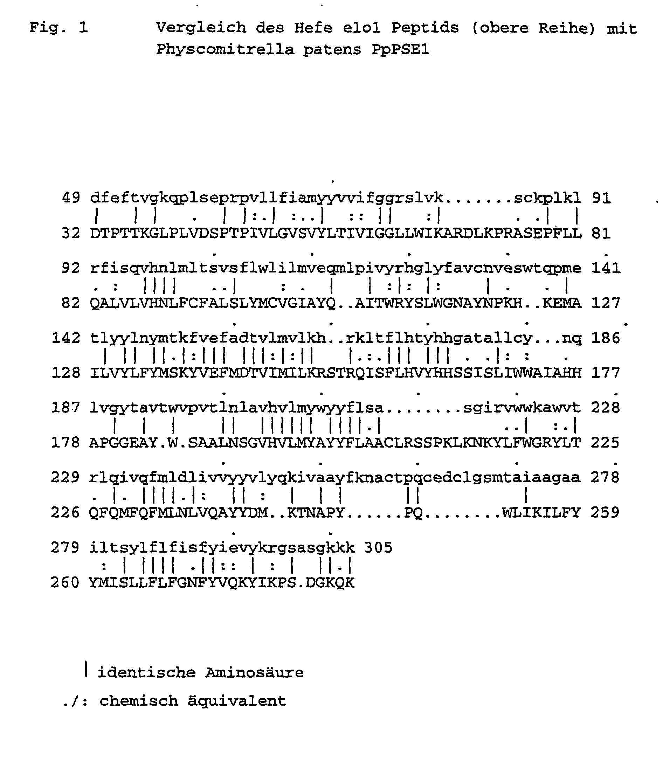

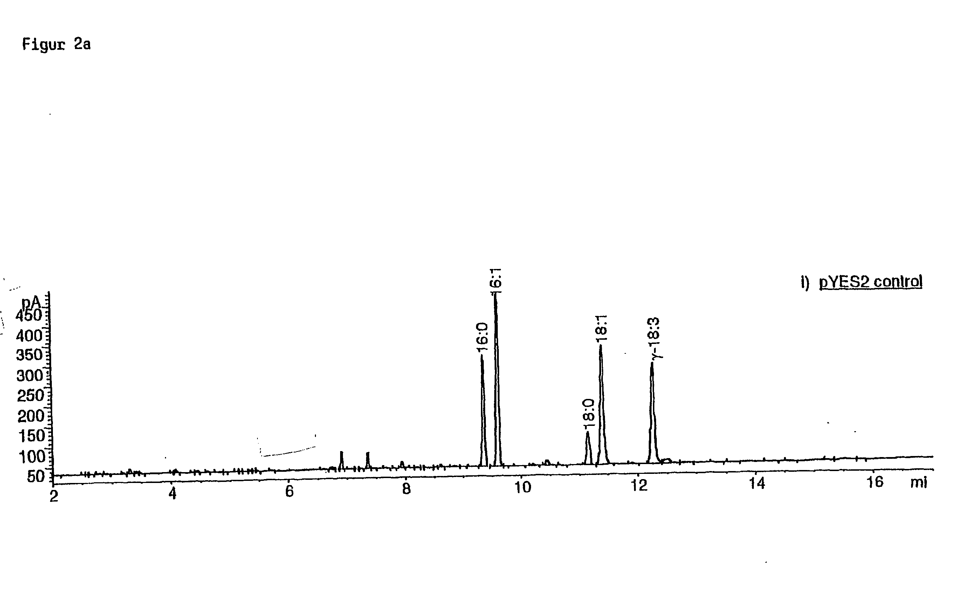

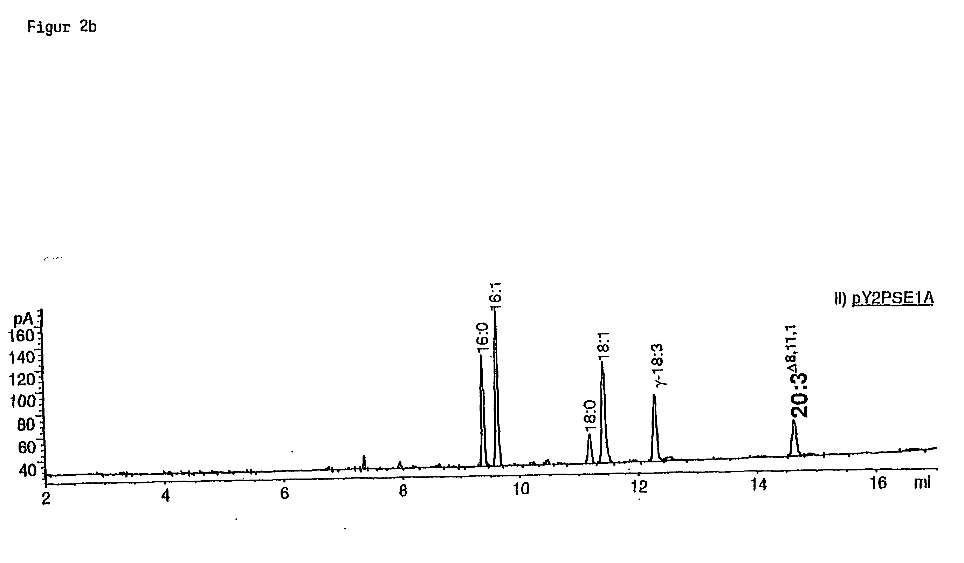

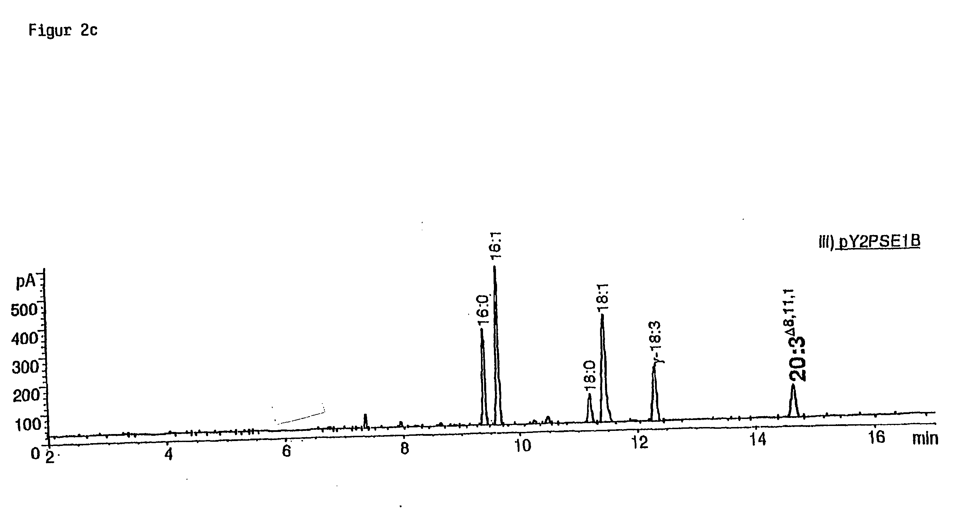

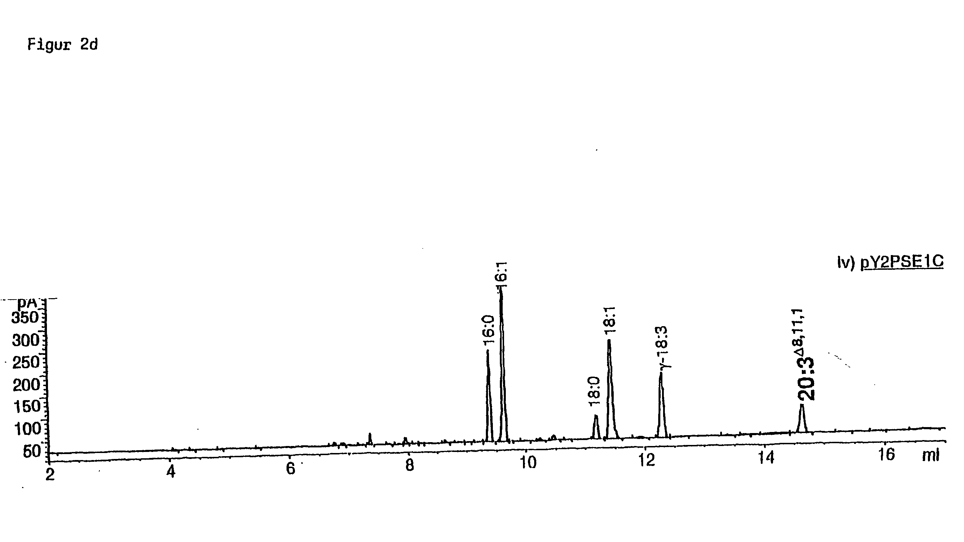

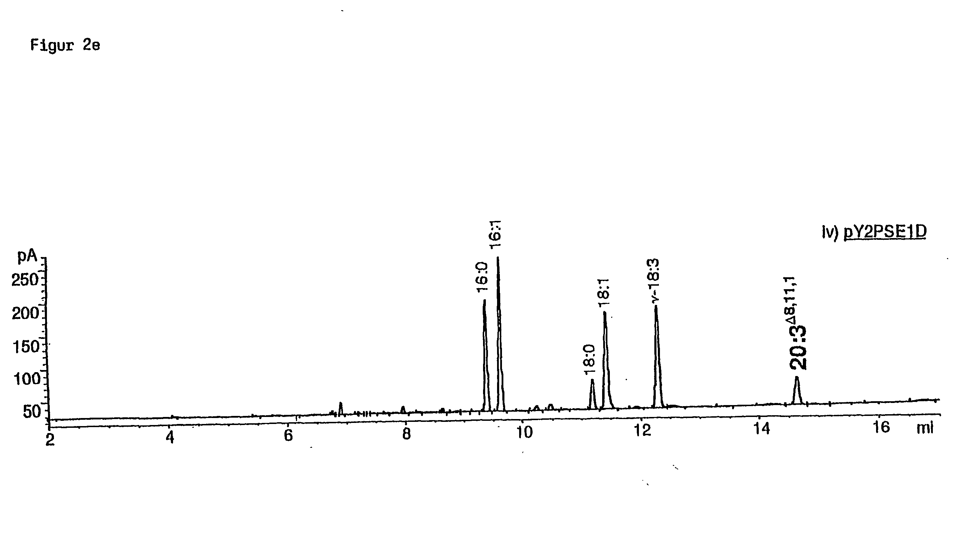

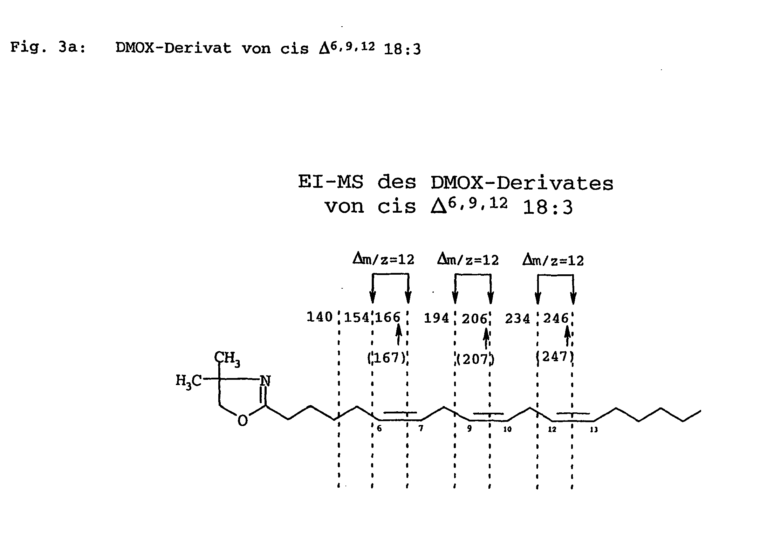

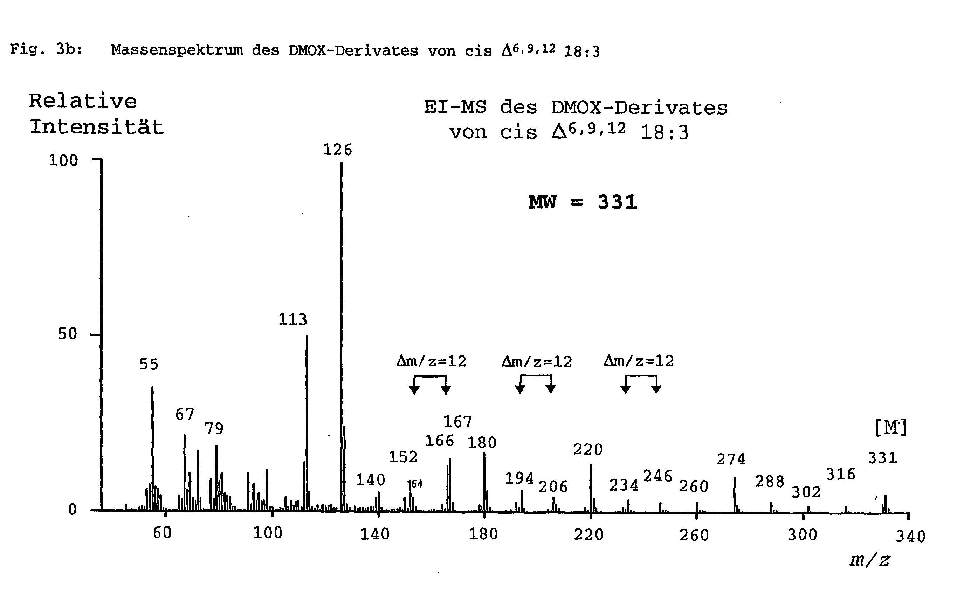

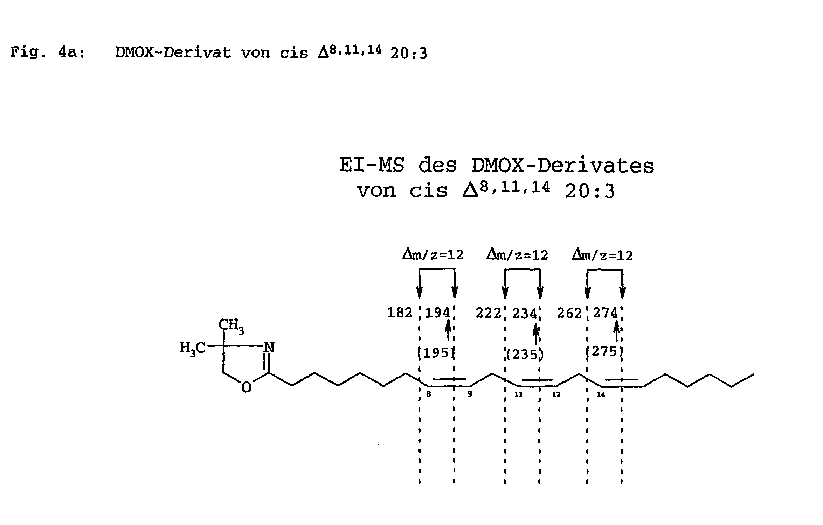

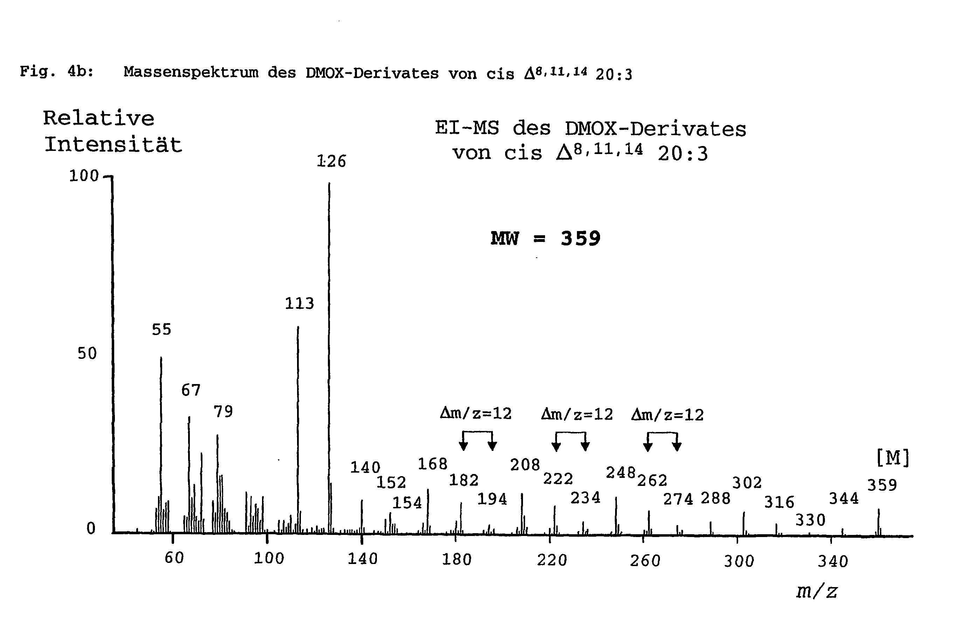

Die GC-Analyse von FAMES, die aus Gesamt-Lipiden der mit pYES2 (i/Kontrolle) und pY2PSE1 (ii-iv c+d/ jeweils transformiert mit pY2PSE1A, pY2PSE1B, pY2PSE1C, pY2PSE1D) transformierten Hefen ist in Figur 2a-e gezeigt. Für die Analyse wurden die transgenen Hefen in Anwesenheit von g-18:3 gezüchtet. Die Tab. 1 zeigt ihre Fettsäuremuster in Mol.-%. Die Aufnahme von g-18:3 ist durch fettgedruckte Zahlen hervorgehoben, das Elongationsprodukt Dihomo-g-linolensäure (20:3D8,11,14) ist unterstrichen und das Verhältnis g-18:3-Elongationsprodukt (ebenfalls in Mol.-%) durch fettgedruckte Zahlen (letzte Zeile). Die Struktur und die Massenspektren des DMOX-Derivates von cis-D6,9,12 C18:3 sind aus Fig. 3a+b ersichtlich. Die Struktur und die Massenspektren des DMOX-Derivates von D8,11,14 C20:3 sind aus Fig. 4a+b ersichtlich.

Die Ergebnisse zeigen, daß g-18:3 in großen Mengen in alle transgenen Hefen eingebaut worden ist. Alle vier transgenen Hefeklone, die mit pY2PSE1 transformiert wurden, zeigen einen zusätzlichen Peak im Gaschromatogramm, der durch Vergleich der Retentionszeiten als 20:3 DB,11,14 identifiziert wurde. Eine Gaschromatographie/Massenspektroskopie kann zusätzliche Unterstützung zur Bestätigung dieser Identität liefern. Der Anteil an elongierter g-18:3 betrug, wie in Tabelle 1 gezeigt, 23,7 bis 40,5 %. Ferner konnte keine signifikante Elongation von Palmitinsäure (16:0), Palmitoleinsäure (16:1), Stearinsäure (18:0) oder Ölsäure (18:1 D9) beobachtet werden.

Die identifizierten Produkte zeigen, daß die Nukleotidsequenz von PpPSE1 eine D6-selektive Fettsäure-Elongase aus dem Moos Physcomitrella patens codiert, die zur Bildung neuer Fettsäuren in transgenen Hefen führt.

Die Verhältnisse der zugegebenen und aufgenommenen Fettsäuresubstrate können wie vorstehend ermittelt werden und so Quantität und Qualität der Elongasereaktion erfaßt werden.

Die Struktur und die Massenspektren von DMOX-Derivaten ergeben auch die jeweilige Position einer Doppelbindung.

Weitere Fütterungsexperimente mit verschiedensten anderen Fettsäuren (z.B. Arachidonsäure, Eicosapentaensäure etc.) können zur detaillierteren Bestätigung der Substratselektivität dieser Elongase durchgeführt werden.

### Beispiel 18: Reinigung des gewünschten Produktes aus transformierten Organismen allgemein

Die Gewinnung des gewünschten Produktes aus Pflanzenmaterial oder Pilzen, Algen, Ciliaten, tierischen Zellen oder aus dem Überstand der vorstehend beschriebenen Kulturen kann durch verschiedene, im Fachgebiet bekannte Verfahren erfolgen. Wird das gewünschte Produkt nicht aus den Zellen sezerniert, können die Zellen aus der Kultur durch langsame Zentrifugation geerntet werden, die Zellen können durch Standardtechniken, wie mechanische Kraft oder Ultraschallbehandlung, lysiert werden. Organe von Pflanzen können mechanisch von anderem Gewebe oder anderen Organen getrennt werden. Nach der Homogenisation werden die Zelltrümmer durch Zentrifugation entfernt, und die Überstandsfraktion, welche die löslichen Proteine enthält, wird zur weiteren Reinigung der gewünschten Verbindung aufbewahrt. Wird das Produkt aus gewünschten Zellen sezerniert, werden die Zellen durch langsame Zentrifugation aus der Kultur entfernt, und die Überstandsfraktion wird zur weiteren Reinigung aufbewahrt.

Die Überstandsfraktion aus jedem Reinigungsverfahren wird einer Chromatographie mit einem geeigneten Harz unterworfen, wobei das gewünschte Molekül entweder auf dem Chromatographieharz zurückgehalten wird, viele Verunreinigungen in der Probe jedoch nicht, oder die Verunreinigungen auf dem Harz zurückbleiben, die Probe hingegen nicht. Diese Chromatographieschritte können wenn nötig wiederholt werden, wobei die gleichen oder andere Chromatographieharze verwendet werden. Der Fachmann ist in der Auswahl geeigneter Chromatographieharze und ihrer wirksamsten Anwendung für ein bestimmtes zu reinigendes Molekül bewandert. Das gereinigte Produkt kann durch Filtration oder Ultrafiltration konzentriert und bei einer Temperatur aufbewahrt werden, bei der die Stabilität des Produktes maximal ist.

Im Fachgebiet ist ein breites Spektrum an Reinigungsverfahren bekannt, und das vorstehende Reinigungsverfahren soll nicht beschränkend sein. Diese Reinigungsverfahren sind zum Beispiel beschrieben in Bailey, J.E., & Ollis, D.F., Biochemical Engineering Fundamentals, McGraw-Hi11: New York (1986).

Die Identität und Reinheit der isolierten Verbindungen kann durch Standardtechniken des Fachgebiets bestimmt werden. Dazu gehören Hochleistungs-Flüssigkeitschromatographie (HPLC), spektroskopische Verfahren, Färbeverfahren, Dünnschichtchromatographie, NIRS, Enzymtest oder mikrobiologisch. Eine Übersicht über diese Analyseverfahren siehe in: Patek et al. (1994) Appl. Environ. Microbiol. 60:133-140; Malakhova et al. (1996) Biotekhnologiya 11:27-32; und Schmidt et al. (1998) Bioprocess Engineer. 19:67-70. Ulmann's Encyclopedia of Industrial Chemistry (1996) Bd. A27, VCH: Weinheim, S. 89-90, S. 521-540, S. 540-547, S. 559-566, 575-581 und S. 581-587; Michal, G (1999) Biochemical Pathways: An Atlas of Biochemistry and Molecular Biology, John Wiley and Sons; Fallon, A., et al. (1987) Applications of HPLC in Biochemistry in: Laboratory Techniques in Biochemistry and Molecular Biology, Bd. 17.

### Äquivalente

Der Fachmann erkennt oder kann viele Äquivalente der hier beschriebenen erfindungsgemäßen spezifischen Ausführungsformen feststellen, indem er lediglich Routineexperimente verwendet. Diese Äquivalente sollen von den Patentansprüchen umfaßt sein.

## Patentansprüche

1. Isolierte Nukleinsäure, umfassend eine Nukleotidsequenz, die ein Polypeptid codiert, das C₁₆-, C₁₈- oder C₂₀-Fettsäuren mit mindestens zwei Doppelbindungen in der Fettsäure um mindestens zwei Kohlenstoffatome verlängert, ausgewählt aus der Gruppe, bestehend aus
a) einer in SEQ ID NO: 1, SEQ ID NO: 3 oder SEQ ID NO: 9 dargestellten Nukleinsäuresequenz,
b) einer Nukleinsäure, die gemäß dem degenerierten genetischen Code von der in SEQ ID NO:1, SEQ ID NO:3 oder SEQ ID NO:9 dargestellten Sequenz stammt,
c) Derivate der in SEQ ID NO:1, SEQ ID NO:3 oder SEQ ID NO:9 dargestellten Sequenz, die für Polypeptide der in SEQ ID NO:2, SEQ ID NO:4 oder SEQ ID NO:10 dargestellten Aminosäuresequenz codieren und mindestens 50 % Identität auf Aminosäureebene aufweisen, ohne dass die enzymatische Wirkung der Polypeptide wesentlich reduziert ist.

2. Isolierte Nukleinsäure nach Anspruch 1, wobei die Sequenz von einer Pflanze, Alge oder Pilz stammt.

3. Isolierte Nukleinsäuresequenz nach Anspruch 1 oder Anspruch 2, wobei die Sequenz von Physcomitrella oder Thraustochytrium stammt.

4. Genkonstrukt, umfassend eine isolierte Nukleinsäure nach einem der Ansprüche 1 bis 3, wobei die Nukleinsäure funktionell mit einem oder mehreren Regulationssignalen verbunden ist.

5. Genkonstrukt nach Anspruch 4, dessen Genexpression durch die Regulationssignale verstärkt wird.

6. Genkonstrukt, enthaltend eine Nukleinsäuresequenz nach den Ansprüchen 1 bis 3 oder ein Genkonstrukt nach Anspruch 4 oder 5 und mindestens eine weitere Nukleinsäure, die für ein Gen der Fettsäurebiosynthese codiert, ausgewählt aus der Gruppe Δ19-, Δ17-, Δ15-, Δ12-, Δ9-, Δ8-, Δ6-, Δ5-, Δ4-Desaturase, die verschiedenen Hydroxylasen, die Δ12-Acetylenase, die Acyl-ACP-Thioesterasen, β-Ketoacyl-ACP-Synthasen oder β-Ketoacyl-ACP-Reductasen.

7. Aminosäuresequenz, die durch die isolierte Nukleinsäuresequenz nach den Ansprüchen 1 bis 3 oder das Genkonstrukt nach einem der Ansprüche 4 bis 6 codiert wird.

8. Vektor, umfassend eine Nukleinsäure nach einem der Ansprüche 1 bis 3 oder ein Genkonstrukt nach einem der Ansprüche 4 bis 6.

9. Transgener nicht-menschlicher Organismus, umfassend mindestens eine Nukleinsäure nach einem der Ansprüche 1 bis 3, ein Genkonstrukt nach einem der Ansprüche 4 bis 6 oder einen Vektor nach Anspruch 8.

10. Transgener Organismus nach Anspruch 9, wobei der Organismus ein Mikroorganismus, ein Tier oder eine Pflanze ist.

11. Transgener Organismus nach Anspruch 9 oder 10, wobei der Organismus eine transgene Pflanze ist.

12. Antikörper, der spezifisch ein Polypeptid, das von einer der Nukleinsäuren nach einem der Ansprüche 1 bis 3 codiert wird, bindet.

13. Antisense-Nukleinsäuremolekül, das die Komplementärsequenz der Nukleinsäure nach einem der Ansprüche 1 bis 3 umfasst.

14. Verfahren zur Herstellung von PUFAs, wobei das Verfahren das Züchten eines nicht-menschlichen Organismus umfaßt, der eine Nukleinsäure nach Anspruch 1, ein Genkonstrukt nach Anspruch 5 oder einen Vektor nach Anspruch 8 umfaßt, codierend ein Polypeptid, das C₁₆-, C₁₈- oder C₂₀-Fettsäuren mit mindestens zwei Doppelbindungen im Fettsäuremolekül um mindestens zwei Kohlenstoffatome unter Bedingungen verlängert, unter denen PUFAs in dem Organismus gebildet werden.

15. Verfahren nach Anspruch 14, wobei die durch das Verfahren hergestellten PUFAs C₁₈-, C₂₀- oder C₂₂-Fettsäuremoleküle mit mindestens zwei Doppelbindungen im Fettsäuremolekül sind.

16. Verfahren nach Anspruch 14 oder 15, wobei die C₁₈-, C₂₀- oder C₂₂-Fettsäuremoleküle aus dem Organismus in Form eines Öls, Lipids oder einer freien Fettsäure isoliert werden.

17. Verfahren nach einem der Ansprüche 14 bis 16, wobei der Organismus ein Mikroorganismus, ein Tier oder eine Pflanze ist.

18. Verfahren nach einem der Ansprüche 14 bis 16, wobei der Organismus eine transgene Pflanze ist.

19. Verfahren nach einem der Ansprüche 14 bis 18, wobei die C₁₆-, C₁₈- oder C₂₀-Fettsäure eine Fettsäure mit drei Doppelbindungen im Molekül ist.

## Claims

1. An isolated nucleic acid comprising a nucleotide sequence encoding a polypeptide which elongates C₁₆-, C₁₈- or C₂₀-fatty acids with at least two double bonds in the fatty acid by at least two carbon atoms, selected from the group consisting of
a) a nucleic acid sequence shown in SEQ ID NO:1, SEQ ID NO:3 or SEQ ID NO:9,
b) a nucleic acid which, in accordance with the degeneracy of the genetic code, is derived from the sequence shown in SEQ ID NO:1, SEQ ID NO:3 or SEQ ID NO:9,
c) derivatives of the sequence shown in SEQ ID NO: 1, SEQ ID NO: 3 or SEQ ID NO: 9 which encode polypeptides of the amino acid sequence shown in SEQ ID NO:2, SEQ ID NO: 4 or SEQ ID NO: 9 and which have at least 50% identity at the amino acid level without the enzymatic action of the polypeptides being substantially reduced.

2. The isolated nucleic acid sequence according to claim 1, wherein the sequence is derived from a plant, alga or fungus.

3. The isolated nucleic acid sequence according to claim 1 or 2, wherein the sequence is derived from Physcomitrella or Thraustochytrium.

4. A gene construct comprising an isolated nucleic acid according to any of claims 1 to 3, wherein the nucleic acid is functionally linked to one or more regulatory signals.

5. The gene construct according to claim 4, whose gene expression is enhanced by the regulatory signals.

6. A gene construct comprising a nucleic acid sequence according to any of claims 1 to 3 or a gene construct according to claim 4 or 5 and at least one further nucleic acid which encodes a fatty acid biosynthesis gene, selected from the group consisting of Δ19-, Δ17-, Δ15-, Δ12-, Δ9-, Δ8-, Δ6-, Δ5-, Δ4-desaturase, the various hydroxylases, Δ12-acetylenase, acyl-ACP thioesterases, β-ketoacyl-ACP synthases or β-ketoacyl-ACP reductases.

7. An amino acid sequence which is encoded by the isolated nucleic acid sequence according to any of claims 1 to 3 or the gene construct according to any of claims 4 to 6.

8. A vector comprising a nucleic acid according to any of claims 1 to 3 or a gene construct according to any of claims 4 to 6.

9. A transgenic nonhuman organism comprising at least one nucleic acid according to any of claims 1 to 3, a gene construct according to any of claims 4 to 6 or a vector according to claim 8.

10. A transgenic organism according to claim 9, wherein the organism is a microorganism, an animal or a plant.

11. A transgenic organism according to claim 9 or 10, wherein the organism is a transgenic plant.

12. An antibody which binds specifically to a polypeptide which is encoded by one of the nucleic acids according to any of claims 1 to 3.

13. An antisense nucleic acid molecule which encompasses the complementary sequence of the nucleic acid according to any one of claims 1 to 3.

14. A process for the preparation of PUFAs, which comprises culturing a nonhuman organism which encompasses a nucleic acid according to claim 1, a gene construct according to claim 5 or a vector according to claim 8, encoding a polypeptide which elongates C₁₆-, C₁₈- or C₂₀-fatty acids with at least two double bonds in the fatty acid molecule by at least two carbon atoms under conditions under which PUFAs are formed in the organism.

15. The process according to claim 14, wherein the PUFAs prepared by the process are C₁₈-, C₂₀- or C₂₂-fatty acid molecules with at least two double bonds in the fatty acid molecule.

16. The process according to claim 14 or 15, wherein the C₁₈-, C₂₀- or C₂₂-fatty acid molecules are isolated from the organism in the form of an oil, lipid or a free fatty acid.

17. The process according to any of claims 14 to 16, wherein the organism is a microorganism, an animal or a plant.

18. The process according to any of claims 14 to 16, wherein the organism is a transgenic plant.

19. The process according to any of claims 14 to 18, wherein the C₁₆-, C₁₈- or C₂₀-fatty acid is a fatty acid with three double bonds in the molecule.

## Revendications

1. Acide nucléique isolé, comprenant une séquence nucléotidique codant pour un polypeptide qui prolonge d'au moins deux atomes- de carbone des acides gras en C₁₆, C₁₈ ou C₂₀ comportant au moins deux doubles liaisons dans l'acide gras, choisi dans le groupe constitué par
a) une séquence d'acide nucléique représentée dans SEQ ID n° 1, SEQ ID n° 3 ou SEQ ID n° 9,
b) une séquence d'acide nucléique qui dérive, selon le code génétique dégénéré, de la séquence représentée dans SEQ ID n° 1, SEQ ID n° 3 ou SEQ ID n° 9,
c) des dérivés de la séquence représentée dans SEQ ID n° 1, SEQ ID n° 3 ou SEQ ID n° 9, qui codent pour des polypeptides de la séquence d'aminoacides représentée dans SEQ ID n° 2, SEQ ID n° 4 ou SEQ ID n° 10 et présentent une identité d'au moins 50 % sur le plan des aminoacides, sans que l'activité enzymatique des polypeptides soit réduite de façon importante.

2. Acide nucléique isolé selon la revendication 1, dans lequel la séquence est issue d'une plante, d'une algue ou d'un champignon.

3. Séquence d'acide nucléique isolé selon la revendication 1 ou la revendication 2, la séquence provenant de *Physcomitrella* ou *Thraustochytrium.*

4. Produit de construction génique, comprenant un acide nucléique isolé selon l'une quelconque des revendications 1 à 3, dans lequel l'acide nucléique est fonctionnellement lié à un ou plusieurs signaux de régulation.

5. Produit de construction génique selon la revendication 4, dont l'expression de gène est renforcée par des signaux de régulation.

6. Produit de construction génique, contenant une séquence d'acide nucléique selon les revendications 1 à 3 ou un produit de construction génique selon la revendication 4 ou 5 et au moins un autre acide nucléique qui code pour un gène de la biosynthèse des acides gras, choisi dans le groupe constitué par la D19-, D17-, D15-, D12-, D9-, D8-, D6-, D5-, D4-désaturase, les diverses hydrolases, la D12-acétylénase, les acyl-ACP-thioestérases, les β-cétoacyl-ACP-synthases et les β-cétoacyl-ACP-réductases.

7. Séquence d'aminoacides, qui est codée par la séquence d'acide nucléique isolée selon les revendications 1 à 3 ou le produit de construction génique selon l'une quelconque des revendications 4 à 6.

8. Vecteur, comprenant un acide nucléique selon l'une quelconque des revendications 1 à 3 ou un produit de construction génique selon l'une quelconque des revendications 4 à 6.

9. Organisme non humain transgénique, comprenant au moins un acide nucléique selon l'une quelconque des revendications 1 à 3, un produit de construction génique selon l'une quelconque des revendications 4 à 6 ou un vecteur selon la revendication 8.

10. Organisme transgénique selon la revendication 9, l'organisme étant un micro-organisme, un animal ou une plante.

11. Organisme transgénique selon la revendication 9 ou 10, l'organisme étant une plante transgénique.

12. Anticorps qui se lie spécifiquement à un polypeptide qui est codé par l'un des acides nucléiques selon l'une quelconque des revendications 1 à 3.

13. Molécule d'acide nucléique antisens, qui comprend la séquence complémentaire de l'acide nucléique selon l'une quelconque des revendications 1 à 3.

14. Procédé pour la production de PUFA (acides gras polyinsaturés), le procédé comprenant la culture/l'élevage d'un organisme non humain qui comprend un acide nucléique selon la revendication 1, un produit de construction génique selon la revendication 5 ou un vecteur selon la revendication 8, codant pour un polypeptide qui prolonge d'au moins deux atomes de carbone des acides gras en C₁₆, C₁₈ ou C₂₀ comportant au moins deux doubles liaisons dans la molécule d'acide gras, dans des conditions dans lesquelles des PUFA sont formés dans l'organisme.

15. Procédé selon la revendication 14, dans lequel les PUFA produits par le procédé sont des molécules d'acides gras en C₁₈, C₂₀ ou C₂₂ comportant au moins deux doubles liaisons dans la molécule d'acide gras.

16. Procédé selon la revendication 14 ou 15, dans lequel les molécules d'acides gras en C₁₈, C₂₀ ou C₂₂ sont isolées à partir de l'organisme sous forme d'une huile, d'un lipide ou d'un acide gras libre.

17. Procédé selon l'une quelconque des revendications 14 à 16, dans lequel l'organisme est un micro-organisme, un animal ou une plante.

18. Procédé selon l'une quelconque des revendications 14 à 16, dans lequel l'organisme est une plante transgénique.

19. Procédé selon l'une quelconque des revendications 14 à 18 dans lequel l'acide gras en C₁₆, C₁₈ ou C₂₀ est un acide gras à trois doubles liaisons dans la molécule.


### SEQUENZPROTOKOLL

<110> BASF Aktiengesellschaft
<120> New Elongase gene and a process for the production of polyunsaturated fatty acids
<130> 0050/51159
<140> 20000081 <141> 2000-02-09
<160> 12
<170> PatentIn Vers. 2.0
<210> 1
   <211> 1192
   <212> DNA
   <213> Physcomitrella patens
<220>
   <221> CDS
   <222> (58)..(930)
<400> 1
   ctgcttcgtc tcatcttggg ggtgtgattc gggagtgggt tgagttggtg gagcgca    57
   atg gag gtc gtg gag aga ttc tac ggt gag ttg gat ggg aag gtc tcg   105
   Met Glu Val Val Glu Arg Phe Tyr Gly Glu Leu Asp Gly Lys Val Ser
    1               5                  10                  15
   cag ggc gtg aat gca ttg ctg ggt agt ttt ggg gtg gag ttg acg gat   153
   Gln Gly Val Asn Ala Leu Leu Gly Ser Phe Gly Val Glu Leu Thr Asp
               20                  25                  30
   acg ccc act acc aaa ggc ttg ccc ctc gtt gac agt ccc aca ccc atc   201
   Thr Pro Thr Thr Lys Gly Leu Pro Leu Val Asp Ser Pro Thr Pro Ile
           35                  40                  45
   gtc ctc ggt gtt tct gta tac ttg act att gtc att gga ggg ctt ttg   249
   Val Leu Gly Val Ser Val Tyr Leu Thr Ile Val Ile Gly Gly Leu Leu
       50                  55                  60
   tgg ata aag gcc agg gat ctg aaa ccg cgc gcc tcg gag cca ttt ttg   297
   Trp Ile Lys Ala Arg Asp Leu Lys Pro Arg Ala Ser Glu Pro Phe Leu
   65                  70                  75                  80
   ctc caa gct ttg gtg ctt gtg cac aac ctg ttc tgt ttt gcg ctc agt   345
   Leu Gln Ala Leu Val Leu Val His Asn Leu Phe Cys Phe Ala Leu Ser
                   85                  90                  95
   ctg tat atg tgc gtg ggc atc gct tat cag gct att acc tgg cgg tac   393
   Leu Tyr Met Cys Val Gly Ile Ala Tyr Gln Ala Ile Thr Trp Arg Tyr
               100                 105                 110
   tct ctc tgg ggc aat gca tac aat cct aaa cat aaa gag atg gcg att   441
   Ser Leu Trp Gly Asn Ala Tyr Asn Pro Lys His Lys Glu Met Ala Ile
           115                 120                 125
   ctg gta tac ttg ttc tac atg tct aag tac gtg gaa ttc atg gat acc   489
   Leu Val Tyr Leu Phe Tyr Met Ser Lys Tyr Val Glu Phe Met Asp Thr
       130                 135                 140
   gtt atc atg ata ctg aag cgc agc acc agg caa ata agc ttc ctc cac   537
   Val Ile Met Ile Leu Lys Arg Ser Thr Arg Gln Ile Ser Phe Leu His
   145                 150                 155                 160
   gtt tat cat cat tct tca att tcc ctc att tgg tgg gct att gct cat   585
   Val Tyr His His Ser Ser Ile Ser Leu Ile Trp Trp Ala Ile Ala His
                   165                 170                 175
   cac gct cct ggc ggt gaa gca tat tgg tct gcg gct ctg aac tca gga   633
   His Ala Pro Gly Gly Glu Ala Tyr Trp Ser Ala Ala Leu Asn Ser Gly
               180                 185                 190
   gtg cat gtt ctc atg tat gcg tat tac ttc ttg gct gcc tgc ctt cga   681
   Val His Val Leu Met Tyr Ala Tyr Tyr Phe Leu Ala Ala Cys Leu Arg
           195                 200                 205
   agt agc cca aag tta aaa aat aag tac ctt ttt tgg ggc agg tac ttg   729
   Ser Ser Pro Lys Leu Lys Asn Lys Tyr Leu Phe Trp Gly Arg Tyr Leu
       210                 215                 220
   aca caa ttc caa atg ttc cag ttt atg ctg aac tta gtg cag gct tac   777
   Thr Gln Phe Gln Met Phe Gln Phe Met Leu Asn Leu Val Gln Ala Tyr
   225                 230                 235                 240
   tac gac atg aaa acg aat gcg cca tat cca caa tgg ctg atc aag att   825
   Tyr Asp Met Lys Thr Asn Ala Pro Tyr Pro Gln Trp Leu Ile Lys Ile
                   245                 250                 255
   ttg ttc tac tac atg atc tcg ttg ctg ttt ctt ttc ggc aat ttt tac   873
   Leu Phe Tyr Tyr Met Ile Ser Leu Leu Phe Leu Phe Gly Asn Phe Tyr
               260                 265                 270
   gta caa aaa tac atc aaa ccc tct gac gga aag caa aag gga gct aaa    921
   Val Gln Lys Tyr Ile Lys Pro Ser Asp Gly Lys Gln Lys Gly Ala Lys
           275                 280                 285
   act gag tga gctgtatcaa gccatagaaa ctctattatg ttagaacctg            970
   Thr Glu
       290
   aagttggtgc tttcttatct ccacttatct tttaagcagc atcagttttg aaatgatgtg 1030
   tgggcgtggt ctgcaagtag tcatcaatat aatcggcctg agcacttcag atggattgtt 1090
   agaacatgag taaaagcggt tattacggtg tttattttgt accaaatcac cgcacgggtg 1150
   aattgaaata tttcagattt gatcaatttc atctgaaaaa aa                     1192
<210> 2
   <211> 290
   <212> PRT
   <213> Physcomitrella patens
<400> 2
   Met Glu Val Val Glu Arg Phe Tyr Gly Glu Leu Asp Gly Lys Val Ser
   1 5 10 15
   Gln Gly Val Asn Ala Leu Leu Gly Ser Phe Gly Val Glu Leu Thr Asp
   20 25 30
   Thr Pro Thr Thr Lys Gly Leu Pro Leu Val Asp Ser Pro Thr Pro Ile
   35 40 45
   Val Leu Gly Val Ser Val Tyr Leu Thr Ile Val Ile Gly Gly Leu Leu
   50 55 60
   Trp Ile Lys Ala Arg Asp Leu Lys Pro Arg Ala Ser Glu Pro Phe Leu
   65 70 75 80
   Leu Gln Ala Leu Val Leu Val His Asn Leu Phe Cys Phe Ala Leu Ser
   85 90 95
   Leu Tyr Met Cys Val Gly Ile Ala Tyr Gln Ala Ile Thr Trp Arg Tyr
   100 105 110
   Ser Leu Trp Gly Asn Ala Tyr Asn Pro Lys His Lys Glu Met Ala Ile
   115 120 125
   Leu Val Tyr Leu Phe Tyr Met Ser Lys Tyr Val Glu Phe Met Asp Thr
   130 135 140
   Val Ile Met Ile Leu Lys Arg Ser Thr Arg Gln Ile Ser Phe Leu His
   145 150 155 160
   Val Tyr His His Ser Ser Ile Ser Leu Ile Trp Trp Ala Ile Ala His
   165 170 175
   His Ala Pro Gly Gly Glu Ala Tyr Trp Ser Ala Ala Leu Asn Ser Gly
   180 185 190
   Val His Val Leu Met Tyr Ala Tyr Tyr Phe Leu Ala Ala Cys Leu Arg
   195 200 205
   Ser Ser Pro Lys Leu Lys Asn Lys Tyr Leu Phe Trp Gly Arg Tyr Leu
   210 215 220
   Thr Gln Phe Gln Met Phe Gln Phe Met Leu Asn Leu Val Gln Ala Tyr
   225 230 235 240
   Tyr Asp Met Lys Thr Asn Ala Pro Tyr Pro Gln Trp Leu Ile Lys Ile
   245 250 255
   Leu Phe Tyr Tyr Met Ile Ser Leu Leu Phe Leu Phe Gly Asn Phe Tyr
   260 265 270
   Val Gln Lys Tyr Ile Lys Pro Ser Asp Gly Lys Gln Lys Gly Ala Lys
   275 280 285
   Thr Glu
   290
<210> 3
   <211> 687
   <212> DNA
   <213> Thraustochytrium
<220>
   <221> CDS
   <222> (1).. (588)
<400> 3
   cgc agc gtg cat aac ctc ggg ctc tgc ctc ttc tcg ggc gcc gtg tgg   48
   Arg Ser Val His Asn Leu Gly Leu Cys Leu Phe Ser Gly Ala Val Trp
     1               5                  10                  15
   atc tac acg agc tac ctc atg atc cag gat ggg cac ttt cgc agc ctc   96
   Ile Tyr Thr Ser Tyr Leu Met Ile Gln Asp Gly His Phe Arg Ser Leu
               20                  25                  30
   gag gcg gca acg tgc gag ccg ctc aag cat ccg cac ttc cag ctc atc   144
   Glu Ala Ala Thr Cys Glu Pro Leu Lys His Pro His Phe Gln Leu Ile
           35                  40                  45
   agc ttg ctc ttt gcg ctg tcc aag atc tgg gag tgg ttc gac acg gtg   192
   Ser Leu Leu Phe Ala Leu Ser Lys Ile Trp Glu Trp Phe Asp Thr Val
       50                  55                  60
   ctc ctc atc gtc aag ggc aac aag ctc cgc ttc ctg cac gtc ttg cac   240
   Leu Leu Ile Val Lys Gly Asn Lys Leu Arg Phe Leu His Val Leu His
   65                  70                  75                  80
   cac gcc acg acc ttt tgg ctc tac gcc atc gac cac atc ttt ctc tcg   288
   His Ala Thr Thr Phe Trp Leu Tyr Ala Ile Asp His Ile Phe Leu Ser
                   85                  90                  95
   tcc atc aag tac ggc gtc gcg gtc aat gct ttc atc cac acc gtc atg   336
   Ser Ile Lys Tyr Gly Val Ala Val Asn Ala Phe Ile His Thr Val Met
               100                 105                 110
   tac gcg cac tac ttc cgc cca ttc ccg aag ggc ttg cgc ccg ctt att   384
   Tyr Ala His Tyr Phe Arg Pro Phe Pro Lys Gly Leu Arg Pro Leu Ile
           115                 120                 125
   acg cag ttg cag atc gtc cag ttc atc ttc agc atc ggc atc cat acc   432
   Thr Gln Leu Gln Ile Val Gln Phe Ile Phe Ser Ile Gly Ile His Thr
       130                 135                 140
   gcc atc tac tgg cac tac gac tgc gag ccg ctc gtg cat acc cac ttt   480
   Ala Ile Tyr Trp His Tyr Asp Cys Glu Pro Leu Val His Thr His Phe
   145                 150                 155                 160
   tgg gaa tac gtc acg ccc tac ctc ttc gtc gtg ccc ttc ctc atc ctc   528
   Trp Glu Tyr Val Thr Pro Tyr Leu Phe Val Val Pro Phe Leu Ile Leu
                   165                 170                 175
   ttt ctc aat ttc tac ctg cag cag tac gtc ctc gcg ccc gca aaa acc   576
   Phe Leu Asn Phe Tyr Leu Gln Gln Tyr Val Leu Ala Pro Ala Lys Thr
               180                 185                 190
   aag aag gca tag ccacgtaaca gtagaccagc agcgccgagg acgcgtgccg       628
   Lys Lys Ala
           195
   cgttatcgcg aagcacgaaa taaagaagat catttgattc aaaaaaaaaa aaaaaaaaa    687
<210> 4
   <211> 195
   <212> PRT
   <213> Thraustochytrium
<400> 4
   Arg Ser Val His Asn Leu Gly Leu Cys Leu Phe Ser Gly Ala Val Trp
   1               5                   10                  15
   Ile Tyr Thr Ser Tyr Leu Met Ile Gln Asp Gly His Phe Arg Ser Leu
               20                  25                  30
   Glu Ala Ala Thr Cys Glu Pro Leu Lys His Pro His Phe Gln Leu Ile
           35                  40                  45
   Ser Leu Leu Phe Ala Leu Ser Lys Ile Trp Glu Trp Phe Asp Thr Val
       50                  55                  60
   Leu Leu Ile Val Lys Gly Asn Lys Leu Arg Phe Leu His Val Leu His
   65                  70                  75                  80
   His Ala Thr Thr Phe Trp Leu Tyr Ala Ile Asp His Ile Phe Leu Ser
                   85                  90                  95
   Ser Ile Lys Tyr Gly Val Ala Val Asn Ala Phe Ile His Thr Val Met
               100                 105                 110
   Tyr Ala His Tyr Phe Arg Pro Phe Pro Lys Gly Leu Arg Pro Leu Ile
           115                 120                 125
   Thr Gln Leu Gln Ile Val Gln Phe Ile Phe Ser Ile Gly Ile His Thr
       130                 135                 140
   Ala Ile Tyr Trp His Tyr Asp Cys Glu Pro Leu Val His Thr His Phe
   145                 150                 155                 160
   Trp Glu Tyr Val Thr Pro Tyr Leu Phe Val Val Pro Phe Leu Ile Leu
                   165                 170                 175
   Phe Leu Asn Phe Tyr Leu Gln Gln Tyr Val Leu Ala Pro Ala Lys Thr
               180                 185                 190
   Lys Lys Ala
           195
<210> 5
   <211> 955
   <212> DNA
   <213> Thaustochytrium
<220>
   <221> CDS
   <222> (1)..(894)
<400> 5
   gtc att tcg ggc ctc gac ctt ctc ccc gtg ctc tcg tgg gag act atg      48
   Val Ile Ser Gly Leu Asp Leu Leu Pro Val Leu Ser Trp Glu Thr Met
   1               5                   10                  15
   aag ttc gac act gcc gaa gtt gtc tcg gtc tgg ctg cgc acg cac atg      96
   Lys Phe Asp Thr Ala Glu Val Val Ser Val Trp Leu Arg Thr His Met
               20                  25                  30
   tgg gtc ccc ttc ctg atg tgc ttc atc tac ctg gtc gtc atc ttc ggc      144
   Trp Val Pro Phe Leu Met Cys Phe Ile Tyr Leu Val Val Ile Phe Gly
           35                  40                  45
   atc cag tac tac atg gag gac cgc aag gag ttc gat ctg cgc aag ccg      192
   Ile Gln Tyr Tyr Met Glu Asp Arg Lys Glu Phe Asp Leu Arg Lys Pro
       50                  55                  60
   ctg gcc gcc tgg agc gcc ttc ttg gcc att ttc agc atc ggc gcc tcc      240
   Leu Ala Ala Trp Ser Ala Phe Leu Ala Ile Phe Ser Ile Gly Ala Ser
   65                  70                  75                  80
   atc cgc acc gtg ccc gtc ctg ctc aag atg ctc tac gaa aag ggc acg      288
   Ile Arg Thr Val Pro Val Leu Leu Lys Met Leu Tyr Glu Lys Gly Thr
                   85                  90                  95
   cac cac gtg ctc tgc ggc gac acg cgc aac gac tgg gtc att gac aac      336
   His His Val Leu Cys Gly Asp Thr Arg Asn Asp Trp Val Ile Asp Asn
               100                 105                 110
   ccg gcc ggc gtc tgg acc atg gcc ttt atc ttt tcc aag att ccc gag      384
   Pro Ala Gly Val Trp Thr Met Ala Phe Ile Phe Ser Lys Ile Pro Glu
           115                 120                 125
   ctc atc gac acc ctc ttt atc gtg ctc cgc aag cgc aag ctc atc acc      432
   Leu Ile Asp Thr Leu Phe Ile Val Leu Arg Lys Arg Lys Leu Ile Thr
       130                 135                 140
   ctc cac tgg tac cac cac gtg acc gtg ctc ctg ttc tgc tgg cac gcc      480
   Leu His Trp Tyr His His Val Thr Val Leu Leu Phe Cys Trp His Ala
   145                 150                 155                 160
   tgg gcc acc ttt gcg ctc acc ggc atc gtc ttt gcc gcc atc aac gcc    528
   Trp Ala Thr Phe Ala Leu Thr Gly Ile Val Phe Ala Ala Ile Asn Ala
                   165                 170                 175
   tcg gtg cac gcc atc atg tac gcc tat tac gcc ttc acg gcc ctc ggc    576
   Ser Val His Ala Ile Met Tyr Ala Tyr Tyr Ala Phe Thr Ala Leu Gly
               180                 185                 190
   tac cga cca acc tcg tac gcc atc tac att acg ctc att cag atc atg    624
   Tyr Arg Pro Thr Ser Tyr Ala Ile Tyr Ile Thr Leu Ile Gln Ile Met
           195                 200                 205
   cag atg gtc gtc ggc acc gcc gtc acc ttt tac att ggc tac gac atg    672
   Gln Met Val Val Gly Thr Ala Val Thr Phe Tyr Ile Gly Tyr Asp Met
       210                 215                 220
   gcc ttt gtc acg ccg cag ccc ttc cgc ctt gac atg aaa ctc aac tgg    720
   Ala Phe Val Thr Pro Gln Pro Phe Arg Leu Asp Met Lys Leu Asn Trp
   225                 230                 235                 240
   gac ccg ctc agc aag ggc gag aac acc gag ccc acc tgc aag ggc gcc    768
   Asp Pro Leu Ser Lys Gly Glu Asn Thr Glu Pro Thr Cys Lys Gly Ala
                   245                 250                 255
   aac tcc tcc aac gcc atc ttc ggc gtc atc atg tac gcc tcg tac ctc    816
   Asn Ser Ser Asn Ala Ile Phe Gly Val Ile Met Tyr Ala Ser Tyr Leu
               260                 265                 270
   tac ctc ttc tgc ctc ttc ttc tac atg gcc tac ctg cgc ccg aag aag    864
   Tyr Leu Phe Cys Leu Phe Phe Tyr Met Ala Tyr Leu Arg Pro Lys Lys
           275                 280                 285
   tcg acg ccc gcg gcc aag aag aca aac taa tcgcacacta ccaaacaatc      914
   Ser Thr Pro Ala Ala Lys Lys Thr Asn
       290                 295
   ttccactcga cctagaaaaa aaaaaaaaaa aaaaactcga g                      955
<210> 6
   <211> 297
   <212> PRT
   <213> Thaustochytrium
<400> 6
   Val Ile Ser Gly Leu Asp Leu Leu Pro Val Leu Ser Trp Glu Thr Met
   1               5                   10                  15
   Lys Phe Asp Thr Ala Glu Val Val Ser Val Trp Leu Arg Thr His Met
               20                  25                  30
   Trp Val Pro Phe Leu Met Cys Phe Ile Tyr Leu Val Val Ile Phe Gly
           35                  40                  45
   Ile Gln Tyr Tyr Met Glu Asp Arg Lys Glu Phe Asp Leu Arg Lys Pro
       50                  55                  60
   Leu Ala Ala Trp Ser Ala Phe Leu Ala Ile Phe Ser Ile Gly Ala Ser
   65                  70                  75                  80
   Ile Arg Thr Val Pro Val Leu Leu Lys Met Leu Tyr Glu Lys Gly Thr
                   85                  90                  95
   His His Val Leu Cys Gly Asp Thr Arg Asn Asp Trp Val Ile Asp Asn
               100                 105                 110
   Pro Ala Gly Val Trp Thr Met Ala Phe Ile Phe Ser Lys Ile Pro Glu
           115                 120                 125
   Leu Ile Asp Thr Leu Phe Ile Val Leu Arg Lys Arg Lys Leu Ile Thr
       130                 135                 140
   Leu His Trp Tyr His His Val Thr Val Leu Leu Phe Cys Trp His Ala
   145                 150                 155                 160
   Trp Ala Thr Phe Ala Leu Thr Gly Ile Val Phe Ala Ala Ile Asn Ala
                   165                 170                 175
   Ser Val His Ala Ile Met Tyr Ala Tyr Tyr Ala Phe Thr Ala Leu Gly
               180                 185                 190
   Tyr Arg Pro Thr Ser Tyr Ala Ile Tyr Ile Thr Leu Ile Gln Ile Met
           195                 200                 205
   Gln Met Val Val Gly Thr Ala Val Thr Phe Tyr Ile Gly Tyr Asp Met
       210                 215                 220
   Ala Phe Val Thr Pro Gln Pro Phe Arg Leu Asp Met Lys Leu Asn Trp
   225                 230                 235                 240
   Asp Pro Leu Ser Lys Gly Glu Asn Thr Glu Pro Thr Cys Lys Gly Ala
                   245                 250                 255
   Asn Ser Ser Asn Ala Ile Phe Gly Val Ile Met Tyr Ala Ser Tyr Leu
                   260                 265                 270
   Tyr Leu Phe Cys Leu Phe Phe Tyr Met Ala Tyr Leu Arg Pro Lys Lys
               275                 280                 285
   Ser Thr Pro Ala Ala Lys Lys Thr Asn
           290                 295
<210> 7
   <211> 708
   <212> DNA
   <213> Crypthecodinium
<220>
   <221> CDS
   <222> (1)..(645)
<400> 7
   cgg cac gag gta cac atg acc gag aag agg gga ctg cag ttc acg atc    48
   Arg His Glu Val His Met Thr Glu Lys Arg Gly Leu Gln Phe Thr Ile
    1               5                  10                  15
   tgc ggc tct act ggt gag ttg gtg cag aat ctc cag gat ggt ccc act    96
   Cys Gly Ser Thr Gly Glu Leu Val Gln Asn Leu Gln Asp Gly Pro Thr
               20                  25                  30
   gcc ttg gcg ttg tgc ctc ttt tgc ttc agc aaa att ccc gag ttg atg    144
   Ala Leu Ala Leu Cys Leu Phe Cys Phe Ser Lys Ile Pro Glu Leu Met
           35                  40                  45
   gac acg gtc ttt ctc atc ttg aag ggc aag aag gtt cgc ttt ttg cag    192
   Asp Thr Val Phe Leu Ile Leu Lys Gly Lys Lys Val Arg Phe Leu Gln
       50                  55                  60
   tgg tac cac cac gct acc gtg atg ctc ttc tgc tgg ttg gca ctg gct    240
   Trp Tyr His His Ala Thr Val Met Leu Phe Cys Trp Leu Ala Leu Ala
   65                  70                  75                  80
   acg gag tac acc ccg ggc ctc tgg ttc gcg gcc act aac tac ttc gtg    288
   Thr Glu Tyr Thr Pro Gly Leu Trp Phe Ala Ala Thr Asn Tyr Phe Val
                   85                  90                  95
   cac tcc atc atg tac atg tac ttc ttc ttg atg acc ttc aag acg gcc    336
   His Ser Ile Met Tyr Met Tyr Phe Phe Leu Met Thr Phe Lys Thr Ala
               100                 105                 110
   gca aag gtc gtg aag ccc att gcc cct ctc atc acc atc atc cag atc    384
   Ala Lys Val Val Lys Pro Ile Ala Pro Leu Ile Thr Ile Ile Gln Ile
            115                 120                 125
   gcc cag atg gtc tgg ggt ctc atc gtc aac ggc atc gcg atc acc act      432
   Ala Gln Met Val Trp Gly Leu Ile Val Asn Gly Ile Ala Ile Thr Thr
       130                 135                 140
   ttc ttc acc acg ggc gcc tgc cag atc cag tcc gtg acg gtc tac tcg      480
   Phe Phe Thr Thr Gly Ala Cys Gln Ile Gln Ser Val Thr Val Tyr Ser
   145                 150                 155                 160
   gcc att gtg atg tac gct tcg tac ttc tac ctc ttc tcc cag ctc ttc      528
   Ala Ile Val Met Tyr Ala Ser Tyr Phe Tyr Leu Phe Ser Gln Leu Phe
                   165                 170                 175
   ctg gag gca tac gga tcc gct ggc aag aac aag aag aag ctc gcc cgc      576
   Leu Glu Ala Tyr Gly Ser Ala Gly Lys Asn Lys Lys Lys Leu Ala Arg
               180                 185                 190
   gag ctc tcc cga aag atc tcc gag gct ctc ctg aat agt ggc gac gag      624
   Glu Leu Ser Arg Lys Ile Ser Glu Ala Leu Leu Asn Ser Gly Asp Glu
           195                 200                 205
   gta gcc aag cac ctc aag tga actgagcgac ctcatcttgg tctggtccgc        675
   Val Ala Lys His Leu Lys
       210                 215
   caaattgccg cgtgcatgtg catgagatgc tgt                                708
<210> 8
   <211> 214
   <212> PRT
   <213> Crypthecodinium
<400> 8
   Arg His Glu Val His Met Thr Glu Lys Arg Gly Leu Gln Phe Thr Ile
   1 5 10 15
   Cys Gly Ser Thr Gly Glu Leu Val Gln Asn Leu Gln Asp Gly Pro Thr
   20 25 30
   Ala Leu Ala Leu Cys Leu Phe Cys Phe Ser Lys Ile Pro Glu Leu Met
   35 40 45
   Asp Thr Val Phe Leu Ile Leu Lys Gly Lys Lys Val Arg Phe Leu Gln
   50 55 60
   Trp Tyr His His Ala Thr Val Met Leu Phe Cys Trp Leu Ala Leu Ala
   65 70 75 80
   Thr Glu Tyr Thr Pro Gly Leu Trp Phe Ala Ala Thr Asn Tyr Phe Val
   85 90 95
   His Ser Ile Met Tyr Met Tyr Phe Phe Leu Met Thr Phe Lys Thr Ala
   100 105 110
   Ala Lys Val Val Lys Pro Ile Ala Pro Leu Ile Thr Ile Ile Gln Ile
   115 120 125
   Ala Gln Met Val Trp Gly Leu Ile Val Asn Gly Ile Ala Ile Thr Thr
   130 135 140
   Phe Phe Thr Thr Gly Ala Cys Gln Ile Gln Ser Val Thr Val Tyr Ser
   145 150 155 160
   Ala Ile Val Met Tyr Ala Ser Tyr Phe Tyr Leu Phe Ser Gln Leu Phe
   165 170 175
   Leu Glu Ala Tyr Gly Ser Ala Gly Lys Asn Lys Lys Lys Leu Ala Arg
   180 185 190
   Glu Leu Ser Arg Lys Ile Ser Glu Ala Leu Leu Asn Ser Gly Asp Glu
   195 200 205
   Val Ala Lys His Leu Lys
   210
<210> 9
   <211> 1054
   <212> DNA
   <213> Thraustochytrium
<220>
   <221> CDS
   <222> (43)..(858)
<400> 9
   gaattcggca cgagagcgcg cggagcggag acctcggccg cg atg atg gag ccg    54
                                                  Met Met Glu Pro
                                                  1
   ctc gac agg tac agg gcg ctg gcg gag ctc gcc gcg agg tac gcc agc   102
   Leu Asp Arg Tyr Arg Ala Leu Ala Glu Leu Ala Ala Arg Tyr Ala Ser
   5                   10                  15                  20
   tcg gcg gcc ttc aag tgg caa gtc acg tac gac gcc aag gac agc ttc    150
   Ser Ala Ala Phe Lys Trp Gln Val Thr Tyr Asp Ala Lys Asp Ser Phe
                   25                  30                  35
   gtc ggg ccc ctg gga atc cgg gag ccg ctc ggg ctc ctg gtg ggc tcc    198
   Val Gly Pro Leu Gly Ile Arg Glu Pro Leu Gly Leu Leu Val Gly Ser
               40                  45                  50
   gtg gtc ctc tac ctg agc ctg ctg gcc gtg gtc tac gcg ctg cgg aac    246
   Val Val Leu Tyr Leu Ser Leu Leu Ala Val Val Tyr Ala Leu Arg Asn
           55                  60                  65
   tac ctt ggc ggc ctc atg gcg ctc cgc agc gtg cat aac ctc ggg ctc    294
   Tyr Leu Gly Gly Leu Met Ala Leu Arg Ser Val His Asn Leu Gly Leu
       70                  75                  80
   tgc ctc ttc tcg ggc gcc gtg tgg atc tac acg agc tac ctc atg atc    342
   Cys Leu Phe Ser Gly Ala Val Trp Ile Tyr Thr Ser Tyr Leu Met Ile
   85                  90                  95                  100
   cag gat ggg cac ttt cgc agc ctc gag gcg gca acg tgc gag ccg ctc    390
   Gln Asp Gly His Phe Arg Ser Leu Glu Ala Ala Thr Cys Glu Pro Leu
                   105                 110                 115
   aag cat ccg cac ttc cag ctc atc agc ttg ctc ttt gcg ctg tcc aag    438
   Lys His Pro His Phe Gln Leu Ile Ser Leu Leu Phe Ala Leu Ser Lys
               120                 125                 130
   atc tgg gag tgg ttc gac acg gtg ctc ctc atc gtc aag ggc aac aag    486
   Ile Trp Glu Trp Phe Asp Thr Val Leu Leu Ile Val Lys Gly Asn Lys
           135                 140                 145
   ctc cgc ttc ctg cac gtc ttg cac cac gcc acg acc ttt tgg ctc tac    534
   Leu Arg Phe Leu His Val Leu His His Ala Thr Thr Phe Trp Leu Tyr
       150                 155                 160
   gcc atc gac cac atc ttt ctc tcg tcc atc aag tac ggc gtc gcg gtc    582
   Ala Ile Asp His Ile Phe Leu Ser Ser Ile Lys Tyr Gly Val Ala Val
   165                 170                 175                 180
   aat gct ttc atc cac acc gtc atg tac gcg cac tac ttc cgc cca ttc    630
   Asn Ala Phe Ile His Thr Val Met Tyr Ala His Tyr Phe Arg Pro Phe
                   185                 190                 195
   ccg aag ggc ttg cgc ccg ctt att acg cag ttg cag atc gtc cag ttc    678
   Pro Lys Gly Leu Arg Pro Leu Ile Thr Gln Leu Gln Ile Val Gln Phe
               200                 205                 210
   att ttc agc atc ggc atc cat acc gcc att tac tgg cac tac gac tgc     726
   Ile Phe Ser Ile Gly Ile His Thr Ala Ile Tyr Trp His Tyr Asp Cys
           215                 220                     225
   gag ccg ctc gtg cat acc cac ttt tgg gaa tac gtc acg ccc tac ctt     774
   Glu Pro Leu Val His Thr His Phe Trp Glu Tyr Val Thr Pro Tyr Leu
       230                 235                 240
   ttc gtc gtg ccc ttc ctc atc ctc ttt ttc aat ttt tac ctg cag cag     822
   Phe Val Val Pro Phe Leu Ile Leu Phe Phe Asn Phe Tyr Leu Gln Gln
   245                 250                 255                 260
   tac gtc ctc gcg ccc gca aaa acc aag aag gca tag ccacgtaaca          868
   Tyr Val Leu Ala Pro Ala Lys Thr Lys Lys Ala
                   265                 270
   gtagaccagc agcgccgagg acgcgtgccg cgttatcgcg aagcacgaaa taaagaagat 928
   catttgattc aacgaggcta cttgcggcca cgagaaaaaa aaaaaaaaaa aaaaaaaaaa 988
   aaaaaaaaaa aaaaaaaaaa aaaaaaaaaa aaaaaaaaaa aaaaaaaaaa aaaaaaaaaa 1048
   ctcgag                                                            1054
<210> 10
   <211> 271
   <212> PRT
   <213> Thraustochytrium
<400> 10
   Met Met Glu Pro Leu Asp Arg Tyr Arg Ala Leu Ala Glu Leu Ala Ala
   1               5                   10                  15
   Arg Tyr Ala Ser Ser Ala Ala Phe Lys Trp Gln Val Thr Tyr Asp Ala
               20                  25                  30
   Lys Asp Ser Phe Val Gly Pro Leu Gly Ile Arg Glu Pro Leu Gly Leu
           35                  40                  45
   Leu Val Gly Ser Val Val Leu Tyr Leu Ser Leu Leu Ala Val Val Tyr
       50                  55                  60
   Ala Leu Arg Asn Tyr Leu Gly Gly Leu Met Ala Leu Arg Ser Val His
   65                  70                  75                  80
   Asn Leu Gly Leu Cys Leu Phe Ser Gly Ala Val Trp Ile Tyr Thr Ser
                   85                  90                  95
   Tyr Leu Met Ile Gln Asp Gly His Phe Arg Ser Leu Glu Ala Ala Thr
               100                 105                 110
   Cys Glu Pro Leu Lys His Pro His Phe Gln Leu Ile Ser Leu Leu Phe
           115                 120                 125
   Ala Leu Ser Lys Ile Trp Glu Trp Phe Asp Thr Val Leu Leu Ile Val
       130                 135                 140
   Lys Gly Asn Lys Leu Arg Phe Leu His Val Leu His His Ala Thr Thr
   145                 150                 155                 160
   Phe Trp Leu Tyr Ala Ile Asp His Ile Phe Leu Ser Ser Ile Lys Tyr
                   165                 170                 175
   Gly Val Ala Val Asn Ala Phe Ile His Thr Val Met Tyr Ala His Tyr
               180                 185                 190
   Phe Arg Pro Phe Pro Lys Gly Leu Arg Pro Leu Ile Thr Gln Leu Gln
           195                 200                 205
   Ile Val Gln Phe Ile Phe Ser Ile Gly Ile His Thr Ala Ile Tyr Trp
       210                 215                 220
   His Tyr Asp Cys Glu Pro Leu Val His Thr His Phe Trp Glu Tyr Val
   225                 230                 235                 240
   Thr Pro Tyr Leu Phe Val Val Pro Phe Leu Ile Leu Phe Phe Asn Phe
                   245                 250                 255
   Tyr Leu Gln Gln Tyr Val Leu Ala Pro Ala Lys Thr Lys Lys Ala
               260                 265                 270
<210> 11
   <211> 421
   <212> DNA
   <213> Phytophthora infestans
<220>
   <221> CDS
   <222> (1)..(279)
<400> 11
   cac acc atc atg tac act tac tac ttc gtc agc gcc cac acg cgc aac    48
   His Thr Ile Met Tyr Thr Tyr Tyr Phe Val Ser Ala His Thr Arg Asn
    1               5                  10                  15
   att tgg tgg aag aag tac ctc acg cgc att cag ctt atc cag ttc gtg    96
   Ile Trp Trp Lys Lys Tyr Leu Thr Arg Ile Gln Leu Ile Gln Phe Val
               20                  25                  30
   acc atg aac gtg cag ggc tac ctg acc tac tct cga cag tgc cca ggc   144
   Thr Met Asn Val Gln Gly Tyr Leu Thr Tyr Ser Arg Gln Cys Pro Gly
           35                  40                  45
   atg cct cct aag gtg ccg ctc atg tac ctt gtg tac gtg cag tca ctc   192
   Met Pro Pro Lys Val Pro Leu Met Tyr Leu Val Tyr Val Gln Ser Leu
       50                  55                  60
   ttc tgg ctc ttc atg aat ttc tac att cgc gcg tac gtg ttc ggc ccc   240
   Phe Trp Leu Phe Met Asn Phe Tyr Ile Arg Ala Tyr Val Phe Gly Pro
   65                  70                  75                  80
   aag aaa ccg gcc gtg gag gaa tcg aag aag aag ttg taa cggcgcttgt    289
   Lys Lys Pro Ala Val Glu Glu Ser Lys Lys Lys Leu
                   85                  90
   taaaaagtct aacctcgctg taacagctta aaacacacac acacacaacg ctttgtagag 349
   gaggtaagta gtggcaactc gtgtagaaat gcagcatgcc catcaaatac atcccgtatg 409
   attcatacta ct                                                     421
<210> 12
   <211> 92
   <212> PRT
   <213> Phytophthora infestans
<400> 12
   His Thr Ile Met Tyr Thr Tyr Tyr Phe Val Ser Ala His Thr Arg Asn
    1               5                  10                  15
   Ile Trp Trp Lys Lys Tyr Leu Thr Arg Ile Gln Leu Ile Gln Phe Val
               20                  25                  30
   Thr Met Asn Val Gln Gly Tyr Leu Thr Tyr Ser Arg Gln Cys Pro Gly
           35                  40                  45
   Met Pro Pro Lys Val Pro Leu Met Tyr Leu Val Tyr Val Gln Ser Leu
       50                  55                  60
   Phe Trp Leu Phe Met Asn Phe Tyr Ile Arg Ala Tyr Val Phe Gly Pro
   65                  70                  75                  80
   Lys Lys Pro Ala Val Glu Glu Ser Lys Lys Lys Leu
                   85                  90